# EUROPÄISCHE PATENTSCHRIFT

(11) **EP 1 773 906 B1**
(45) Veröffentlichungstag und Bekanntmachung des Hinweises auf die Patenterteilung: **29.04.2009**
(21) Anmeldenummer: 05770627.7
(22) Anmeldetag: 22.07.2005
(51) Int. Cl.: C08G 18/72, C08G 18/48, C08G 18/08

(54) **VERNETZTE POLYTETRAHYDROFURAN-HALTIGE POLYURETHANE**
CROSS-LINKED POLYTETRAHYDROFURAN-CONTAINING POLYURETHANES
POLYURETHANES RETICULES CONTENANT UN POLYTETRAHYDROFURANE

(30) Priorität: 26.07.2004 DE 102004036146
(43) Veröffentlichungstag der Anmeldung: 18.04.2007
(73) Patentinhaber: BASF SE, 67056 Ludwigshafen (DE)
(72) Erfinder: NGUYEN-KIM, Son, 69502 Hemsbach (DE); KAISER, Thomas, 67454 Hassloch (DE); HAUCK, Rolf, 67133 Maxdorf (DE)
(74) Vertreter: Reitstötter - Kinzebach
(86) Internationale Anmeldenummer: PCT/EP2005/008040
(87) Internationale Veröffentlichungsnummer: WO 2006/010571

(56) Entgegenhaltungen:
- EP-A- 0 916 647
- DE-A1- 10 214 971
- GB-A- 2 386 898

## Beschreibung

Die vorliegende Erfindung betrifft ein vernetztes Polyurethan, das mindestens ein Polytetrahydrofuran und ein Polyisocyanatgemisch eingebaut enthält, kosmetische oder pharmazeutische Mittel, die ein solches Polyurethan enthalten sowie die Verwendung dieser Polyurethane.

Kosmetisch und pharmazeutisch akzeptable wasserlösliche oder wasserdispergierbare Polymere finden in Kosmetik und Medizin verbreitet Anwendung. Sie dienen beispielsweise ganz allgemein als Verdicker für diverse Arten von Formulierungen, wie z. B. Gele, Cremes oder Emulsionen. Häufig werden für diese Anwendungen verzweigte oder vernetzte wasserlösliche Polymere mit anionischen Funktionalitäten, wie z. B. vernetzte Polyacrylsäure, eingesetzt. Speziell für die Haarkosmetik werden vernetzte Polymere mit filmbildenden Eigenschaften als Conditioner dazu eingesetzt, um die Trocken- und Nasskämmbarkeit, das Anfassgefühl, den Glanz und/oder die Erscheinungsform des Haars zu verbessern sowie dem Haar antistatische Eigenschaften zu verleihen. Neben den zuvor genannten Carboxylatgruppen-haltigen Polymeren werden als Conditioner häufig vernetzte Polymere mit kationischen Funktionalitäten eingesetzt, die eine hohe Affinität zur strukturell bedingt negativ geladenen Oberfläche des Haars aufweisen. Dazu zählen beispielsweise vernetzte Copolymere aus N-Vinylpyrrolidon, quaterniertem N-Vinylimidazol, Acrylamid und Diallyldimethylammoniumchlorid (DADMAC).

Schwierigkeiten bereitet oft die Bereitstellung von Produkten mit einem komplexen Eigenschaftsprofil. So besteht ein Bedarf an Polymeren für kosmetische und pharmazeutische Mittel, die zum einen gute filmbildende Eigenschaften und zum Anderen eine gute Flexibilität der erhaltenen Filme aufweisen.

Die WO 94/03510 beschreibt die Verwendung von Polyurethanen aus
a) mindestens einer Verbindung, die zwei oder mehrere aktive Wasserstoffatome pro Molekül enthält,
b) mindestens einem Säure- oder Salzgruppen enthaltenden Diol und
c) mindestens einem Diisocyanat
mit einer Glasübergangstemperatur von mindestens 15°C und Säurezahlen von 12 bis 150 oder den Salzen dieser Polyurethane in kosmetischen Zubereitungen und als Bindemittel oder Überzugsmittel in pharmazeutischen Zubereitungen.

Die EP-A-619 111 beschreibt die Verwendung von Polyurethanen auf Basis von organischen Diisocyanaten, Diolen und 2,2-Hydroxymethyl-substituierten Carboxylaten der Formel 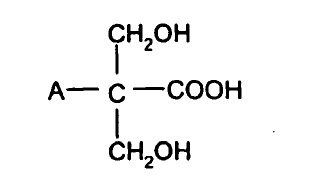 worin A für ein Wasserstoffatom oder eine C₁-C₂₀-Alkylgruppe steht, in Haarfixierungsmitteln. Zumindest ein Teil der Carbonsäuregruppen wird dabei mit einer organischen oder anorganischen Base neutralisiert. Die Diole weisen dabei ein Molekulargewicht im Bereich von 300 bis 20000 auf, wobei als geeignete Diolkomponente unter anderem auch Polytetrahydrofurane genannt werden.

Die WO 94/13724 beschreibt die Verwendung von kationischen Polyurethanen und Polyharnstoffen aus
(a) mindestens einem Diisocyanat, welches bereits vorher mit einer oder mehreren Verbindungen, die zwei oder mehrere aktive Wasserstoffatome pro Molekül enthalten, umgesetzt sein kann, und
(b) mindestens einem ein oder mehrere tertiäre, quartäre oder protonierte tertiäre Aminstickstoffatome enthaltenden Diol, primären oder sekundären Aminoalkohol, primären oder sekundären Diamin oder primären oder sekundären Triamin
mit einer Glasübergangstemperatur von mindestens 25 °C und einer Aminzahl von 50 bis 200, bezogen auf die nicht quatemisierten oder protonierten Verbindungen, oder sonstigen Salzen dieser Polyurethane und Polyharnstoffe als Hilfsmittel in kosmetischen und pharmazeutischen Zubereitungen.

WO 01/16200 beschreibt ein kosmetisches Mittel, enthaltend wasserlösliche oder wasserdispergierbare Polyurethane aus einem Oligomer oder Polymer, aus
A) mindestens einem Diisocyanat,
B) mindestens einer Verbindung mit wenigstens zwei gegenüber Isocyanatgruppen reaktiven Gruppen, die ausgewählt ist unter
   B1) aliphatischen und cycloaliphatischen Polyolen, Polyaminen und/oder Aminoalkoholen,
   B2) Polyetherolen und/oder Diaminopolyethern,
   B3) Polysiloxanen mit wenigstens zwei aktiven Wasserstoffatomen pro Molekül,
   B4) Polyesterpolyolen,
   und Mischungen davon, und
C) gegebenenfalls mindestens einer Dicarbonsäure und/oder Hydroxycarbonsäure,
   wobei das Oligomer pro Molekül wenigstens zwei Urethan- und/oder Harnstoffgruppen und zusätzlich wenigstens zwei weitere funktionelle Gruppen, die ausgewählt sind unter Hydroxyl-, primären und/oder sekundären Aminogruppen, umfasst.

EP-A-938 889 beschreibt ein kosmetisches Mittel, enthaltend wenigstens ein wasserlösliches oder wasserdispergierbares Polyurethan aus
a) mindestens einem Polymerisat mit zwei aktiven Wasserstoffatomen pro Molekül, welches ausgewählt ist unter Polytetrahydrofuranen, Polysiloxanen und Mischungen davon,
b) mindestens einem Polyesterdiol,
c) mindestens einer Verbindung mit einem Molekulargewicht im Bereich von 56 bis 300, die zwei aktive Wasserstoffatome pro Molekül enthält,
d) mindestens einer Verbindung, die zwei aktive Wasserstoffatome und mindestens eine anionogene bzw. anionische Gruppe pro Molekül aufweist,
e) mindestens einem Diisocyanat,
oder die Salze davon, wobei das Polyurethan keine von einem primären oder sekundären Amin, welches eine ionogene bzw. ionische Gruppe aufweist, stammende Einheit enthält.

Die WO 99/58100 beschreibt ein kosmetisches Mittel, enthaltend wenigstens ein vernetztes, wasserlösliches oder wasserdispergierbares Polyurethan aus mindestens einem Polyurethanpräpolymer mit endständigen Isocyanatgruppen und mindestens einem Polymerisat mit gegenüber Isocyanatgruppen reaktiven Gruppen, wobei wenigstens eine der Komponenten eine Siloxangruppe enthält.

Die WO 01/85821 beschreibt Polyurethanen auf Basis von mindestens einem Polyether mit zwei aktiven Wasserstoffatomen pro Molekül und deren Verwendung zur Modifizierung rheologische Eigenschaften.

Die DE-A-1 102 59 036 beschreibt Allylgruppen-haltige Polyetherurethane, Polymere, die diese einpolymerisiert enthalten sowie kosmetische oder pharmazeutische Mittel auf Basis dieser Polymere,

Die EP-A-957 119 beschreibt vernetzte, wasserlösliche oder wasserdispergierbare Polyurethane aus
A) mindestens einem wasserlöslichen oder wasserdispergierbaren Polyurethanpräpolymer mit endständigen Isocyanatgruppen aus
   a) mindestens einer Verbindung mit einem Molekulargewicht im Bereich von 56 bis 300, die zwei aktive Wasserstoffatome pro Molekül enthält,
   b) mindestens einem Polymerisat mit zwei aktiven Wasserstoffatomen pro Molekül,
   c) mindestens einer Verbindung, die zwei aktive Wasserstoffatome und mindestens eine ionogene bzw. ionische Gruppe pro Molekül aufweist,
   d) mindestens einem Diisocyanat,
B) mindestens einem Polymerisat mit gegenüber Isocyanatgruppen reaktiven Gruppen, die ausgewählt sind unter Hydroxyl-, primären und sekundären Amino-und/oder Carboxylgruppen,
   oder die Salze davon, und die Verwendung dieser Polyurethane als Hilfsmittel in der Kosmetik.

Die WO 03/085019 beschreibt vernetzte Polyurethane auf Basis von Polytetrahydrofuran und deren Verwendung in kosmetischen und pharmazeutischen Mitteln.

Die GB-A-2 386 898 beschreibt eine wässrige Polyurethandispersion, enthaltend ein Polyurethan aus
(A) einem Isocyanat-terminiertem Präpolymer gebildet aus Komponenten enthaltend
   a) von 10 bis 25 Gew.-% Polyisocyanat(en);
   b) 40 bis 80 Gel.-% Polytetrahydrofuranpolyol(en);
   c) 0 bis 5 Gew,-% Polyole enthaltend ionische oder potenziell ionische wasserdisperierende Gruppen mit zwei oder mehreren Isocyanat-reaktiven Gruppen;
   d) 0 bis 50 Gew.-% Komponente(n), die nicht in a), b) oder c) enthalten ist (sind), wobei a), b), c) und d) zusammen 100 Gel.-% ergeben;
   und
(B) einer kettenerweitemden Verbindung mit aktiven Wasserstoffatomen, Zusätzlich werden Polyurethandispersionen beschrieben, wobei die Polyisocyanate eine Mischung aus Hexamethylendiisocyanat und Isophorondiisocyanat umfassen.

Die EP-A-916 647 beschreibt Hydrophilierungsmittel hergestellt durch Michael-Addition von 1-Amino-3,3,5-trimethyl-5-amino-methyl-cyclohexan mit 1 bis 2 Mol einer α,β-ungesättigten Carbonsäure und gegebenenfalls anschließende Neutralisierung mit bis zu der COOH-Gruppe äquivalenten Menge (Erd)alkalihydroxid und/oder Aminen und/oder Ammoniak; sowie die Verwendung dieser Hydrophobierungsmittel zur Herstellung von PUR-Dispersionen.

Der vorliegenden Erfindung liegt die Aufgabe zu Grunde, neue filmbildende Polymere zur Verfügung zu stellen, die sich zur Herstellung von kosmetischen und/oder pharmazeutischen Mitteln eignen. Diese Polymere sollen neben einem insgesamt guten Anwendungsprofil für diesen Einsatzbereich vor allem über sehr gute Filmbildungseigenschaften verfügen, die zu Filmen mit hoher Festigungswirkung und gleichzeitig hoher Flexibilität führt. Zudem sollen die erhaltenen Filme möglichst klar sein.

Überraschenderweise wurde nun gefunden, dass diese Aufgabe durch vernetzte Polyurethane gelöst wird, die mindestens ein Polytetrahydrofuran und als Isocyanatkomponente ein Polyisocyanatgemisch, das Isophorondilsocyanat und Hexamethylendlisocyanat enthält, eingebaut enthalten. Gegenstand der Erfindung Ist daher ein vernetztes Polyurethan, das
A) mindestens ein Polytetrahydrofuran mit zwei endständigen Hydroxylgruppen pro Molekül und einem zahlenmittleren Molekulargewicht im Bereich von 650 bis 2000,
B) mindestens eine Verbindung, die mehr als zwei aktive Wasserstoffatome pro Molekül enthält,
C) mindestens eine Verbindung, die mindestens zwei aktive Wasserstoffatome und mindestens eine anlonogene und/oder anionische Gruppe pro Molekül enthält,
D) ein Polyisocyanatgemisch, das Isophorondiisocyanat und Hexamethylendiisocyanat enthält,
   wobei das Gewichtsmengenverhältnis von Isophorondiisocyanat zu Hexamethylendiisocyanat in einem Bereich von 4;1 bis 15:1 liegt, und
E) mindestens eine von A) bis D) verschiedene Verbindung mit mindestens zwei aktiven Wasserstoffatomen und einem Molekulargewicht von 60 bis 5000,
eingebaut enthält und die Salze davon.

Eine geeignete Ausführungsform der erfindungsgemäßen vernetzten Polyurethane sind solche, die zusätzlich als Komponente F) mindestens ein Polysiloxan mit wenigstens zwei aktiven Wasserstoffatomen pro Molekül eingebaut enthalten.

Im Rahmen der vorliegenden Erfindung umfasst der Ausdruck Alkyl geradkettige und verzweigte Alkylgruppen. Geeignete kurzkettige Alkylgruppen sind z. B. geradkettige oder verzweigte C₁-C₆-Alkyl-, bevorzugt C₁-C₆-Alkyl- und besonders bevorzugt C₁-C₄-Alkylgruppen. Dazu zählen insbesondere Methyl, Ethyl, Propyl, Isopropyl, n-Butyl, 2-Butyl, sec.-Butyl, tert-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methytpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl,1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, Octyl. Geeignete längerkettige C₈-C₃₀-Alkyl- bzw. C₈-C₃₀-Alkenylgruppen sind geradkettige und verzweigte Alkyl- bzw. Alkenylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen, die gegebenenfalls zusätzlich einfach, zweifach oder mehrfach ungesättigt sein können. Dazu zählen z. B. n-Hexyl(en), n-Heptyl(en), n-Octyl(en), n-Nonyl(en), n-Decyl(en), n-Undecyl(en), n-Dodecyl(en), n-Tridecyl(en), n-Tetradecyl(en), n-Pentadecyl(en), n-Hexadecyl(en), n-Heptadecyl(en), n-Octadecyl(en), n-Nonadecyl(en).

Cycloalkyl steht vorzugsweise für C₅-C₈-Cycloalkyl, wie Cyclopentyl, Cyclohexyl, Cycloheptyl oder Cyclooctyl.

Aryl umfasst unsubstituierte und substituierte Arylgruppen und steht vorzugsweise für Phenyl, Tolyl, Xylyl, Mesityl, Naphthyl, Fluorenyl, Anthracenyl, Phenanthrenyl, Naphthacenyl und insbesondere für Phenyl, Tolyl, Xylyl oder Mesityl.

Im Folgenden werden Verbindungen, die sich von Acrylsäure und Methacrylsäure ableiten können teilweise verkürzt durch Einfügen der Silbe "(meth)" in die von der Acrylsäure abgeleitete Verbindung bezeichnet.

Polymere auf Basis der erfindungsgemäßen vernetzten Polyurethane lassen sich unter Normalbedingungen (20 °C) vorteilhaft als Gele formulieren. "Gelförmige Konsistenz" zeigen Mittel, die eine höhere Viskosität als eine Flüssigkeit aufweisen und die selbsttragend sind, d. h. die eine ihnen verliehene Form ohne formstabilisierende Umhüllung behalten. Im Gegensatz zu festen Formulierungen lassen sich gelförmige Formulierungen jedoch leicht unter Anwendung von Scherkräften deformieren. Die Viskosität der gelförmigen Mittel liegen vorzugsweise in einem Bereich von größer als 600 bis etwa 60000 mPas. Vorzugsweise handelt es sich bei den Gelen um Haargele, wobei diese eine Viskosität von vorzugsweise 6000 bis 30000 mPas aufweisen.

Unter wasserlöslichen Monomeren und Polymeren werden im Rahmen der vorliegenden Erfindung Monomere und Polymere verstanden, die sich zu mindestens 1 g/l bei 20 °C in Wasser lösen. Hydrophile Monomere sind wasserlöslich oder zumindest wasserdispergierbar. Unter wasserdispergierbaren Polymeren werden Polymere verstanden, die unter Anwendung von Scherkräften beispielsweise durch Rühren in dispergierbare Partikel zerfallen. Die erfindungsgemäßen vernetzten Polyurethane sind vorzugsweise wasserlöslich oder wasserdispergierbar. Die erfindungsgemäßen wasserdispergierbaren Polyurethane eignen sich im Allgemeinen zur Herstellung von Mikrodispersionen.

### Komponente A)

Die Komponente A) ist vorzugsweise ausgewählt unter Polytetrahydrofuranen der allgemeinen Formel 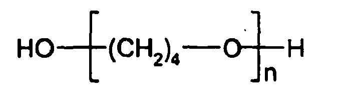 mit n = 4 bis 40, vorzugsweise 6 bis 35,
und Gemischen davon.

Diese Polytetrahydrofurane weisen ein zahlenmittleres Molekulargewicht im Bereich von 650 bis 2000, bevorzugt 750 bis 1800, insbesondere 800 bis 1500 auf.

Geeignete Polytetrahydrofurane können durch kationische Polymerisation von Tetrahydrofuran in Gegenwart von sauren Katalysatoren, wie z. B. Schwefelsäure oder Fluoroschwefelsäure, hergestellt werden. Derartige Herstellungsverfahren sind dem Fachmann bekannt.

### Komponente B)

Bei Komponente B) handelt es sich um Verbindungen, die mehr als zwei aktive Wasserstoffatome pro Molekül enthalten. Geeignet als Komponente B) sind Verbindungen, die mehr als zwei gegenüber NCO-Gruppen reaktive Gruppen aufweisen, die vorzugsweise ausgewählt sind unter Hydroxylgruppen, primären und sekundären Amingruppen.

Vorzugsweise sind die Verbindungen der Komponente B) ausgewählt unter Triolen, Polyolen mit mehr als drei Hydroxylgruppen, Triaminen, Polyaminen mit mehr als drei primären oder sekundären Aminogruppen, Amin- und/oder Hydroxylgruppen-haltigen Polymeren und Mischungen davon.

Bevorzugt geeignet als Komponente B) sind Verbindungen mit einem Molekulargewicht im Bereich von etwa 80 bis weniger als 1000 g/mol und mit etwa 3 bis 20, besonders bevorzugt 3 bis 10, insbesondere 3 bis 5 gegenüber NCO-Gruppen reaktiven Gruppen.

Des Weiteren bevorzugt geeignet als Komponente B) sind Polymere mit einem zahlenmittleren Molekulargewicht von mindestens 1000 und die eine Hydroxylzahl von mindestens 0,1 g KOH/g und/oder eine Aminzahl von mindestens 0,1 g KOH/g aufweisen. Dazu zählen vorzugsweise Hydroxylgruppen-haltige Polymere, die eine OH-Zahl im Bereich von 0,3 bis 60, besonders bevorzugt von 0,9 bis 30 und insbesondere von 1,5 bis 21 aufweisen. Dazu zählen weiterhin Amingruppen-haltige Polymere, die eine Aminzahl im Bereich von 0,3 bis 60, besonders bevorzugt von 0,9 bis 30 und insbesondere von 1,5 bis 21 aufweisen. Dazu zählen weiterhin Hydroxyl- und Amingruppen-haltige Polymere, bei denen die Summe aus Hydroxyl- und Aminzahl in einem Bereich von 0,3 bis 60, bevorzugt von 0,9 bis 30 und insbesondere von 1,5 bis 21, liegt.

Bevorzugt sind die Verbindungen der Komponente B) ausgewählt unter Triolen und höherwertigen Polyolen mit 3 bis 100, bevorzugt 3 bis 70 Kohlenstoffatomen. Bevorzugte Triole sind z. B. Glycerin und Trimethylolpropan. Bevorzugte Triole B) sind weiterhin die Triester von Hydroxycarbonsäuren mit dreiwertigen Alkoholen. Vorzugsweise handelt es sich dabei um Triglyceride von Hydroxycarbonsäuren, wie z. B. Milchsäure, Hydroxystearinsäure und Ricinolsäure. Geeignet sind auch natürlich vorkommende Gemische, die Hydroxycarbonsäuretriglyceride enthalten, insbesondere Ricinusöl. Bevorzugte höherwertige Polyole B) sind z. B. Erythrit, Pentaerythrit und Sorbit.

Bevorzugte Triamine B) sind z. B. Diethylentriamin, N,N'-Diethyldiethylentriamin. Bevorzugte höherwertige Polyamine sind z. B. Triethylentetramin sowie α,ω-Diamihopolyether, die durch Aminierung von Polyalkylenoxiden mit Ammoniak herstellbar sind.

Weitere bevorzugte Verbindungen der Komponente B) sind Hydroxyl- und/oder Amingruppen-haltige Polymere, die ein zahlenmittleres Molekulargewicht von etwa 1000 bis 200000, bevorzugt etwa 5000 bis 100000 aufweisen. Besonders bevorzugt handelt es sich um Amingruppen-haltige Polymere.

In einer bevorzugten Ausführung handelt es sich bei der Komponente B) um ein Amin-und/oder Hydroxylgruppen-haltiges Polymer, das durch radikalische Polymerisation α,β-ethylenisch ungesättigter Monomere erhältlich ist. Dabei wird wenigstens ein α,β-ethylenisch ungesättigtes Monomer eingesetzt, das wenigstens eine funktionelle Gruppe aufweist, die ausgewählt ist unter Hydroxylgruppen, primären oder sekundären Amingruppen oder in solche Amingruppen überführbare Gruppen. Vorzugsweise ist dieses Monomer ausgewählt unter Estern α,β-ethylenisch ungesättigter Mono- und Dicarbonsäuren mit Aminoalkoholen, die wenigstens eine primäre oder sekundäre Aminogruppe aufweisen, Amiden α,β-ethylenisch ungesättigter Mono- und Dicarbonsäuren mit Diaminen, die wenigstens eine primäre oder sekundäre Aminogruppe aufweisen, Estern α,β-ethylenisch ungesättigter Mono- und Dicarbonsäuren mit Diolen, Amiden α,β-ethylenisch ungesättigter Mono- und Dicarbonsäuren mit Aminoalkoholen, die eine primäre oder sekundäre Aminogruppe aufweisen, N,N-Diallylamin und Mischungen davon (= Monomer a)).

Die Amingruppen-haltigen Polymere enthalten vorzugsweise 0,1 bis 20 Gew.%, besonders bevorzugt 0,3 bis 10 Gew.-%, insbesondere 0,5 bis 7 Gew.%, speziell höchstens 4 Gew.-%, bezogen auf das Gesamtgewicht der zur Polymerisation eingesetzten Monomere, wenigstens eines Monomers a) einpolymerisiert.

Geeignete Verbindungen a) sind z. B. die Ester von α,β-ethylenisch ungesättigten Mono- und Dicarbonsäuren mit Aminoalkoholen, vorzugsweise C₂-C₁₂-Aminoalkoholen. Diese können vorzugsweise am Aminstickstoff C₁-C₈-monoalkyliert sein. Als Säurekomponente dieser Ester eignen sich z. B. Acrylsäure, Methacrylsäure, Fumarsäure, Maleinsäure, Itaconsäure, Crotonsäure, Maleinsäureanhydrid, Monobutylmaleat und Gemische davon. Bevorzugt werden Acrylsäure, Methacrylsäure und deren Gemische eingesetzt. Bevorzugt sind N-Methylaminoethyl(meth)acrylat, N-Ethylaminoethyl(meth)acrylat, N-(n-Propyl)aminoethyl(meth)acrylat, N-(n-Butyl)aminoethyl(meth)acrylat, tert.-Butylaminoethyl(meth)acrylat. Besonders bevorzugt ist N-tert.-Butylaminoethylmethacrylat.

Geeignete Monomere a) sind weiterhin die Amide der zuvor genannten α,β-ethylenisch ungesättigten Mono- und Dicarbonsäuren mit Diaminen, welche mindestens eine primäre oder sekundäre Aminogruppe aufweisen.

Geeignet als Monomere a) sind z. B. N-Methylaminoethyl(meth)acrylamid,
N-Ethylaminoethyl(meth)acrylamid, N-(n-Propyl)aminoethyl(meth)acrytamid,
N-(n-Butyl)aminoethyl(meth)acrylamid und N-tert.-Butylaminoethyl(meth)acrylamid.

Geeignete Monomere a) sind weiterhin 2-Hydroxyethylacrylat,
2-Hydroxyethylmethacrylat, 2-Hydroxyethylethacrylat, 2-Hydroxypropylacrylat,
2-Hydroxypropylmethacrylat, 3-Hydroxypropylacrylat, 3-Hydroxypropylmethacrylat,
3-Hydroxybutylacrylat, 3-Hydroxybutylmethacrylat, 4-Hydroxybutylacrylat,
4-Hydroxybutylmethacrylat, 6-Hydroxyhexylacrylat, 6-Hydroxyhexylmethacrylat,
3-Hydroxy-2-ethylhexylacrylat und 3-Hydroxy-2-ethylhexylmethacrylat.

Geeignete Monomere a) sind weiterhin 2-Hydroxyethylacrylamid,
2-Hydroxyethylmethacrylamid, 2-Hydroxyethylethacrylamid, 2-Hydroxypropylacrylamid, 2-Hydroxypropylmethacrylamid, 3-Hydroxypropylacrylamid,
3-Hydroxypropylmethacrylamid, 3-Hydroxybutylacrylamid,
3-Hydroxybutylmethacrylamid, 4-Hydroxybutylacrylamid,
4-Hydroxybutylmethacrylamid, 6-Hydroxyhexylacrylamid,
6-Hydroxyhexylmethacrylamid, 3-Hydroxy-2-ethylhexylacrylamid und
3-Hydroxy-2-ethylhexylmethacrylamid.

Ein bevorzugtes Monomer a) ist weiterhin N,N-Diallylamin.

Bevorzugt als Monomere a) sind tert.-Butylaminoethylmethacrylat, Diallylamin und Mischungen davon.

Die Amingruppen- und/oder Hydroxylgruppen-haltigen Polymere können zusätzlich wenigstens ein weiteres von den zuvor genannten Monomeren a) verschiedenes, damit copolymerisierbares, nichtionisches, Amidgruppen-haltiges, α,β-ethylenisch ungesättigtes Monomer einpolymerisiert enthalten (= Monomer b)). Dabei handelt es sich vorzugsweise um wasserlösliche Monomere.

Vorzugsweise enthalten die Amingruppen-haltigen Polymere 0 bis 80 Gew.%, besonders bevorzugt 1 bis 75 Gew.-%, insbesondere 10 bis 70 Gew.-%, bezogen auf das Gesamtgewicht der zur Polymerisation eingesetzten Monomere, wenigstens eines Monomers b) einpolymerisiert.

Vorzugsweise ist diese Komponente b) ausgewählt ist unter N-Vinyllactamen, N-Vinylamiden gesättigter C₁-C₈-Monocarbonsäuren, primären Amiden α,β-ethylenisch ungesättigter Monocarbonsäuren und deren N-Alkyl- und N,N-Dialkylderivaten, und Mischungen davon.

Bevorzugt enthalten die Amingruppen-haltigen Polymere zusätzlich wenigstens ein N-Vinyllactam b) einpolymerisiert. Als Monomere b) eignen sich unsubstituierte N-Vinyllactame und N-Vinyllactamderivate, die z. B. einen oder mehrere C₁-C₆-Alkylsubstituenten, wie Methyl, Ethyl, n-Propyl, Isopropyl, n-Butyl, sec.-Butyl, tert.-Butyl, aufweisen können. Dazu zählen z. B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam. Bevorzugt werden N-Vinylpyrrolidon und/oder N-Vinylcaprolactam eingesetzt.

Als Monomere b) geeignete offenkettige N-Vinylamidverbindungen sind beispielsweise N-Vinylformamid, N-Vinyl-N-methylformamid, N-Vinylacetamid, N-Vinyl-N-methylacetamid, N-Vinyl-N-ethylacetamid, N-Vinylpropionamid, N-Vinyl-N-methylpropionamid und N-Vinylbutyramid.

Geeignete Monomere b) sind weiterhin Acrylsäureamid und Methacrylsäureamid.

Geeignete Monomere b) sind weiterhin N-C₁-C₈-Alkyl- und N,N-Di-(C₁-C₈-)alkylamide α,β-ethylenisch ungesättigter Monocarbonsäuren. Geeignete zusätzliche Monomere c) sind N-Methyl(meth)acrylamid, N-Ethyl(meth)acrylamid, N-Propyl(meth)acrylamid, N-Isopropyl(meth)acrylamid, N-(n-Butyl)(meth)acrylamid, N-(tert.-Butyl)(meth)acrylamid, N,N-Dimethyl(meth)acrylamid, N,N-Diethyl(meth)acrylamid, Piperidinyl(meth)acrylamid und Morpholinyl(meth)acrylamid.

Geeignete Monomere b) sind weiterhin N-(n-Octyl)(meth)acrylamid,
N-(1,1,3,3-Tetramethylbutyl)(meth)acrylamid, N-Ethylhexyl(meth)acrylamid,
N-(n-Nonyl)(meth)acrylamid, N-(n-Decyl)(meth)acrylamid,
N-(n-Undecyl)(meth)acrylamid, N-Tridecyl(meth)acrylamid, N-Myristyl(meth)acrylamid,
N-Pentadecyl(meth)acrylamid, N-Palmityl(meth)acrylamid,
N-Heptadecyl(meth)acrylamid, N-Nonadecyl(meth)acrylamid,
N-Arrachinyl(meth)acrylamid, N-Behenyl(meth)acrylamid,
N-Lignocerenyl(meth)acrylamid, N-Cerotinyl(meth)acrylamid,
N-Melissinyl(meth)acrylamid, N-Palmitoleinyl(meth)acrylamid,
N-Oleyl(meth)acrylamid, N-Linolyl(meth)acrylamid, N-Linolenyl(meth)acrylamid,
N-Stearyl(meth)acrylamid, N-Lauryl(meth)acrylamid.

Die Amingruppen-haltigen Polymere können zusätzlich wenigstens ein, vorzugsweise wasserlösliches, Monomer einpolymerisiert enthalten, das ausgewählt ist unter α,β-ethylenisch ungesättigten Verbindungen mit anionogenen und/oder anionischen Gruppen (= Monomer c)). Vorzugsweise enthalten die Amingruppen-haltigen Polymere 0 bis 30 Gew.-%, besonders bevorzugt 0,1 bis 20 Gew.-%, insbesondere 0,5 bis 15 Gew.-%, bezogen auf das Gesamtgewicht der zur Polymerisation eingesetzten Monomere, wenigstens eines Monomers c) einpolymerisiert.

Vorzugsweise sind die anionogenen/anionischen Monomere c) ausgewählt unter monoethylenisch ungesättigten Carbonsäuren, Sulfonsäuren, Phosphonsäuren und Mischungen davon.

Zu den Monomeren c) zählen monoethylenisch ungesättigte Mono- und Dicarbonsäuren mit 3 bis 25 vorzugsweise 3 bis 6 C-Atomen, die auch in Form ihrer Salze oder Anhydride eingesetzt werden können. Beispiele hierfür sind Acrylsäure, Methacrylsäure, Ethacrylsäure, α-Chloracrylsäure, Crotonsäure, Maleinsäure, Maleinsäureanhydrid, Itaconsäure, Citraconsäure, Mesaconsäure, Glutaconsäure, Aconitsäure und Fumarsäure. Zu den Monomeren c) zählen weiterhin die Halbester von monoethylenisch ungesättigten Dicarbonsäuren mit 4 bis 10 vorzugsweise 4 bis 6 C-Atomen, z. B. von Maleinsäure wie Maleinsäuremonomethylester. Zu den Monomeren c) zählen auch monoethylenisch ungesättigte Sulfonsäuren und Phosphonsäuren, beispielsweise Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Styrolsulfonsäure, 2-Acrylamido-2-methylpropansulfonsäure, Vinylphosphonsäure und Allylphosphonsäure. Zu den Monomeren c) zählen auch die Salze der zuvor genannten Säuren, insbesondere die Natrium-, Kalium- und Ammoniumsalze sowie die Salze mit den zuvor genannten Aminen.

Die Monomere c) können als solche oder als Mischungen untereinander eingesetzt werden. Die angegebenen Gewichtsanteile beziehen sich sämtlich auf die freie Basenform oder die freie Säureform.

Besonders bevorzugt als Monomer c) sind Acrylsäure, Methacrylsäure und Mischungen davon.

Die Aminogruppen- und/oder Hydroxylgruppen-haltigen Polymere können zusätzlich wenigstens ein weiteres, von den Amingruppen-haltigen Monomeren a) verschiedenes, Monomer einpolymerisiert enthalten, das ausgewählt ist unter α,β-ethylenisch ungesättigten Verbindungen mit kationogenen und/oder kationischen Gruppen (= Monomer d)). Vorzugsweise enthalten die Polymere 0 bis 30 Gew.%, besonders bevorzugt 0,1 bis 20 Gew.-%, insbesondere 0,5 bis 15 Gew.-%, bezogen auf das Gesamtgewicht der zur Polymerisation eingesetzten Monomere, wenigstens eines Monomers d) einpolymerisiert.

Bevorzugt handelt es sich bei den kationogenen und/oder kationischen Gruppen der Komponente d) um stickstoffhaltige Gruppen, wie tertiäre Aminogruppen sowie quaternäre Ammoniumgruppen. Geladene kationische Gruppen lassen sich aus Aminstickstoffen entweder durch Protonierung, z. B. mit Carbonsäuren, wie Milchsäure, oder Mineralsäuren, wie Phosphorsäure, Schwefelsäure und Salzsäure, oder durch Quaternisierung, z. B. mit Alkylierungsmitteln, wie C₁-C₄-Alkylhalogeniden oder -sulfaten, erzeugen. Beispiele solcher Alkylierungsmittel sind Ethylchlorid, Ethylbromid, Methylchlorid, Methylbromid, Dimethylsulfat und Diethylsulfat.

Geeignet als Monomere d) sind z. B. N,N-Dialkylaminoalkyl(meth)acrylate, wie
N,N-Dimethylaminomethyl(meth)acrylat, N,N-Dimethylaminoethyl(meth)acrylat,
N,N-Diethylaminoethyl(meth)acrylat, N,N-Dimethylaminopropyl(meth)acrylat,
N,N-Diethylaminopropyl(meth)acrylat, N,N-Dimethylaminocyclohexyl(meth)acrylat.

Geeignet als Monomere d) sind weiterhin N,N-Dialkylaminoalkyl(meth)acrylamide, wie
N-[2-(dimethylamino)ethyl]acrylamid, N-[2-(dimethylamino)ethyl]methacrylamid,
N-[3-(dimethylamino)propyl]acrylamid, N-[3-(dimethylamino)propyl]methacrylamid,
N-[4-(dimethylamino)butyl]acrylamid, N-[4-(dimethylamino)butyl]methacrylamid,
N-[2-(diethylamino)ethyl]acrylamid, N-[2-(diethylamino)ethyl]methacrylamid,
N-[4-(dimethylamino)cyclohexyl]acrylamid und
N-[4-(dimethylamino)cyclohexyl]methacrylamid. Bevorzugt sind
N,N-Dimethylaminopropylacrylat, N,N-Dimethylaminopropylmethacrylat,
N-[3-(dimethylamio)propyl]acrylamid und
N-[3-(dimethylamio)propy]methacrylamid.

Geeignete Monomere d) sind weiterhin vinyl- und allylsubstituierte Stickstoffheterocyclen, wie Vinylimidazol, N-Vinyl-2-alkylimidazole, z. B. N-Vinyl-2-methylimidazol sowie 2-und 4-Vinylpyridin, 2- und 4-Allylpyridin, und die Salze davon.

Geeignete Monomere d) sind außerdem Alkylallylamine und Allylammoniumsalze, wie Diallylmethylamin und Diallyldimethylammoniumchlorid (DADMAC).

Bevorzugt als Monomer d) ist N-Vinylimidazol.

Werden zur Herstellung der Amin- und/oder Hydroxylgruppen-haltigen Polymere Monomere mit kationogenen/kationischen Gruppen, die ausgewählt sind unter den entsprechenden Monomeren a) und/oder d), und anionogene/anionische Monomere c) eingesetzt, so können diese Monomere zumindest teilweise gemeinsam in Form so genannter _{"}Salzpaare" zur Polymerisation eingesetzt werden. Bevorzugte Kombinationen der Komponenten a) und/oder d) mit c), die z. B. als Salzpaar zur radikalischen Polymerisation eingesetzt werden können, sind (Meth)acrylsäure/N-tert.-Butylaminoethyl(meth)acrylat und (Meth)acrylsäure/Vinylimidazol. Im Allgemeinen beträgt der Anteil der in Form von "Salzpaaren" eingesetzten Monomere 0 bis 50 Gew.-%, insbesondere 0,1 bis 40 Gew.%, bezogen auf das Gesamtgewicht der zur Polymerisation eingesetzten Monomere. Der Einsatz von "Salzpaaren" hat sich insbesondere bewährt, wenn die Amino- und/oder Hydroxylgruppen-haltigen Polymere Monomere b) einpolymerisiert enthalten, die ausgewählt sind unter Vinylpyrrolidon, Vinylcaprolactam, Vinylformamid und Mischungen davon.

Die Amingruppen- und/oder Hydroxylgruppen-haltigen Polymere können zusätzlich wenigstens ein weiteres Monomer e) einpolymerisiert enthalten, das vorzugsweise ausgewählt ist unter Estern α,β-ethylenisch ungesättigter Mono- und Dicarbonsäuren mit C₁-C₃₀-Alkanolen, Estern von Vinylalkohol und Allylalkohol mit C₁-C₃₀-Monocarbonsäuren, Vinylethern, Vinylaromaten, Vinylhalogeniden, Vinylidenhalogeniden, C₁-C₈-Monoolefinen, nicht aromatischen Kohlenwasserstoffen mit mindestens zwei konjugierten Doppelbindungen und Mischungen davon.

Vorzugsweise enthalten die Amingruppen- und/oder Hydroxylgruppen-haltigen Polymere bis zu 50 Gew.%, besonders bevorzugt bis zu 30 Gew.% und insbesondere bevorzugt bis zu 15 Gew.%, bezogen auf das Gesamtgewicht der zur Polymerisation eingesetzten Monomere, wenigstens eines Monomers e) einpolymerisiert. Wenn zur Polymerisation wenigstens ein Monomer e) eingesetzt wird, dann bevorzugt in einer Menge von mindestens 0,1 Gew.%, besonders bevorzugt von mindestens 1 Gew.-%.

Geeignete Monomere e) sind dann Methyl(meth)acrylat, Methylethacrylat, Ethyl(meth)acrylat, Ethylethacrylat, n-Butyl(meth)acrylat, tert.-Butyl(meth)acrylat, tert.-Butylethacrylat, n-Octyl(meth)acrylat, 1,1,3,3-Tetramethylbutyl(meth)acrylat,
Ethylhexyl(meth)acrylat, n-Nonyl(meth)acrylat, n-Decyl(meth)acrylat,
n-Undecyl(meth)acrylat, Tridecyl(meth)acrylat, Myristyl(meth)acrylat,
Pentadecyl(meth)acrylat, Palmityl(meth)acrylat, Heptadecyl(meth)acrylat,
Nonadecyl(meth)acrylat, Arrachinyl(meth)acrylat, Behenyl(meth)acrylat,
Lignocerenyl(meth)acrylat, Cerotinyl(meth)acrylat, Melissinyl(meth)acrylat,
Palmitoteinyl(meth)acrylat, Oleyl(meth)acrylat, Linolyl(meth)acrylat,
Linolenyl(meth)acrylat, Stearyl(meth)acrylat, Lauryl(meth)acrylat und Mischungen davon. Bevorzugte Monomere e) sind die Ester α,β-ethylenisch ungesättigter Mono- und Dicarbonsäuren mit C₁-C₄-Alkanolen.

Geeignete Monomere e) sind weiterhin Vinylacetat, Vinylpropionat, Vinylbutyrat und Mischungen davon.

Geeignete Monomere e) sind weiterhin Ethylen, Propylen, Isobutylen, Butadien, Styrol, α-Methylstyrol. Acrylnitril, Methacrylnitril, Vinylchlorid, Vinylidenchlorid, Vinylfluorid, Vinylidenfluorid und Mischungen davon.

Die zuvor genannten Monomere e) können einzeln oder in Form von beliebigen Mischungen eingesetzt werden.

Besonders bevorzugt sind Amingruppen-haltige Polymere, die
- N-tert.-Butylaminoethylmethacrylat
- Vinylimidazol
- Vinylpyrrolidon und
- Methacrylsäure
einpolymerisiert enthalten.

Besonders bevorzugt sind Amingruppen-haltige Polymere, die
- 0,1 bis 10 Gew.-% N-tert.-Butylaminoethylmethacrylat
- 50 bis 99,9 Gew.% Vinylpyrrolidon,
- 0 bis 40 Gew.% Methacrylsäure und Vinylimidazol (Molverhältnis 1:1) einpolymerisiert enthalten.

Die Herstellung der Aminogruppen-haltigen Polymere erfolgt nach üblichen, dem Fachmann bekannten Verfahren, z. B. durch Lösungs-, Fällungs-, Suspensions- oder Emulsionspolymerisation. Bevorzugt ist die Herstellung durch Lösungs- oder Fällungspolymerisation.

Bevorzugte Lösemittel zur Lösungspolymerisation sind wässrige Lösungsmittel, wie Wasser, wassermischbare Lösungsmittel und Gemische aus Wasser mit wassermischbaren Lösungsmitteln, beispielsweise Ketone, wie Aceton und Methylethylketon, Alkohole, wie Methanol, Ethanol, n-Propanol, Isopropanol, n-Butanol, sek.-Butanol, tert.-Butanol, n-Hexanol und Cyclohexanol sowie Glykole, wie Ethylenglykol, Propylenglykol und Butylenglykol sowie die Methyl- oder Ethylether der zweiwertigen Alkohole, Diethylenglykol, Triethylenglykol, Polyethylenglykolen mit zahlenmittleren Molekulargewichten bis etwa 3000, Glycerin und Dioxan.

Die Fällungspolymerisation erfolgt beispielsweise in einem Ester, wie Essigsäureethylester oder Essigsäurebutylester als Lösungsmittel. Die resultierenden Polymerteilchen fallen aus der Reaktionslösung aus und können durch übliche Verfahren, wie Filtration mittels Unterdruck, isoliert werden. Bei der Fällungspolymerisation werden in der Regel Polymere mit höheren Molekulargewichten als bei der Lösungspolymerisation erhalten.

Die Polymerisationstemperaturen liegen vorzugsweise in einem Bereich von etwa 30 bis 120 °C, besonders bevorzugt 40 bis 100 °C. Die Polymerisation erfolgt üblicherweise unter atmosphärischem Druck, sie kann jedoch auch unter vermindertem oder erhöhtem Druck ablaufen. Ein geeigneter Druckbereich liegt zwischen 1 und 5 bar.

Zur Herstellung der Polymere können die Monomeren mit Hilfe von Radikale bildenden Initiatoren polymerisiert werden.

Als Initiatoren für die radikalische Polymerisation können die hierfür üblichen Peroxo-und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azobisisobutyronitril, 2,2'-Azobis(2-amidinopropan)hydrochloride (V50 von Wako Pure Chemicals Industries, Ltd.), oder 2,2'-Azobis(2-methyl-butyronitril). Geeignet sind auch Initiatormischungen oder Redox-Initiator-Systeme, wie z. B. Ascorbinsäure/Eisen(II)sulfat/Natriumperoxodisulfat, tert.-Butylhydroperoxid/Natriumdisulfit, tert.-Butylhydroperoxid/Natriumhydroxymethansulfinat, H₂O₂/Cu^{l}.

Zur Einstellung des Molekulargewichts kann die Polymerisation in Gegenwart wenigstens eines Reglers erfolgen. Als Regler können die üblichen, dem Fachmann bekannten Verbindungen, wie z. B. Schwefelverbindungen, z. B. Mercaptoethanol, ₂-Ethylhexylthioglycolat, Thioglycolsäure oder Dodecylmercaptan sowie Tribromchlormethan oder andere Verbindungen, die regelnd auf das Molekulargewicht der erhaltenen Polymerisate wirken, eingesetzt werden. Ein bevorzugter Regler ist Cystein.

### Komponente C)

Bevorzugte Verbindungen C) mit zwei aktiven Wasserstoffatomen und mindestens einer anionogenen und/oder anionischen Gruppe pro Molekül sind z. B. Verbindungen mit Carboxylat-, Sulfonat- und/oder Phosphatgruppen. Als Komponente C) sind 2,2-Hydroxymethylalkylcarbonsäuren, wie Dimethylolpropansäure, und Mischungen, die 2,2-Hydroxymethylalkylcarbonsäuren, wie Dimethylolpropansäure, enthalten, besonders bevorzugt.

Geeignete Diamine und/oder Diole C) mit anionogenen oder anionischen Gruppen sind Verbindungen der Formel 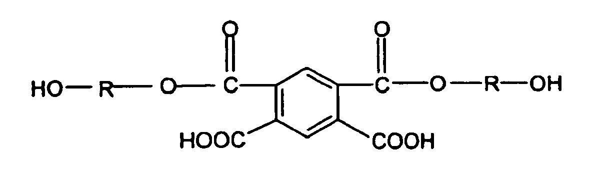 und/oder 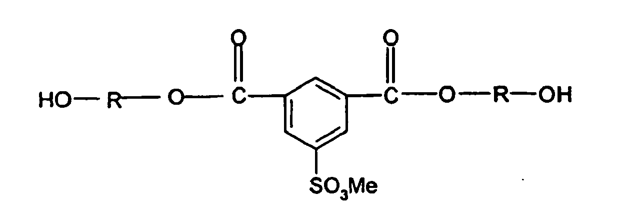 worin R jeweils für eine C₂-C₁₈-Alkylengruppe steht und Me für Na oder K steht.

Als Komponente C) brauchbar sind auch Verbindungen der Formel

H₂N(CH₂)_{w}-NH-(CH₂)ₓ-COO⁻M⁺

H₂N(CH₂)_{w}-NH-(CH₂)ₓ-SO₃⁻M⁺

worin w und x unabhängig voneinander für eine ganze Zahl von 1 bis 8, insbesondere 1 bis 6, stehen und M für Li, Na oder K steht, und Verbindungen der Formel

H₂N(CH₂CH₂O)_{y}(CH₂CH(CH₃)O)_{z}(CH₂)_{w}-NH-(CH₂)ₓ-SO₃⁻M⁺

worin w und x die zuvor angegebenen Bedeutungen besitzen, y und z unabhängig voneinander für eine ganze Zahl von 0 bis 50 stehen, wobei wenigstens eine der beiden Variablen y oder z > 0 ist. Die Reihenfolge der Alkylenoxideinheiten ist dabei beliebig. Die zuletzt genannten Verbindungen weisen vorzugsweise ein zahlenmittleres Molekulargewicht im Bereich von etwa 400 bis 3000 auf. Eine geeignete Verbindung dieses Typs ist z. B. Poly ESP 520 der Fa. Raschig.

### Komponente D)

Die erfindungsgemäßen vernetzten Polyurethane enthalten als Komponente D) ein Polyisocyanatgemisch, das Isophorondiisocyanat und Hexamethylendiisocyanat enthält. Zusätzlich kann das Polyisocyanatgemisch wenigstens ein weiteres Polyisocyanat enthalten. Vorzugsweise enthält die Komponente D), bezogen auf das Gesamtgewicht der eingesetzten Polyisocyanate, 0 bis 30 Gew.-%, besonders bevorzugt 0,1 bis 15 Gew.%, insbesondere 0,5 bis 5 Gew.-%, wenigstens eines weiteren Polyisocyanats.

Geeignete weitere Polyisocyanate D) sind ausgewählt unter Verbindungen mit 2 bis 5 Isocyanatgruppen, Isocyanatpräpolymeren mit mittleren Anzahl von 2 bis 5 Isocyanatgruppen, und Mischungen davon. Dazu zählen z. B. aliphatische, cycloaliphatische und aromatische Di-, Tri- und Polyisocyanate. Geeignete Diisocyanate D) sind z. B. Tetramethylendiisocyanat, 2,3,3-Trimethylhexamethylendiisocyanat, 1,4-Cyclohexylendiisocyanat, 1,4-Phenylendiisocyanat, 2,4- und 2,6-Toluylendüsocyanat und deren Isomerengemische (z. B. 80 % 2,4- und 20 % 2,6-Isomer), 1,5-Naphthylendiisocyanat, 2,4- und 4,4'-Diphenylmethandiisocyanat. Ein geeignetes Triisocyanat ist z. B. Triphenylmethan-4,4',4"-triisocyanat. Weiterhin geeignet sind Isocyanatpräpolymere und Polyisocyanate, die durch Addition der zuvor genannten Isocyanate an polyfunktionelle Hydroxyl- oder Amingruppen-haltige Verbindungen erhältlich sind. Weiterhin geeignet sind Polyisocyanate, die durch Biuret-, Allophanat- oder Isocyanuratbildung entstehen.

Besonders bevorzugt umfasst die Komponente D) ausschließlich Isophorondiisocyanat, Hexamethylendiisocyanat, und deren Biurete, Allophanate und/oder Isocyanurate.

Insbesondere besteht die Komponente D) aus einem Gemisch von Isophorondiisocyanat und Hexamethylendiisocyanat.

Besonders bevorzugt ist in der Komponente D) das Gewichtsmengenverhältnis von Isophorondilsocyanat zu Hexamethylendiisocyanat in einem Bereich von 4:1 bis 10:1.

### Komponente E)

Die erfindungsgemäßen vernetzten Polyurethane enthalten wenigstens eine Verbindung E) eingebaut, die vorzugsweise ausgewählt ist unter
E1) Verbindungen mit einem Molekulargewicht in einem Bereich von 60 bis 286 g/mol, die zwei gegenüber Isocyanatgruppen reaktive Gruppen pro Molekül aufweisen und
E2) Polymerisate mit einem zahlenmittleren Molekulargewicht im Bereich von etwa 300 bis 6000, die zwei gegenüber Isocyanatgruppen reaktive Gruppen pro Molekül enthalten:
und Mischungen davon.

Geeignete Verbindungen E1) sind z. B. Diole, Diamine, Aminoalkohole und Mischungen davon.

Bevorzugt werden als Komponente E1) Diole eingesetzt, deren Molekulargewicht in einem Bereich von etwa 62 bis 286 g/mol liegt. Dazu zählen z. B. Diole mit 2 bis 18 Kohlenstoffatomen, vorzugsweise 2 bis 10 Kohlenstoffatomen, wie 1,2-Ethandiol, 1,3-Propandiol, 1,4-Butandiol, 1,8-Hexandiol, 1,5-Pentandiol, 1,10-Decandiol, 2-Methyl-1,3-propandiol, 2,2-Dimethyl-1,3-propandiol, Di-, Tri-, Tetra-, Penta- und Hexaethylenglykol, Neopentylglykol, Cyclohexandimethylol und Mischungen davon, Besonders bevorzugt ist Neopentylglykol,

Bevorzugte Aminoalkohole E1) sind z. B. 2-Aminoethanol, 2-(N-Methylamino)ethanol,
3-Aminopropanol, 4-Aminobutanol, 1-Ethylaminobutan-2-ol,
2-Amino-2-methyl-1-propanol, 4-Methyl-4-aminopentan-2-ol.

Bevorzugte Diamine E1) sind z. B. Ethylendiamin, Propylendiamin, 1,4-Diaminobutan,
1,5-Diaminopentan und 1,6-Diaminohexan.

Die als Komponente E1) genannten Verbindungen können einzeln oder in Mischungen eingesetzt werden. Besonders bevorzugt werden 1,2-Ethandiol, 1,4-Butandiol, 1,6-Hexandiol, Neopentylglykol, Diethylenglykol, Cyclohexandimethylol und Mischungen davon eingesetzt.

Bei der Komponente E2) handelt es sich bevorzugt um ein Polymerisat mit einem zahlenmittleren Molekulargewicht im Bereich von etwa 300 bis 5000, besonders bevorzugt etwa 400 bis 4000, insbesondere 500 bis 3000. Brauchbare Polymerisate E2) sind z. B. Polyesterdiole, Polyetherole, Polysiloxane und Mischungen davon. Polyetherole sind vorzugsweise Polyalkylenglykole, z.B. Polyethylenglykole, Polypropylenglykole, Copolymerisate aus Ethylenoxid und Propylenoxid oder Blockcopolymerisate aus Ethylenoxid, Propylenoxid und Butylenoxid, die die Alkylenoxideinheiten statistisch verteilt oder in Form von Blöcken einpolymerisiert enthalten. Geeignet sind auch α,ω-Diaminopolyether, die durch Aminierung von Polyalkylenoxiden mit Ammoniak herstellbar sind. Vorzugsweise werden als Komponente E2) Polyesterdiole und Mischungen, die diese enthalten, eingesetzt.

Bevorzugte Polyesterdiole E2) weisen ein zahlenmittleres Molekulargewicht im Bereich von etwa 400 bis 5000, bevorzugt 500 bis 3000, insbesondere 600 bis 2000, auf.

Als Polyesterdiole E2) kommen alle diejenigen in Betracht, die üblicherweise zur Herstellung von Polyurethanen eingesetzt werden, insbesondere solche auf Basis aromatischer Dicarbonsäuren, wie Terephthalsäure, Isophthalsäure, Phthalsäure, Na- oder K-Sulfoisophthalsäure, aliphatischer Dicarbonsäuren, wie Adipinsäure oder Bernsteinsäure, und cycloaliphatischer Dicarbonsäuren, wie 1,2-, 1,3- oder 1,4-Cyclohexandicarbonsäure. Als Diole kommen insbesondere aliphatische Diole in Betracht, wie Ethylenglykol, Propylenglykol, 1,6-Hexandiol, Neopentylglykol, Diethylenglykol, Polyethylenglykole, Polypropylenglykole, 1,4-Dimethylolcyclohexan.

Bevorzugt sind Polyesterdiole E2) auf Basis von aromatischen und aliphatischen Dicarbonsäuren und aliphatischen Diolen, insbesondere solche, bei denen die aromatische Dicarbonsäure 10 bis 95 Mol-%, insbesondere 40 bis 90 Mol% des gesamten Dicarbonsäureanteils (Rest aliphatische Dicarbonsäuren) ausmacht.

Besonders bevorzugte Polyesterdiole E2) sind die Umsetzungsprodukte aus Phthalsäure/Diethylenglykol, Isophthalsäure/1,4-Butandiol, Isophthalsäure/Adipinsäure/1,6-Hexandiol, 5-NaSO₃-Isophthalsäure/Phthalsäure/Adipinsäure/1,6-Hexandiol, Adipinsäure/Ethylenglykol, Isophthalsäure/Adipinsäure/Neopentylglykol, Isophthalsäure/Adipinsäure/Neopentylglykol/Diethylenglykol/Dimethylolcyclohexan und 5-NaSO₃-Isophthalsäure/Isophthalsäure/Adipinsäure/Neopentylglykol/Diethylenglykol/Dimethylolcyclohexan, Isophthalsäure/Adipinsäure, Neopentylglykol/Dimethylolcyclohexan.

Bevorzugt als Komponente E2) sind weiterhin Polyesterdiole auf Basis von linearen oder verzweigten, C₈-C₃₀-Di- oder Polycarbonsäuren und C₈-C₃₀-Hydroxycarbonsäuren. Bevorzugte Carbonsäuren und Hydroxycarbonsäuren sind z. B. Acelainsäure, Dodecandisäure, Korksäure, Pimelinsäure, Sebacinsäure, Tetradecandisäure, Citronensäure, Ricinolsäure, Hydroxystearinsäure und Gemische davon. Als Diolkomponente zur Herstellung dieser Polyesterdiole werden vorzugsweise 1,4-Butandiol, 1,5-Pentandiol, 1,6-Hexandiol, Neopentylglykol, 1,4-Dimethylolcyclohexan, Diethylenglykol und Gemische davon eingesetzt.

### Komponente F)

Bei der Komponente F) handelt es sich bevorzugt um ein Polysiloxan mit einem zahlenmittleren Molekulargewicht im Bereich von etwa 300 bis 100 000, beispielsweise 300 bis 30 000, vorzugsweise 300 bis 10 000, insbesondere 300 bis 4000, speziell 400 bis 3000.

Geeignete Verbindungen der Komponente F) sind Polysiloxane der allgemeinen Formel I 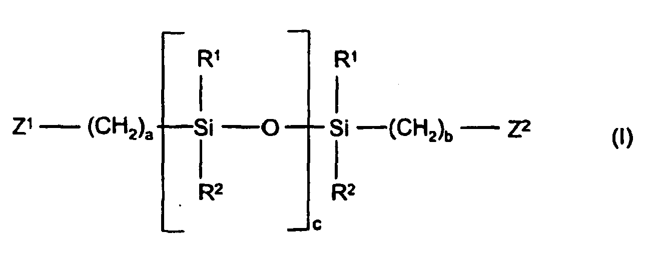 worin
a und b unabhängig voneinander für 1 bis 8, vorzugsweise 2 bis 6, stehen,
c für 2 bis 100, vorzugsweise 3 bis 50, steht,
R¹ und R² unabhängig voneinander für C₁-C₈-Alkyl, Benzyl, C₅-C₈-Cycloalkyl oder Phenyl stehen,
Z¹ und Z² unabhängig voneinander für OH, NHR³ oder einen Rest der Formel II

-(CH₂CH₂O)ᵥ(CH₂CH(CH₃)O)_{w}-H (II)

stehen, wobei
in der Formel 11 die Reihenfolge der Alkylenoxideinheiten beliebig ist und
v und w unabhängig voneinander für eine ganze Zahl von 0 bis 200 stehen, wobei die Summe aus v und w > 0 ist,
R³ für Wasserstoff, C₁-C₈-Alkyl oder C₅-C₈-Cycloalkyl steht.

Bevorzugt sind in den Verbindungen der Formel I die Reste R¹ und R² unabhängig voneinander ausgewählt unter Methyl, Ethyl, Benzyl, Phenyl und Cyclohexyl. Besonders bevorzugt stehen R¹ und R² beide für Methyl.

Nach einer geeigneten Ausführungsform weisen die Polysiloxane F) der allgemeinen Formel I keine Alkylenoxidreste der allgemeinen Formel II auf. Diese Polysiloxane F) weisen dann vorzugsweise ein zahlenmittleres Molekulargewicht im Bereich von etwa 300 bis 10000, bevorzugt 400 bis 5000 auf.

Geeignete Polysiloxane F), die keine Alkylenoxidreste aufweisen sind z. B. die Tegomer®-Marken der Fa. Goldschmidt.

Nach einer weiteren geeigneten Ausführungsform handelt es sich bei den Polysiloxanen F) um Siliconpoly(alkylenoxid)-Copolymere der Formel I, wobei wenigstens einer oder beide Reste Z¹ und/oder Z² für einen Rest der allgemeinen Formel 11 stehen.

Vorzugsweise ist in der Formel II die Summe aus v und w so gewählt, dass das Molekulargewicht der Polysiloxane F) in einem Bereich von etwa 300 bis 30000 liegt.

Bevorzugt liegt die Gesamtzahl der Alkylenoxideinheiten der Polysiloxane F), das heißt die Summe aus v und w in der Formel II in einem Bereich von etwa 3 bis 200, bevorzugt 5 bis 180.

Geeignete Siliconpoly(alkylenoxid)-Copolymere F), die z. B. unter dem internationalen Freinamen Dimethicone bekannt sind, sind die Tegopren®-Marken der Fa. Goldschmidt, Belsil® 6031 und 6032 der Fa. Wacker, Silvet® L der Fa. Witco und Pluriol® ST 4005 der Fa. BASF Aktiengesellschaft.

Geeignete Polysiloxane F) sind auch die in der EP-A-277 816 beschriebenen Hydroxylgruppen-haltigen Polydimethylsiloxane, auf die hier Bezug genommen wird.

Geeignete Verbindungen der Komponente F) sind weiterhin Verbindungen der allgemeinen Formel III 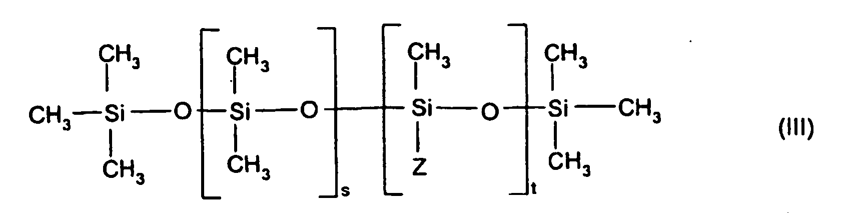 worin
die Reihenfolge der Siloxaneinheiten beliebig ist,
- s: für einen Wert von 5 bis 200, bevorzugt 10 bis 100, steht,
- Z: für einen Rest der Formel (Z-I) = -(CH₂)ᵤ-NH₂ steht, worin u für eine ganze Zahl von 1 bis 10, bevorzugt 2 bis 6 steht,
und/oder
- Z: für einen Rest der Formel (Z-II) = -(CH₂)ₓ-NH-(CH₂)_{y}-NH₂ steht, worin x und y unabhängig voneinander für 0 bis 10, bevorzugt 1 bis 6, stehen, wobei die Summe aus x und y für 1 bis 10, bevorzugt 2 bis 6, steht,
- t: für einen Wert von 3 bis 20, bevorzugt 3 bis 10 steht, falls Z = Z-I ist,
- t: für einen Wert von 2 bis 20, bevorzugt 2 bis 10, steht, falls Z = Z-II ist.

Dazu zählen z. B. die MAN- und MAR-Marken der Fa. Hüls sowie die Finish-Marken der Fa. Wacker, z. B. Finish WT 1270.
Besonders geeignete Polyalkylenoxid-haltige Silikonderivate F) sind solche, die die folgenden Strukturelemente der allgemeinen Formel IV enthalten: 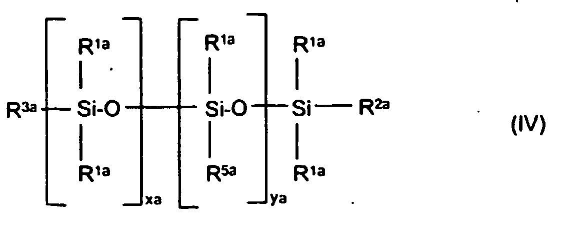 wobei xa und ya ganze Zahlen derart sind, dass das Molekulargewicht des Polysiloxans zwischen 300 und 30000 liegt,
und wobei die Reste R^{1a} identisch oder unterschiedlich sein können, und entweder aus der Gruppe der aliphatischen Kohlenwasserstoffe mit 1 bis 20 Kohlenstoffatomen stammen, cyclische aliphatische Kohlenwasserstoffe mit 3 bis 20 C-Atomen sind, oder aromatischer Natur sind,
und wobei die Reste R^{2a}, R^{3a}, R^{5a} identisch oder unterschiedlich sein können, und entweder aus der Gruppe der aliphatischen Kohlenwasserstoffe mit 1 bis 20 Kohlenstoffatmen stammen, cyclische aliphatische Kohlenwasserstoffe mit 3 bis 20 C-Atomen sind, oder aromatischer Natur oder gleich R^{6a} sind, wobei: 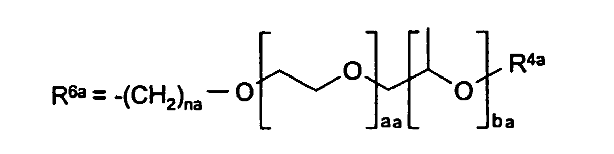 mit der Maßgabe, dass mindestens einer der Reste R^{2a}, R^{3a} oder R^{5a} ein Polyalkylenoxid-haltiger Rest nach obengenannter Definition R^{6a} ist und na eine ganze Zahl von 1 bis 6 ist, insbesondere ist na = 3,
aa, ba ganze Zahlen zwischen 0 und 50 sein können mit der Maßgabe, dass die Summe aus aa und ba größer als 0 ist, wobei 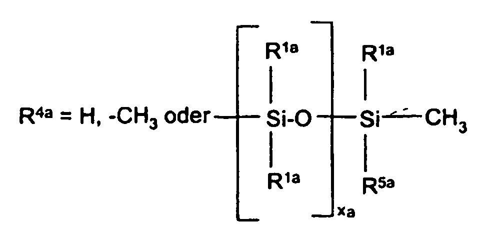 ist.

Bevorzugt werden die Gruppen R^{1a} aus der folgenden Gruppe ausgewählt: Methyl, Ethyl, Propyl, Butyl, Isobutyl, Pentyl, Isopentyl, Hexyl, Octyl, Decyl, Dodecyl und Octadecyl, cycloaliphatische Reste, speziell Cyclohexyl, aromatische Gruppen speziell Phenyl oder Naphthyl, gemischt aromatisch-aliphatische Reste wie Benzyl oder Phenylethyl sowie Tolyl und Xylyl.

Besonders bevorzugt sind Methyl, Ethyl und Phenyl.

Bevorzugt werden die Gruppen R^{2a}, R^{3a} und R^{5a} aus der folgenden Gruppe ausgewählt: Methyl, Ethyl, Propyl, Butyl, Isobutyl, Pentyl, Isopentyl, Hexyl, Octyl, Decyl, Dodecyl und Octadecyl, cycloaliphatische Reste, speziell Cyclohexyl, aromatische Gruppen speziell Phenyl oder Naphthyl, gemischt aromatisch-aliphatische Reste wie Benzyl oder Phenylethyl sowie Tolyl und Xylyl und R⁶.

Bevorzugte Reste R^{6a} sind solche, bei denen die Summe aus aa + ba zwischen 5 und 30 beträgt.

Geeignete Verbindungen der Komponente F) sind weiterhin Verbindungen der allgemeinen Formel V 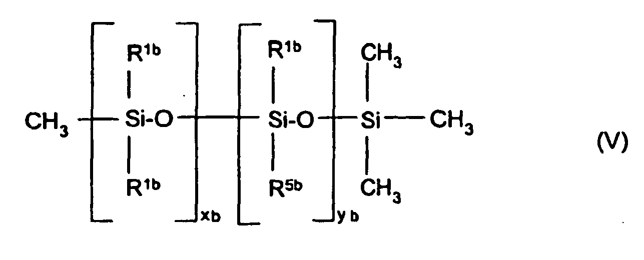 wobei
R^{1b} = -CH₃ 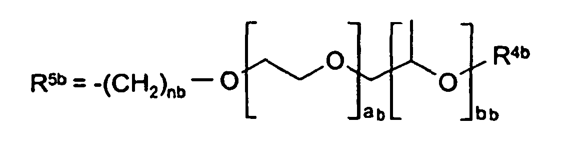 R^{4b} = -H; -COCH₃, C₁-C₄-Alkyl, -OH
nb = 1 bis 6, bevorzugt 2 bis 4, z. B. 3,
wobei yb so gewählt ist, dass mindestens 3 OH-Gruppen im Molekül vorhanden sind, das heißt für 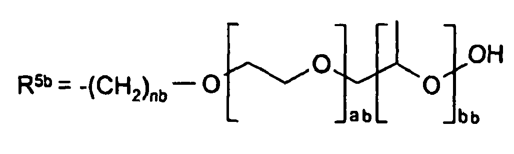 ist yb ≥ 3
xb und yb ganze Zahlen derart sind, dass das Molekulargewicht des Polysiloxan-Blocks zwischen 1000 bis 10000 liegt,
ab, bb ganze Zahlen zwischen 0 und 50 sein können mit der Maßgabe, dass die Summe aus ab und bb größer als 0 ist.

Als Komponente F) können weiterhin silikonhaltige Polyaminoverbindungen eingesetzt werden.

Bevorzugt handelt es sich dabei um ein Diaminopolyethersiloxan der Formel VI, das ausgewählt ist unter
- Polysiloxanen mit Wiederholungseinheiten der allgemeinen Formel VI.1 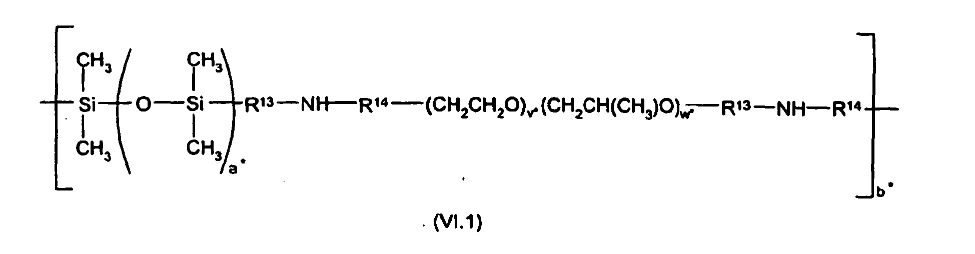 worin
   - a*: für eine ganze Zahl von 0 bis 100 steht,
   - b*: für eine ganze Zahl von 2 bis 8 steht,
   - R¹³ und R¹⁴: unabhängig voneinander für C₁-C₈-Alkylen stehen,
   die Reihenfolge der Alkylenoxideinheiten beliebig ist und v* und w* unabhängig voneinander für eine ganze Zahl von 0 bis 200 stehen, wobei die Summe aus v* und w* > 0 ist,
- Polysiloxanen der allgemeinen Formel VI.2 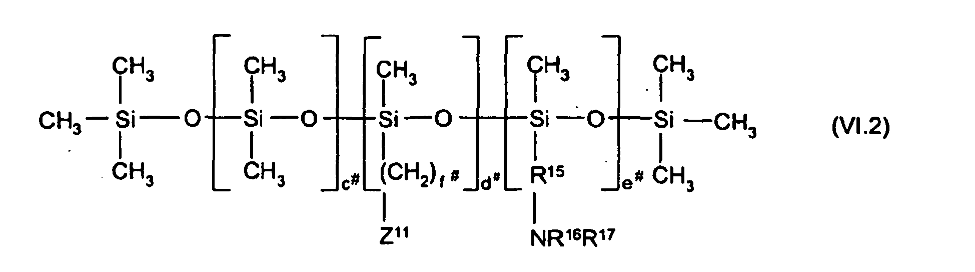 worin
   - R¹⁵: für einen C₁-C₈-Alkylenrest steht,
   - R¹⁶ und R¹⁷: unabhängig voneinander für Wasserstoff, C₁-C₈-Alkyl oder C₅-C₈-Cycloalkyl stehen, die Reihenfolge der Siloxaneinheiten beliebig ist,
   - c^{#}, d^{#} und e^{#}: unabhängig voneinander für 0 bis 100 stehen, wobei die Summe aus c^{#}, d^{#} und e^{#} mindestens 3 ist, und wobei d^{#} und e^{#} so gewählt sind, dass mindestens 3 aktive Wasserstoffatome im Molekül vorhanden sind,
   - f^{#}: für eine ganze Zahl von 2 bis 8 steht,
   - Z¹¹: für einen Rest der Formel VII

   -R¹⁸-(CH₂CH₂O)_{g#}(CH₂CH(CH₃)O)_{h#}-H (VII)

   steht, worin
   die Reihenfolge der Alkylenoxideinheiten beliebig ist und g^{#} und h^{#} unabhängig voneinander für eine ganze Zahl von 0 bis 200 stehen, wobei die Summe aus g" und h^{#} > 0 ist,
   R¹⁸ für einen C₁-C₈-Alkylenrest steht,
und Mischungen davon.

Polysiloxane der allgemeinen Formel VI.2 mit mehr als zwei aktiven Wasserstoffatomen sind solche, bei denen d^{#} ≥ 3 für den Fall, dass R¹⁶ und R¹⁷ ≠H; bzw. d^{#} + e^{#} ≥ 3 für den Fall, dass R¹⁶, R¹⁷ = H.

Bevorzugt stehen in der Formel VI.1 R¹³ und R¹⁴ unabhängig voneinander für einen C₂-C₄-Alkylenrest. Insbesondere stehen R¹³ und R¹⁴ unabhängig voneinander für einen C₂-C₃-Alkylenrest.

Vorzugsweise liegt das Molekulargewicht der Verbindung der Formel VI.1 in einem Bereich von etwa 300 bis 100000 liegt.

Vorzugsweise steht in der Formel VI.1 a* für eine ganze Zahl von 1 bis 20, wie z. B. 2 bis 10.

Bevorzugt liegt die Gesamtzahl der Alkylenoxideinheiten der Verbindung der Formel VI.1, d. h. die Summe aus v* und w*, in einem Bereich von etwa 3 bis 200, bevorzugt 5 bis 180.

Bevorzugt sind die Endgruppen der Polysiloxane mit Wiederholungseinheiten der allgemeinen Formel VI.1 ausgewählt unter (CH₃)₃SiO, H, C₁-C₈-AlKyl und Mischungen davon.

Geeignete alkoxylierte Siloxanamine der Formel VI.1 sind z. B. in der WO-A-97/32917 beschrieben, auf die hier in vollem Umfang Bezug genommen wird. Kommerziell erhältliche Verbindungen sind z. B. die Silsoft®-Marken der Fa. Witco, z. B. Silsoft® A-843.

Bevorzugt steht in der Formel VI.2 der Rest R¹⁵ für einen C₂-C₄-Alkylenrest.

Bevorzugt stehen in der Formel VI.2 R¹⁶ und R¹⁷ unabhängig voneinander für Wasserstoff oder C₁-C₄-Alkyl.

Vorzugsweise wird die Summe aus c^{#}, d^{#} und e^{#} so gewählt, dass das Molekulargewicht der Verbindung der Formel VI.2 in einem Bereich von etwa 300 bis 100000, bevorzugt 500 bis 50000, liegt.

Bevorzugt liegt die Gesamtzahl der Alkylenoxideinheiten des Restes der Formel VII, d. h. die Summe aus g^{#} und h^{#}, in einem Bereich von etwa 3 bis 200, bevorzugt 5 bis 80.

Bevorzugt steht in der Formel VII der Rest R¹⁸ für C₂-C₄-Alkylen.

Eine geeignete Verbindung der Formel VI.2 ist z. B. Silsoft® A-858 der Fa. Witco.

Bevorzugt sind vernetzte Polyurethane, wie zuvor beschrieben, die aufgebaut sind aus
- 12 bis 35 Gew.-%, bevorzugt 18 bis 30 Gew.%, wenigstens einer Verbindung der Komponente A),
- 0,1 bis 20 Gew.-%, bevorzugt 0,3 bis 15 Gew.%, wenigstens einer Verbindung der Komponente B),
- 5 bis 20 Gew.%, bevorzugt 7 bis 18 Gew.%, wenigstens einer Verbindung der Komponente C),
- 23 bis 60 Gew.%, bevorzugt 27 bis 55 Gew.%, wenigstens einer Verbindung der Komponente D),
- 2 bis 20 Gew.%, bevorzugt 3 bis 15 Gew.%, wenigstens einer Verbindung der Komponente E),
- 0 bis 20 Gew.-%, bevorzugt 0,1 bis 18 Gew.%, wenigstens einer Verbindung der Komponente F),
wobei sich die Komponenten zu 100 Gew.% addieren.

Besonders bevorzugt sind vernetzte Polyurethane, die aufgebaut sind aus
- 12 bis 35 Gew.-%, bevorzugt 15 bis 30 Gew.%, wenigstens einer Verbindung der Komponente A),
- 1 bis 20 Gew.%, bevorzugt 3 bis 15 Gew.-%, wenigstens einer Verbindung der Komponente B), die ausgewählt ist unter Amingruppen-haltigen Polymeren mit einer Aminzahl von mindestens 0,1 g KOH/g und einem zahlenmittleren Molekulargewicht von mindestens 1000,
- 5 bis 20 Gew.-%, bevorzugt 7 bis 18 Gew.%, insbesondere 10 bis 15 Gew.-% wenigstens einer Verbindung der Komponente C),
- 23 bis 60 Gew.%, bevorzugt 27 bis 55 Gew.-%, insbesondere 32 bis 50 Gew.-% wenigstens einer Verbindung der Komponente D),
- 2 bis 20 Gew.-%, bevorzugt 3 bis 15 Gew.-%, insbesondere 5 bis 12 Gew.-% wenigstens einer Verbindung der Komponente E),
- 0 bis 20 Gew.-%, bevorzugt 0 bis 10 Gew.-% (beispielsweise 0,1 bis 7 Gew.-%), wenigstens einer Verbindung der Komponente F),
wobei sich die Komponenten zu 100 Gew.-% addieren.

Weiterhin bevorzugt sind vernetzte Polyurethane, wie zuvor beschrieben, die aufgebaut sind aus
- 15 bis 35 Gew.-%, bevorzugt 18 bis 30 Gew.-%, wenigstens einer Verbindung der Komponente A),
- 0,1 bis 5 Gew.-%, bevorzugt 0,5 bis 3 Gew.-%, insbesondere 0,7 bis 2,5 Gew.-%, wenigstens einer Verbindung der Komponente B), die ausgewählt ist unter Verbindungen, die mehr als zwei gegenüber NCO-Gruppen reaktive Gruppen aufweisen, mit einem Molekulargewicht im Bereich von etwa 80 bis weniger als 1000 g/mol, insbesondere Trimethylolpropan,
- 8 bis 20 Gew.-%, bevorzugt 10 bis 18 Gew.-%, insbesondere 12 bis 15 Gew.-% wenigstens einer Verbindung der Komponente C),
- 25 bis 60 Gew.-%, bevorzugt 30 bis 55 Gew.-%, insbesondere 35 bis 50 Gew.-% wenigstens einer Verbindung der Komponente D),
- 3 bis 20 Gew.-%, bevorzugt 5 bis 15 Gew.-%, insbesondere 7 bis 12 Gew.-% wenigstens einer Verbindung der Komponente E),
- 0 bis 20 Gew.-%, bevorzugt 0 bis 18 Gew.-% (beispielsweise 0,1 bis 15 Gew.-%), wenigstens einer Verbindung der Komponente F),
wobei sich die Komponenten zu 100 Gew.-% addieren.

Besonders bevorzugt sind vernetzte Polyurethane, die aufgebaut sind aus
A) mindestens einem Polytetrahydrofuran mit zwei endständigen Hydroxylgruppen pro Molekül und einem zahlenmittleren Molekulargewicht im Bereich von 650 bis 2000,
B) Trimethylolpropan,
C) Dimethylolpropansäure,
D) einem Polyisocyanatgemisch, das Isophorondiisocyanat und Hexamethylendiisocyanat enthält,
   wobei das Gewichtsmengenverhältnis von Isophorondiisocyanat zu Hexamethylendiisocyanat in einem Bereich von 4:1 bis 15:1 liegt, und
E) Neopentylglykol.

Des Weiteren besonders bevorzugt sind vernetzte Polyurethane, die aufgebaut sind aus
A) mindestens einem Polytetrahydrofuran mit zwei endständlgen Hydroxylgruppen pro Molekül und einem zahlenmittleren Molekulargewicht im Bereich von 650 bis 2000.
B) wenigstens einem Amingruppen-haltigen Polymer, insbesondere einem Copolymer aus N-tert-Butylaminoethylmethacrylat, Vinylpyrrolidon, Vinylimidazol und Methacrylsäure,
C) Dimethylolpropansäure,
D) einem Polylsocyanatgernisch, das Isophorondiisocyanat und Hexamethylendiisocyanat enthält,
   wobei das Gewichtsmengenverhältnis von Isophorondiisocyanat zu Hexamethylendiisocyanat in einem Bereich von 4:1 bis 15:1 liegt, und
E) Neopentylglykol.

Die Herstellung der in den erfindungsgemäßen Mitteln eingesetzten Polyurethane erfolgt durch Umsetzung der Verbindungen der Komponenten A), B), C), D), E) und gegebenenfalls F). Die Temperatur liegt dabei in einem Bereich von etwa 30 bis 140 °C, bevorzugt etwa 40 bis 100 °C. Die Reaktion kann ohne Lösungsmittel oder in einem geeigneten inerten Lösungsmittel oder Lösungsmittelgemisch erfolgen. Geeignete Lösungsmittel sind aprotisch polare Lösungsmittel, z. B. Tetrahydrofuran, Essigsäureethylester, N-Methylpyrrolidon, Dimethylformamid und bevorzugt Ketone, wie Aceton und Methylethylketon. Vorzugsweise erfolgt die Reaktion unter einer Inertgasatmosphäre, wie z. B. unter Stickstoff. Die Komponenten werden in solchen Mengen eingesetzt, dass das Verhältnis von NCO-Äquivalent der Verbindungen der Komponente D) zu Äquivalent aktives Wasserstoffatom der Komponenten A), B), C), D), E) und F) in einem Bereich von etwa 0,8:1 bis 1,25:1, bevorzugt 0,85:1 bis 1,2:1, insbesondere 1,05:1 bis 1,15:1, liegt, Weisen die resultierenden Polyurethane noch freie Isocyanatgruppen auf, so werden diese abschließend durch Zusatz von Aminen, vorzugsweise Aminoalkoholen inaktiviert. Geeignete Aminoalkohole sind die zuvor als Komponente E) beschriebenen, bevorzugt 2-Amino-2-methyl-1-propanol, sowie wässrige NaOH oder wässrige KOH.

Wird zur Herstellung der erfindungsgemäßen vernetzten Polyurethane eine Komponente B) eingesetzt, die Amingruppen-haltige Polymere umfasst, so ist es zweckmäßig, zunächst aus diesen Polymeren der Komponente B) und zumindest einem Teil der Polyisocyanate D) ein Prepolymer herzustellen, das freie NCO-Gruppen aufweist und anschließend dieses Prepolymer, gegebenenfalls nach einer Isolierung und/oder Reinigung, mit den Komponenten A), gegebenenfalls niedermolekularen Verbindungen B), C), gegebenenfalls noch nicht eingesetzten Polyisocyanaten D) und E) zu den erfindungsgemäß vernetzen Polyurethanen umzusetzen. Zur Herstellung des Prepolymers liegt die Reaktionstemperatur in einem Bereich von etwa 30 bis 100 °C. Gewünschtenfalls kann zur Herstellung des Prepolymers auch ein Lösungsmittel oder Lösungsmittelgemisch eingesetzt werden, welches aktive Wasserstoffatome aufweist. Dann werden bevorzugt Alkohole, wie Methanol und Ethanol, und Gemische aus Alkoholen und Wasser eingesetzt. Vor der Umsetzung des Prepolymers mit den übrigen Komponenten wird das Lösungsmittel dann vorzugsweise gegen eines der zuvor genannten aprotisch polaren Lösungsmittel ausgetauscht.

Die Säuregruppen enthaltenden Polyurethane können durch teilweise oder vollständige Neutralisation mit einer Base in eine wasserlösliche bzw. wasserdispergierbare Form überführt werden.

In aller Regel weisen die erhaltenen Salze der Polyurethane eine bessere Wasserlöslichkeit oder Dispergierbarkeit in Wasser auf als die nicht neutralisierten Polyurethane. Als Base für die Neutralisation der Polyurethane können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxid, Calciumoxid, Magnesiumhydroxid oder Magnesiumcarbonat sowie Ammoniak und Amine verwendet werden. Geeignete Amine sind z. B. C₁-C₆-Alkylamine, bevorzugt n-Propylamin und n-Butylamin, Dialkylamine, bevorzugt Diethylpropylamin und Dipropylmethylamin, Trialkylamine, bevorzugt Triethylamin und Triisopropylamin, C₁-C₆-Alkyldiethyanolamine, bevorzugt Methyl- oder Ethyldiethanolamin und Di-C₁-C₆-Alkylethanolamine. Besonders für den Einsatz in Haarbehandlungsmitteln haben sich zur Neutralisation der Säuregruppen enthaltenden Polyurethane NaOH, KOH, 2-Amino-2-methyl-1-propanol, Diethylaminopropylamin und Triisopropanolamin bewährt. Die Neutralisation der Säuregruppen enthaltenden Polyurethane kann auch mit Hilfe von Mischungen mehrerer Basen vorgenommen werden, z. B. Mischungen aus Natronlauge und Triisopropanolamin. Die Neutralisation kann je nach Anwendungszweck partiell z. B. zu 20 bis 40 % oder vollständig, d. h. zu 100 % erfolgen.

Wird bei der Herstellung der Polyurethane ein wassermischbares organisches Lösungsmittel eingesetzt, so kann dieses im Anschluss durch übliche, dem Fachmann bekannte Verfahren, z. B. durch Destillation bei vermindertem Druck, entfernt werden. Vor dem Abtrennen des Lösungsmittels kann dem Polyurethan zusätzlich Wasser zugegeben werden. Nach Ersatz des Lösungsmittels durch Wasser erhält man eine Lösung oder Dispersion des Polymers, aus der, falls gewünscht, das Polymer In üblicher Weise gewonnen werden kann, z. B. durch Sprühtrocknung.

Die erfindungsgemäßen Polyurethane weisen K-Werte (gemessen nach E. Fikentscher, Cellulose-Chemie 13 (1932), S. 58-64, an einer 1 %igen Lösung in N-Methylpyrrolidon) in einem Bereich von 15 bis 90, bevorzugt 20 bis 60, auf. Ihre Glasübergangstemperatur beträgt Im Allgemeinen mindestens 0 °C, bevorzugt mindestens 20 °C, insbesondere bevorzugt mindestens 25 °C und speziell mindestens 30 °C.

Ein weiterer Gegenstand der Erfindung ist ein kosmetisches oder pharmazeutisches Mittel, enthaltend
α) wenigstens ein vernetztes Polyurethan, wie zuvor definiert
   wobei in Komponente D) das Gewichtsmengenverhältnis von Isophorondiisocyanat zu Hexamethylendiisocyanat in einem Bereich von 3:1 bis 15:1 liegt, und
β) wenigstens einen kosmetisch oder pharmazeutisch akzeptablen Träger,

Vorzugsweise ist die Komponente β) ausgewählt unter
i) Wasser,
ii) wassermischbaren organischen Lösungsmitteln, vorzugsweise C₂-C₄-Alkanolen, insbesondere Ethanol,
iii) Ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen,
viii) Treibgasen,
und Mischungen davon.

Die erfindungsgemäßen Mittel weisen z.B. eine Öl- bzw. Fettkomponente β) auf, die ausgewählt ist unter Kohlenwasserstoffen geringer Polarität, wie Mineralölen; linearen gesättigten Kohlenwasserstoffen, vorzugsweise mit mehr als 8 C-Atomen, wie Tetradecan, Hexadecan, Octadecan; cyclischen Kohlenwasserstoffen, wie Decahydronaphthalin; verzweigten Kohlenwasserstoffen; tierischen und pflanzlichen Ölen; Wachsen; Wachsestem; Vaselin; Estern, bevorzugt Estern von Fettsäuren, wie z. B. die Ester von C₁-C₂₄-Monoalkoholen mit C₁-C₂₂-Monocarbonsäuren, wie Isopropylisostearat, n-Propylmyristat, iso-Propylmyristat, n-Propylpalmitat, iso-Propylpalmitat, Hexacosanylpalmitat, Octacosanylpalmitat, Triacontanylpalmitat, Dotriacontanylpalmitat, Tetratriacontanylpalmitat, Hexacosanylstearat, Octacosanylstearat, Triacontanylstearat, Dotriacontanylstearat, Tetratriacontanylstearat; Salicylaten, wie C₁-C₁₀-Salicylaten, z. B. Octylsalicylat; Benzoatestern, wie C₁₀-C₁₅-Alkylbenzoaten, Benzylbenzoat; anderen kosmetischen Estern, wie Fettsäuretriglyceriden, Propylenglykolmonolaurat, Polyethylenglykolmonolaurat, C₁₀-C₁₅-Alkyllactaten, und Mischungen davon.

Geeignete Siliconöle β) sind z. B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z. B. unter der Bezeichnung Cyclomethicon kommerziell erhältlich.

Bevorzugte Öl- bzw. Fettkomponenten β) sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkernöl, Ricinusöl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candelillawachs, Walrat sowie Mischungen der zuvor genannten Öl-bzw. Fettkomponenten.

Geeignete kosmetisch und pharmazeutisch verträgliche Öl- bzw. Fettkomponenten β) sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird.

Geeignete hydrophile Träger β) sind ausgewählt unter Wasser, 1-, 2- oder mehrwertigen Alkoholen mit vorzugsweise 1 bis 8 Kohlenstoffatomen, wie Ethanol, n-Propanol, iso-Propanol, Propylenglykol, Glycerin, Sorbit.

Bei den erfindungsgemäßen kosmetischen Mitteln kann es sich um hautkosmetische, haarkosmetische, dermatologische, hygienische oder pharmazeutische Mittel handeln. Aufgrund ihrer filmbildenden und flexiblen Eigenschaften eignen sich die zuvor beschriebenen vernetzten Polyurethane insbesondere als Zusatzstoffe für Haar- und Hautkosmetika.

Vorzugsweise liegen die erfindungsgemäßen Mittel in Form eines Gels, Schaums, Sprays, einer Salbe, Creme, Emulsion, Suspension, Lotion, Milch oder Paste vor. Gewünschtenfalls können auch Liposomen oder Mikrosphären eingesetzt werden.

Die erfindungsgemäßen kosmetisch oder pharmazeutisch aktiven Mittel können zusätzlich kosmetisch und/oder dermatologisch aktive Wirkstoffe sowie Hilfsstoffe enthalten.

Vorzugsweise enthalten die erfindungsgemäßen kosmetischen Mittel wenigstens ein wie vorstehend definiertes vernetztes Polyurethan, wenigstens einen wie vorstehend definierten Träger β) und wenigstens einen davon verschiedenen Bestandteil, der vorzugsweise ausgewählt ist unter kosmetisch aktiven Wirkstoffen, Emulgatoren, Tensiden, Konservierungsmitteln, Parfümölen, Verdickern, Haarpolymeren, Haar- und Hautconditionem, Pfropfpolymeren, wasserlöslichen oder dispergierbaren silikonhaltigen Polymeren, Lichtschutzmitteln, Bleichmitteln, Gelbildnem, Pflegemitteln, Färbemitteln, Tönungsmitteln, Bräunungsmitteln, Farbstoffen. Pigmenten, Konsistenzgebern, Feuchthaltemitteln, Rückfettern, Collagen, Eiweisshydrolysaten, Lipiden, Antioxidantien, Entschäumern, Antistatika, Emollienzien und Weichmachern.

Übliche Verdickungsmittel in derartigen Formulierungen sind vernetzte Polyacrylsäuren und deren Derivate, Polysaccharide und deren Derivate, wie Xanthan-gum, Agar-Agar, Alginate oder Tylosen, Cellulosederivate, z. B. Carboxymethylcellulose oder Hydroxycarboxymethylcellulose, Fettalkohole, Monoglyceride und Fettsäuren, Polyvinylalkohol und Polyvinylpyrrolidon.

Geeignete kosmetisch und/oder dermatologisch aktive Wirkstoffe sind z. B. färbende Wirkstoffe, Haut- und Haarpigmentierungsmittel, Tönungsmittel, Bräunungsmittel, Bleichmittel, Keratin-härtende Stoffe, antimikrobielle Wirkstoffe, Lichlfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, desodorierende Wirkstoffe, sebostatische Wirkstoffe, Pflanzenextrakte, antierythimatös oder antiallergisch aktive Wirkstoffe und Mischungen davon.

Künstlich hautbräunende Wirkstoffe, die geeignet sind, die Haut ohne natürliche oder künstliche Bestrahlung mit UV-Strahlen zu bräunen, sind z. B. Dihydroxyaceton, Alloxan und Walnussschalenextrakt. Geeignete Keratin-härtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat. Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z. B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure. Derartige desodorierend wirkende Stoffe sind z. B. Zinkricinoleat, Triclosan, Undecylensäurealkylolamide, Citronensäuretriethylester, Chlorhexidin. Geeignete Lichtfilterwirkstoffe sind Stoffe, die UV-Strahlen im UV-Bund/oder UV-A-Bereich absorbieren. Geeignete UV-Filter sind z. B. 2,4,6-Triaryl-1,3,5-triazine, bei denen die Arylgruppen jeweils wenigstens einen Substituenten tragen können, der vorzugsweise ausgewählt ist unter Hydroxy, Alkoxy, speziell Methoxy, Alkoxycarbonyl, speziell Methoxycarbonyl und Ethoxycarbonyl und Mischungen davon. Geeignet sind weiterhin p-Aminobenzoesäureester, Zimtsäureester, Benzophenone, Campherderivate sowie UV-Strahlen abhaltende Pigmente, wie Titandioxid, Talkum und Zinkoxid. Geeignete Repellentwirkstoffe sind Verbindungen, die in der Lage sind, bestimmte Tiere, insbesondere Insekten, vom Menschen abzuhalten oder zu vertreiben. Dazu gehört z. B. 2-Ethyl-1,3-hexandiol, N,N-Diethyl-m-toluamid. Geeignete hyperemisierend wirkende Stoffe, welche die Durchblutung der Haut anregen, sind z. B. ätherische Öle, wie Latschenkiefer, Lavendel, Rosmarin, Wacholderbeer, Rosskastanienextrakt, Birkenblätterextrakt, Heublumenextrakt, Ethylacetat, Campher, Menthol, Pfefferminzöl, Rosmarinextrakt, Eukalyptusöl. Geeignete keratolytisch und keratoplastisch wirkende Stoffe sind z. B. Salicylsäure, Katziumthioglykolat, Thioglykolsäure und ihre Salze, Schwefel. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion. Geeignete Antiphlogistika, die Hautreizungen entgegenwirken, sind z. B. Allantoin, Bisabolol, Dragosantol, Kamillenextrakt, Panthenol.

Die erfindungsgemäßen kosmetischen Mittel können als kosmetischen und/oder pharmazeutischen Wirkstoff (wie auch gegebenenfalls als Hilfsstoff) wenigstens ein kosmetisch oder pharmazeutisch akzeptables Polymer enthalten, das sich von den erfindungsgemäßen vernetzten Polyurethanen unterscheidet. Dazu zählen ganz allgemein anionische, kationische, amphotere und neutrale Polymere.

Beispiele für anionische Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane, z. B. Luviset PUR® der Fa. BASF, und Polyharnstoffe. Besonders geeignete Polymere sind Copolymere aus t-Butylacrylat, Ethylacrylat, Methacrylsäure (z. B. Luvimer® 100P), Copolymere aus Ethylacrylat und Methacrylsäure (z. B. Luviumer® MAE), Copolymere aus N-tert.-Butylacrylamid, Ethylacrylat, Acrylsäure (Ultrahold® 8, strong), Copolymere aus Vinylacetat, Crotonsäure und gegebenenfalls weitere Vinylester (z. B. Luviset®-Marken), Maleinsäureanhydridcopolymere, gegebenenfalls mit Alkohol umgesetzt, anionische Polysiloxane, z. B. carboxyfunktionelle, t-Butylacrylat, Methacrylsäure (z. B. Luviskol® VBM), Copolymere von Acrylsäure und Methacrylsäure mit hydrophoben Monomeren, wie z. B. C₄-C₃₀-Alkylester der Meth(acrylsäure), C₄-C₃₀-Alkylvinylester, C₄-C₃₀-Alkylvinylether und Hyaluronsäure. Beispiele für anionische Polymere sind weiterhin Vinylacetat/Crotonsäure-Copolymere, wie sie beispielsweise unter den Bezeichnungen Resyn® (National Starch) und Gafset® (GAF) im Handel sind und Vinylpyrrolidon/Vinylacrylat-Copolymere, erhältlich beispielsweise unter dem Warenzeichen Luviflex® (BASF). Weitere geeignete Polymere sind das unter der Bezeichnung Luviflex® VBM-35 (BASF) erhältliche Vinylpyrrolidon/Acrylat-Terpolymer und Natriumsulfonat-haltige Polyamide oder Natriumsulfonat-haltige Polyester. Weiterhin geeignet sind Vinylpyrrolidon/Ethylmethacrylat/Methacrylsäure-Copolymere wie sie von der Fa. Stepan unter den Bezeichnungen Stepanhold-Extra und -R1 vertrieben werden und die Carboset®-Marken der Fa. BF Goodrich..

Geeignete kationische Polymere sind z. B. kationische Polymere mit der Bezeichnung Polyquaternium nach INCI, z. B. Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quatemisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquatemium-4 und -10), Acrylamidocopolymere (Polyquaternium-7) und Chitosan. Geeignete kationische (quäternisierte) Polymere sind auch Merquat® (Polymer auf Basis von Dimethyldiallylammoniumchlorid), Gafquat® (quatemäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z. B. Guarpolymere, wie die Jaguar®-Marken der Fa. Rhodia.

Weitere geeignete Polymere sind auch neutrale Polymere, wie Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und Derivate. Dazu zählt beispielsweise Luviflex® Swing (teilverseiftes Copolymerisat von Polyvinylacetat und Polyethylenglykol, Fa. BASF).

Geeignete Polymere sind auch nichtionische, wasserlösliche bzw. wasserdispergierbare Polymere oder Oligomere, wie Polyvinylcaprolactam, z. B. Luviskol® Plus (BASF), oder Polyvinylpyrrolidon und deren Copolymere, insbesondere mit Vinylestern, wie Vinylacetat, z. B. Luviskol® VA 37 (BASF); Polyamide, z. B. auf Basis von Itaconsäure und aliphatischen Diaminen, wie sie z. B. in der DE-A-43 33 238 beschrieben sind.

Geeignete Polymere sind auch amphotere oder zwitterionische Polymere, wie die unter den Bezeichnungen Amphomer® (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®).

Geeignete Polymere sind auch nichtionische, siloxanhaltige, wasserlösliche oder -dispergierbare Polymere, z. B. Polyethersiloxane, wie Tegopren® (Fa. Goldschmidt) oder Belsil® (Fa. Wacker).

Die Formulierungsgrundlage erfindungsgemäßer pharmazeutischer Mittel enthält bevorzugt pharmazeutisch akzeptable Hilfsstoffe. Pharmazeutisch akzeptabel sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. DAB Ph. Eur. BP NF) gelisteten sowie andere Hilfsstoffe, deren Eigenschaften einer physiologischen Anwendung nicht entgegenstehen.

Geeignete Hilfsstoffe können sein: Gleitmittel, Netzmittel, emulgierende und suspendierende Mittel, konservierende Mittel, Antioxidantien, Antireizstoffe, Chelatbildner, Emulsionsstabilisatoren, Filmbildner, Gelbildner, Geruchsmaskierungsmittel, Harze, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder Öl-Grundstoffe, Siliconderivate, Stabitisatoren, Sterilantien, Treibmittel, Trocknungsmittel, Trübungsmittel, Verdickungsmittel, Wachse, Weichmacher, Weißöle. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Zur Herstellung der erfindungsgemäßen dermatologischen Mittel können die Wirkstoffe mit einem geeigneten Hilfsstoff (Exzipient) vermischt oder verdünnt werden. Exzipienten können feste, halb feste oder flüssige Materialien sein, die als Vehikel, Träger oder Medium für den Wirkstoff dienen können. Die Zumischung weiterer Hilfsstoffe erfolgt gewünschtenfalls in der dem Fachmann bekannten Weise. Weiterhin sind die vernetzten Polyurethane geeignet als Hilfsmittel in der Pharmazie, bevorzugt als oder in Beschichtungsmittel(n) oder Bindemittel(n) für feste Arzneiformen. Sie können auch in Cremes und als Tablettenüberzugsmittel und Tablettenbindemittel verwendet werden.

Nach einer bevorzugten Ausführungsform handelt es sich bei den erfindungsgemäßen Mitteln um ein Hautreinigungsmittel.

Bevorzugte Hautreinigungsmittel sind Seifen von flüssiger bis gelförmiger Konsistenz, wie Transparentseifen, Luxusseifen, Deoseifen, Cremeseifen, Babyseifen, Hautschutzseifen, Abrasivseifen und Syndets, pasteuse Seifen, Schmierseifen und Waschpasten, flüssige Wasch-, Dusch- und Badepräparate, wie Waschlotionen, Duschbäder und - gele, Schaumbäder, Ölbäder und Scrub-Präparate, Rasierschäume, -lotionen und - cremes.

Nach einer weiteren bevorzugten Ausführungsform handelt es sich bei den erfindungsgemäßen Mitteln um kosmetische Mittel zur Pflege und zum Schutz der Haut, Nagelpflegemittel oder Zubereitungen für die dekorative Kosmetik.

Geeignete hautkosmetische Mittel sind z. B. Gesichtswässer, Gesichtsmasken, Deodorantien und andere kosmetische Lotionen. Mittel für die Verwendung in der dekorativen Kosmetik umfassen beispielsweise Abdeckstifte, Theaterfarben, Mascara und Lidschatten, Lippenstifte, Kajalstifte, Eyelinern, Rouges, Puder und Augenbrauenstifte.

Außerdem können die vernetzten Polyurethane verwendet werden in Nose-Strips zur Porenreinigung, in Antiaknemitteln, Repellents, Rasiermitteln, Haarentfernungsmitteln, Intimpflegemitteln, Fußpflegemitteln sowie in der Babypflege.

Bei den erfindungsgemäßen Hautpflegemitteln handelt es sich insbesondere um W/O-oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen.

Hautkosmetische und dermatologische Mittel auf Basis der zuvor beschriebenen vernetzten Polyurethane zeigen vorteilhafte Wirkungen. Die Polymere können unter anderem zur Feuchthaltung und Konditionierung der Haut und zur Verbesserung des Hautgefühls beitragen. Die Polymere können auch als Verdicker in den Formulierungen wirken. Durch Zusatz der erfindungsgemäßen Polymere kann in bestimmten Formulierungen eine erhebliche Verbesserung der Hautverträglichkeit erreicht werden.

Hautkosmetische und dermatologische Mittel enthalten vorzugsweise wenigstens ein vernetztes Polyurethan in einem Anteil von etwa 0,001 bis 30 Gew.-%, vorzugsweise 0,01 bis 20 Gew.-%, ganz besonders bevorzugt 0,1 bis 12 Gew.-%, bezogen auf das Gesamtgewicht des Mittels.

Besonders Lichtschutzmittel auf Basis der vernetzten Polyurethane besitzen die Eigenschaft, die Verweilzeit der UV-absorbierenden Inhaltsstoffe im Vergleich zu gängigen Hilfsmitteln wie Polyvinylpyrrolidon zu erhöhen.

Je nach Anwendungsgebiet können die erfindungsgemäßen Mittel in einer zur Hautpflege geeigneten Form, wie z. B. als Creme, Schaum, Gel, Stift, Mousse, Milch, Spray (Pumpspray oder treibmittelhaltiger Spray) oder Lotion appliziert werden.

Die hautkosmetischen Zubereitungen können neben den vernetzten Polyurethanen und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Färbemittel, Tönungsmittel, Bräunungsmittel, Collagen, Eiweißhydrolysate, Stabilisatoren, pH-Wert-Regulatoren, Farbstoffe, Salze, Verdicker, Gelbildner, Konsistenzgeber, Silicone, Feuchthaltemittel, Rückfetter und weitere übliche Additive.

Bevorzugte Öl- und Fettkomponenten der hautkosmetischen und dermatologischen Mittel sind die zuvor genannten mineralischen und synthetischen Öle, wie z. B. Paraffine, Siliconöle und aliphatische Kohlenwasserstoffe mit mehr als 8 Kohlenstoffatomen, tierische und pflanzliche Öle, wie z. B. Sonnenblumenöl, Kokosöl, Avocadoöl, Olivenöl, Lanolin, oder Wachse, Fettsäuren, Fettsäureester, wie z. B. Triglyceride von C₆-C₃₀-Fettsäuren, Wachsester, wie z. B. Jojobaöl, Fettalkohole, Vaseline, hydriertes Lanolin und acetyliertes Lanolin sowie Mischungen davon.

Man kann die erfindungsgemäßen Polymere auch mit herkömmlichen Polymeren abmischen, falls spezielle Eigenschaften eingestellt werden sollen.

Zur Einstellung bestimmter Eigenschaften wie z. B. Verbesserung des Anfassgefühls, des Spreitverhaltens, der Wasserresistenz und/oder der Bindung von Wirk- und Hilfsstoffen, wie Pigmenten, können die hautkosmetischen und dermatologischen Zubereitungen zusätzlich auch konditionierende Substanzen auf Basis von Siliconverbindungen enthalten. Geeignete Siliconverbindungen sind beispielsweise Polyalkylsiloxane, Polyarylsiloxane, Polyarylalkylsiloxane, Polyethersiloxane oder Siliconharze.

Die Herstellung der kosmetischen oder dermatologischen Zubereitungen erfolgt nach üblichen, dem Fachmann bekannten Verfahren.

Bevorzugt liegen die kosmetischen und dermatologischen Mittel in Form von Emulsionen insbesondere als Wasser-in-Öl-(W/O)- oder Öl-in-Wasser(O/W)-Emulsionen vor. Es ist aber auch möglich, andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen, usw.

Die Herstellung von Emulsionen erfolgt nach bekannten Methoden. Die Emulsionen enthalten neben wenigstens einem vernetzten Polyurethan in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der Emulsionstypspezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion, z. B. für eine Hautcreme, enthält im Allgemeinen eine wässrige Phase, die mittels eines geeigneten Emulgatorsystems in einer Öl- oder Fettphase emulgiert ist. Zur Bereitstellung der wässrigen Phase kann ein vernetztes Polyurethan eingesetzt werden.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karité-Öl, Hoplostethus-Öl; mineralische Öle, deren Destillationsbeginn unter Atmosphärendruck bei ca. 250 °C und deren Destillationsendpunkt bei 410 °C liegt, wie z. B. Vaselinöl; Ester gesättigter oder ungesättigter Fettsäuren, wie Alkylmyristate, z. B. i-Propyl-, Butyl- oder Cetylmyristat, Hexadecylstearat, Ethyl-oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Siliconglykol-Copolymer, Fettsäuren und Fettalkohole enthalten.

Neben den vernetzten Polyurethanen können auch Wachse verwendet werden, wie z. B. Carnaubawachs, Candelillawachs, Bienenwachs, mikrokristallines Wachs, Ozokeritwachs und Ca-, Mg- und Al-Oleate, -Myristate, -Linoleate und -Stearate.

Weiterhin kann eine erfindungsgemäße Emulsion als O/W-Emulsion vorliegen. Eine derartige Emulsion enthält üblicherweise eine Ölphase, Emulgatoren, die die Ölphase in der Wasserphase stabilisieren, und eine wässrige Phase, die üblicherweise verdickt vorliegt. Als Emulgatoren kommen vorzugsweise O/W-Emulgatoren, wie Polyglycerinester, Sorbitanester oder teilveresterte Glyceride, in Betracht.

Nach einer weiteren bevorzugten Ausführungsform handelt es sich bei den erfindungsgemäßen Mitteln um ein Duschgel, eine Shampoo-Formulierung oder ein Badepräparat.

Solche Formulierungen enthalten wenigstens ein vernetztes Polyurethan sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickern/Gelbildnem, Hautkonditioniermitteln und Feuchthaltemitteln.

Diese Formulierungen enthalten vorzugsweise 2 bis 50 Gew.-%, bevorzugt 5 bis 40 Gew.-%, besonders bevorzugt 8 bis 30 Gew.-% Tenside, bezogen auf das Gesamtgewicht der Formulierung.

In den Wasch-, Dusch- und Badepräparaten können alle in Körperreinigungsmitteln üblicherweise eingesetzten anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten im Molekül aufweisen.

Dazu zählen z. B. Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, ethoxylierte Fettsäureamide, Alkylpolyglycoside oder Sorbitanetherester geeignet.

Außerdem können die Wasch-, Dusch- und Badepräparate übliche kationische Tenside enthalten, wie z. B. quaternäre Ammoniumverbindungen, beispielsweise Cetyltrimethylammoniumchlorid.

Weiterhin können die Duschgel-/Shampoo-Formulierungen Verdicker, wie z. B. Kochsalz, PEG-55, Propylenglykol-Oleat, PEG-120-Methylglucosedioleat und andere, sowie Konservierungsmittel, weitere Wirk- und Hilfsstoffe und Wasser enthalten.

Nach einer weiteren bevorzugten Ausführungsform handelt es sich bei den erfindungsgemäßen Mitteln um ein Haarbehandlungsmittel.

Erfindungsgemäße Haarbehandlungsmittel enthalten vorzugsweise wenigstens ein vernetztes Polyurethan in einer Menge im Bereich von etwa 0,1 bis 30 Gew.-%, bevorzugt 0,5 bis 20 Gew.-%, bezogen auf das Gesamtgewicht des Mittels.

Vorzugsweise liegen die erfindungsgemäßen Haarbehandlungsmittel in Form eines Schaumfestigers, Haarmousses, Haargels, Shampoos, Haarsprays, Haarschaums, Spitzenfluids, Egalisierungsmittels für Dauerwellen, Haarfärbe- und -bleichmittels oder "Hot-Oil-Treatments" vor. Je nach Anwendungsgebiet können die haarkosmetischen Zubereitungen als (Aerosol-)Spray, (Aerosol-)Schaum, Gel, Gelspray, Creme, Lotion oder Wachs appliziert werden. Haarsprays umfassen dabei sowohl Aerosolsprays als auch Pumpsprays ohne Treibgas. Haarschäume umfassen sowohl Aerosolschäume wie auch Pumpschäume ohne Treibgas. Haarsprays und Haarschäume umfassen vorzugsweise überwiegend oder ausschließlich wasserlösliche oder wasserdispergierbare Komponenten. Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.-%. Diese Mikrodispersionen benötigen in der Regel keine Emulgatoren oder Tenside zu ihrer Stabilisierung.

Die erfindungsgemäßen haarkosmetischen Formulierungen enthalten in einer bevorzugten Ausführungsform
a) 0,05 bis 20 Gew.-% wenigstens eines vernetzten Polyurethans,
b) 20 bis 99,95 Gew.-% Wasser und/oder Alkohol,
c) 0 bis 50 Gew.-% wenigstens eines Treibgases,
d) 0 bis 5 Gew.-% wenigstens eines Emulgators,
e) 0 bis 3 Gew.-% wenigstens eines Verdickers, sowie
f) bis zu 25 Gew.-% weitere Bestandteile.

Unter Alkohol sind alle in der Kosmetik üblichen Alkohole zu verstehen, z. B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d. h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z. B. Konservierungsmittel, Parfümöle, Trübungsmittel, Wirkstoffe, UV-Filter, Pflegestoffe wie Panthenol, Collagen, Vitamine, Eiweißhydrolysate, Alpha- und Beta-Hydroxycarbonsäuren, Stabilisatoren, pH-Wert-Regulatoren, Farbstoffe, Viskositätsregulierer, Gelbildner, Salze, Feuchthaltemittel, Rückfetter, Komplexbildner und weitere übliche Additive.

Weiterhin zählen hierzu alle in der Kosmetik bekannten Styling- und Conditionerpolymere, die in Kombination mit den erfindungsgemäßen Polymerisaten eingesetzt werden können, falls ganz spezielle Eigenschaften eingestellt werden sollen.

Als herkömmliche Haarkosmetik-Polymere eignen sich beispielsweise die zuvor genannten kationischen, anionischen, neutralen, nichtionischen und amphoteren Polymere, auf die hier Bezug genommen wird.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die erfindungsgemäßen Polymerisate eignen sich insbesondere als Festigungsmittel in Haarstyling-Zubereitungen, insbesondere Haarsprays (Aerosolsprays und Pumpsprays ohne Treibgas) und Haarschäume (Aerosolschäume und Pumpschäume ohne Treibgas).

In einer bevorzugten Ausführungsform enthalten Spray-Zubereitungen
a) 0,1 bis 10 Gew.-% wenigstens eines vernetzten Polyurethans,
b) 20 bis 99,9 Gew.-% Wasser und/oder Alkohol,
c) 0 bis 70 Gew.-% wenigstens eines Treibmittel,
d) 0 bis 20 Gew.-% weitere Bestandteile.

Treibmittel sind die für Haarsprays oder Aerosolschäume üblich verwendeten Treibmittel. Bevorzugt sind Gemische aus Propan/Butan, Pentan, Dimethylether, 1,1-Difluorethan (HFC-152 a), Kohlendioxid, Stickstoff oder Druckluft.

Eine erfindungsgemäß bevorzugte Formulierung für Aerosolhaarschäume enthält
a) 0,1 bis 10 Gew.-% wenigstens eines vernetzten Polyurethans,
b) 55 bis 99,8 Gew.-% Wasser und/oder Alkohol,
c) 5 bis 20 Gew.-% eines Treibmittel,
d) 0,1 bis 5 Gew.-% eines Emulgators,
e) 0 bis 10 Gew.-% weitere Bestandteile.

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Beispiele für nichtionische Emulgatoren (INCI-Nomenklatur) sind Laurethe, z. B. Laureth-4; Cetethe, z. B. Cetheth-1, Polyethylenglykolcetylether; Cetearethe, z. B. Cetheareth-25, Polyglykolfettsäureglyceride, hydroxyliertes Lecithin, Lactylester von Fettsäuren, Alkylpolyglycoside.

Beispiele für kationische Emulgatoren sind Cetyldimethyl-2-hydroxyethylammoniumdihydrogenphosphat, Cetyltrimoniumchlorid, Cetyltrimmoniumbromid, Cocotrimoniummethylsulfat, Quatemium-1 bis x (INCI).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Eine erfindungsgemäß für Styling-Gele geeignete Zubereitung kann beispielsweise wie folgt zusammengesetzt sein:
a) 0,1 bis 10 Gew.-% wenigstens eines vernetzten Polyurethans,
b) 80 bis 99,9 Gew.-%, vorzugsweise 80 bis 99,85 Gew.-% Wasser und/oder Alkohol,
c) 0 bis 3 Gew.-%, bevorzugt 0,05 bis 2 Gew.-%, eines Gelbildners,
d) 0 bis 20 Gew.-% weitere Bestandteile.

Bei der Herstellung von Gelen auf Basis der erfindungsgemäßen vernetzten Polyurethane können übliche Gelbildner eingesetzt werden, beispielsweise, um spezielle rheologische oder andere anwendungstechnische Eigenschaften der Gele einzustellen. Als Gelbildner können alle in der Kosmetik üblichen Gelbildner eingesetzt werden. Hierzu zählen leicht vernetzte Polyacrylsäure, beispielsweise Carbomer (INCI), Cellulosederivate, z. B. Hydroxypropylcellulose, Hydroxyethylcellulose, kationisch modifizierte Cellulosen, Polysaccharide, z. B. Xanthangummi, Capryl/Caprin-Triglycerid, Natriumacrylat-Copolymere, Polyquatemium-32 (und) Paraffinum Liquidum (INCI), Natriumacrylat-Copolymere (und) Paraffinum Liquidum (und) PPG-1 Trideceth-6, Acrylamidopropyltrimoniumchlorid/Acrylamid-Copolymere, Steareth-10 Allylether AcrylatCopolymere, Polyquatemium-37 (und) Paraffinum Liquidum (und) PPG-1 Trideceth-6, Polyquaternium 37 (und) Propylenglycoldicapratdicaprylat (und) PPG-1 Trideceth-6, Polyquaternium-7, Polyquaternium-44. Als Gelbildner geeignete vernetzte Homopolymere der Acrylsäure sind beispielsweise kommerziell unter dem Namen Carbopol® von der Firma BF GOODRICH erhältlich. Bevorzugt sind auch hydrophob modifizierte vernetzte Polyacrylat Polymere, wie Carbopol® Ultrez 21 von der Firma Noveon. Weitere Beispiele für als Gelbildner geeignete anionische Polymere sind Copolymere von Acrylsäure und Acrylamid und deren Salze; Natriumsalze von Polyhydroxycarbonsäuren, wasserlösliche oder wasserdispergierbare Polyester, Polyurethane und Polyhamstoffe. Besonders geeignete Polymere sind Copolymere aus (Meth)acrylsäure und Polyetheracrylaten, wobei die Polyetherkette mit einem C₈-C₃₀-Alkylrest terminiert ist. Dazu zählen z. B. Acrylat/Beheneth-25-methacrylat-Copolymere, die unter der Bezeichnung Aculyn® von der Firma Rohm und Haas erhältlich sind.

Die erfindungsgemäßen vernetzten Polyurethane können in kosmetischen Zubereitungen als Konditioniermittel eingesetzt werden.

Die erfindungsgemäßen vernetzten Polyurethane können bevorzugt in Shampooformulierungen als Festigungs- und/oder Konditioniermittel eingesetzt werden. Bevorzugte Shampoofomnulierungen enthalten
a) 0,05 bis 10 Gew.-% wenigstens eines vernetzten Polyurethans,
b) 25 bis 94,95 Gew.-% Wasser,
c) 5 bis 50 Gew.-% Tenside,
d) 0 bis 5 Gew.-% eines weiteren Konditioniermittels,
e) 0 bis 10 Gew.-% weitere kosmetische Bestandteile.

In den Shampooformulierungen können alle in Shampoos üblicherweise eingesetzte anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die Shampooformulierungen übliche kationische Tenside enthalten, wie z. B. quaternäre Ammoniumverbindungen, beispielsweise Cetyltrimethylammoniumchlorid.

In den Shampooformulierungen können zur Erzielung bestimmter Effekte übliche Konditioniermittel in Kombination mit den vernetzten Polyurethanen eingesetzt werden. Hierzu zählen beispielsweise die zuvor genannten kationischen Polymere mit der Bezeichnung Polyquaternium nach INCI, insbesondere Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidcopolymere (Polyquaternium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

Ein weiterer Gegenstand der Erfindung ist die Verwendung eines vernetzten Polyurethans, wie zuvor definiert, als Hilfsmittel in der Pharmazie, bevorzugt als oder in Beschichtungsmittel(n) für feste Arzneiformen, zur Modifizierung rheologischer Eigenschaften, als oberflächenaktive Verbindung, als oder in Klebemittel(n) sowie als oder in Beschichtungsmittel(n) für die Textil-, Papier-, Druck- und Lederindustrie.

Die Erfindung wird anhand der folgenden nicht einschränkenden Beispiele näher erläutert.

### Beispiele

### I. Herstellung von vernetzten Polyurethanen

### Beispiel 1:

In einem 4-Hals-Kolben, der mit Rührer, Tropftrichter, Thermometer, Rückflusskühler und einer Vorrichtung für das Arbeiten unter Stickstoff ausgestattet war, wurden 500 g (0,5 Mol) Polytetrahydrofuran (Mₙ = 1000 g/mol), 33,5 g (0,25 Mol) Trimethylolpropan, 335 g (2,5 Mol) Dimethylolpropansäure, 176,8 g (1,7 Mol) Neopentylglykol und 0,85 g 1,4-Diazabicyclo[2.2.2]octan (DABCO) in 690 g Methylethylketon unter Erhitzen auf eine Temperatur von 80 °C und unter Rühren gelöst. Nachdem sich alle Komponenten gelöst hatten, ließ man das Reaktionsgemisch auf ca. 60 °C abkühlen. Anschließend wurde bei dieser Temperatur unter Rühren ein Gemisch aus 134,4 g (0,8 Mol) Hexamethylendiisocyanat und 932,4 g (4,2 Mol) Isophorondiisocyanat so zudosiert, dass die Reaktionstemperatur unterhalb von 90 °C blieb. Danach wurde das Reaktionsgemisch noch drei bis vier Stunden bei 80 °C nachgerührt, bis der NCO-Gehalt des Gemischs praktisch konstant blieb. Nach dem Abkühlen auf 40 °C gab man 405 g (2,375 Mol) einer 50 %igen 2-Amino-2-methyl-1-propanol-Lösung zu dem Reaktionsgemisch und entfernte anschließend das Lösungsmittel unter Vakuum bei 40 °C. Nach dem Behandeln mit Wasserdampf wurde das erhaltene Produkt mit Wasser versetzt, wobei eine 25 gew.-%ige im Wesentlichen klare Mikrodispersion erhalten wurde. Pulverförmige vernetzte Polyurethane lassen sich durch Sprühtrocknen isolieren.

Analog wurden die vernetzten Polyurethane 2 bis 5 hergestellt.

### Beispiel 8:

In einem 4-Hals-Kolben, der mit Rührer, Tropftrichter, Thermometer, Rückflusskühler und einer Vorrichtung für das Arbeiten unter Stickstoff ausgestattet war, wurden 500 g (0,5 Mol) Polytetrahydrofuran (Mₙ = 1000 g/mol), 33,5 g (0,25 Mol) Trimethylolpropan, 335 g (2,5 Mol) Dimethylolpropansäure, 176,8 g (1,7 Mol) Neopentylglykol und 0,85 g DABCO in 690 g Methylethylketon unter Erwärmen auf eine Temperatur von 80 °C und unter Rühren gelöst. Sobald alle Komponenten in Lösung gegangen waren, ließ man das Reaktionsgemisch auf 60 °C abkühlen. Anschließend wurde bei dieser Temperatur unter Rühren ein Gemisch aus 134,4 g (0,8 Mol) Hexamethylendiisocyanat und 932,4 g (4,2 Mol) Isophorondiisocyanat so zudosiert, dass die Reaktionstemperatur unterhalb von 90 °C blieb. Danach ließ man das Reaktionsgemisch bei etwa 80 °C noch ca. drei Stunden nachrühren, bis der NCO-Gehalt praktisch konstant blieb und ließ das Gemisch anschließend auf einer Temperatur von 40 °C abkühlen. Das Reaktionsprodukt wurde mit 467 g (2,5 Mol) 30 %iger KOH-Lösung versetzt und anschließend das Lösungsmittel unter Vakuum bei 40 °C abdestilliert. Nach Behandeln mit Wasserdampf wurde mit Wasser verdünnt, wobei eine 25 gew.-%ige im Wesentlichen klare Mikrodispersion erhalten wurde. Pulverförmige Produkte können durch Sprühtrocknen erhalten werden.

Analog wurden die vernetzten Polyurethane 9 bis 12 hergestellt.

### Beispiel 14:

In einem 4-Hals-Kolben, der mit Rührer, Tropftrichter, Thermometer, Rückflusskühler und einer Vorrichtung für das Arbeiten unter Stickstoff ausgestattet war, wurden 500 g (0,5 Mol) Polytetrahydrofuran (Mₙ = 1000 g/mol), 335 g (2,5 Mol) Dimethylolpropansäure, 176,8 g (1,7 Mol) Neopentylglykol und 0,85 g DABCO in 690 g Methylethylketon unter Erhitzen auf eine Temperatur von 80 °C und unter Rühren gelöst. Sobald alle Komponenten in Lösung gegangen waren, ließ man das Reaktionsgemisch auf 60 °C abkühlen und dosierte bei dieser Temperatur unter Rühren ein Gemisch aus 134,4 g (0,8 Mol) Hexamethylendiisocyanat und 932,4 g (4,2 Mol) Isophorondiisocyanat so langsam zu, dass die Reaktionstemperatur unterhalb von 90 °C blieb. Anschließend ließ man bei etwa 80 °C das Reaktionsgemisch ca. drei Stunden nachrühren, bis der NCO-Gehalt des Gemischs praktisch konstant blieb. Danach ließ man das Gemisch auf eine Temperatur von 40 °C abkühlen und gab 346,5 g einer wässrigen 30 %igen Aminogruppen-haltigen Copolymerlösung (Vinylpyrrolidon/Vinylimidazol/Methacrylsäure/N-tert.-Butylaminoethylmethacrylat-Copolymer) zu und ließ das Gemisch weitere 30 Minuten bei 40 °C rühren. Danach wurde das Reaktionsprodukt mit 467 g (2,5 Mol) 30 %iger wässriger KOH-Lösung neutralisiert und das Lösungsmittel unter Vakuum bei 40 °C entfernt. Nach dem Behandeln mit Wasserdampf verdünnte man das Reaktions-produkt, wobei eine 25 gew.-%ige Dispersion erhalten wurde. Pulverförmige Produkte lassen sich durch Sprühtrocknen erhalten.

Analog wurden die vernetzten Polyurethane 6, 7 und 13 hergestellt.

| | P(THF 1000) | TMP | Pol.-Amin | DMPA | NPG | IPDI | HDI | KOH | AMP | K-Wert |
|---|---|---|---|---|---|---|---|---|---|---|
| | [Mol] | [Mol] | [Gew.-%*)] | [Mol[ | [Mol] | [Mol] | [Mol] | [N.G.%] | [N.G.%] | (1% NMP) |
| 1 | 0,5 | 0,25 | -- | 2,5 | 1,7 | 4,2 | 0,8 | -- | 95 | 35,6 |
| 2 | 0,5 | 0,35 | -- | 2,5 | 1,7 | 4,1 | 0,8 | -- | 95 | 37,9 |
| 3 | 0,5 | 0,35 | -- | 2,5 | 1,7 | 4,0 | 1,0 | -- | 95 | 38,1 |
| 4 | 0,5 | 0,40 | -- | 2,5 | 1,7 | 4,0 | 1,0 | -- | 95 | 39,8 |
| 5 | 0,5 | 0,45 | -- | 2,5 | 1,6 | 4,0 | 1,0 | -- | 95 | 38,5 |
| 6 | 0,5 | -- | 5 | 2,5 | 1,7 | 4,0 | 1,0 | -- | 95 | 37,5 |
| 7 | 0,5 | -- | 5 | 2,5 | 1,7 | 4,2 | 0,8 | -- | 95 | 36,8 |
| 8 | 0,5 | 0,25 | -- | 2,5 | 1,7 | 4,2 | 0,8 | 100 | -- | 39,1 |
| 9 | 0,5 | 0,35 | -- | 2,5 | 1,7 | 4,1 | 0,9 | 100 | -- | 41,3 |
| 10 | 0,5 | 0,40 | - | 2,5 | 1,7 | 4,0 | 1,0 | 100 | - | 43,9 |
| 11 | 0,5 | 0,40 | -- | 2,5 | 1,7 | 4,0 | 1,0 | 100 | -- | 44,6 |
| 12 | 0,5 | 0,45 | -- | 2,5 | 1,6 | 4,0 | 1,0 | 100 | -- | 46,8 |
| 13 | 0,5 | -- | 5 | 2,5 | 1,7 | 4,0 | 1,0 | 100 | -- | 40,6 |
| 14 | 0,5 | -- | 5 | 2,5 | 1,7 | 4,2 | 0,8 | 100 | -- | 38,7 |

| | |
|---|---|
| P(THF)1000 | = Polytetrahydrofuran, MG = 1000 g/mol |
| TMP | = Trimethylolpropan |
| Pol.-Amin | = P(VP/VI/MAS/NtBAEMA) |
| *) | = Pol.-Amin wird in Gewicht, bezogen auf die Gesamtfeststoffe, eingesetzt |
| NPG | = Neopentylglykol |
| DMPA | = Dimethylolpropansäure |
| IPDI | = Isophorondiisocyanat |
| HDI | = Hexamethylendiisocyanat |
| AMP | = 2-Amino-2-methyl-1-propanol |
| KOH | = Kaliumhydroxid |
| N.G. % | = Neutralisationsgrad |
| NMP | = N-Methylpyrrolidon |

### II. Anwendungstechnische Beispiele

### Beispiel 1:

| VOC 80-Aerosol-Haarspray | [%] |
|---|---|
| Polymer aus Beispiel Nr. 1 (25 %ige Lösung) | 12,00 |
| Wasser | 8,00 |
| Dimethylether | 40,00 |
| Ethanol | 40,00 |
| weiterer Zusatz: Silikon, Parfüm, Entschäumer ... | |

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein VOC 80-Aerosol-Haarspray mit guten Eigenschaften erhalten.

### Beispiel 2:

| VOC 55-Aerosol-Haarspray | [%] |
|---|---|
| Polymer aus Beispiel Nr. 1 (25 %ige Lösung) | 12,00 |
| Wasser | 33,00 |
| Dimethylether | 40,00 |
| Ethanol | 15,00 |
| weiterer Zusatz: Silikon, Parfüm, Entschäumer ... | |

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein VOC 55-Aerosol-Haarspray mit guten Eigenschaften erhalten.

### Beispiel 3:

| VOC 55-Aerosol-Haarspray | [%] |
|---|---|
| Polymer aus Beispiel Nr. 1 (25 %ige Lösung) | 10,00 |
| Ultrahold Strong (Fa. BASF) | 1,00 |
| Wasser | 34,00 |
| Dimethylether | 40,00 |
| Ethanol | 15,00 |
| + AMP | auf pH 8,3 |
| weiterer Zusatz: Silikon, Parfüm, Entschäumer ... | |

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein VOC 55-Aerosol-Haarspray mit guten Eigenschaften erhalten.

### Beispiel 4:

| VOC 55-Aerosol-Haarspray | [%] |
|---|---|
| Polymer aus Beispiel Nr. 1 (25 %ige Lösung) | 8,00 |
| Stepanhold R-1^{*)} (Fa. Stepan Chemical Co.) | 1,00 |
| Wasser | 36,00 |
| Dimethylether | 40,00 |
| Ethanol | 15,00 |
| + AMP | auf pH 8,3 |
| weiterer Zusatz: Silikon, Parfüm, Entschäumer ... | |

| | |
|---|---|
| ^{*)} Stepanhold R-1 = Poly(Vinylpyrrolidon/Ethylmethacrylat/Methacrylsäure) | |

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein VOC 55-Aerosol-Haarspray mit guten Eigenschaften erhalten.

### Beispiel 5:

| VOC 55-Handpumpenspray | [%] |
|---|---|
| Polymer aus Beispiel Nr. 1 (25 %ige Lösung) | 12,00 |
| Wasser | 33,00 |
| Ethanol | 55,00 |
| weiterer Zusatz: Silikon, Parfüm, Entschäumer ... | |

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein VOC 55-Handpumpenspray mit guten Eigenschaften erhalten.

### Beispiel 6:

| Wässriges Handpumpenspray | [%] |
|---|---|
| Polymer aus Beispiel Nr. 1 (25 %ige Lösung) | 10,00 |
| Luviset Clear ^{*)} (20 %ige Lösung) | 5,00 |
| Wasser | 85,00 |
| weiterer Zusatz: wasserlösliches Silikon, Parfüm, Entschäumer ... | |

| | |
|---|---|
| ^{*)} Luviset Clear: Poly(Vinylpyrrolidon/Methacrylsäureamid/Vinylimidazol), Fa. BASF | |

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein wässriges Handpumpenspray mit guten Eigenschaften erhalten.

### Beispiel 7:

| Schaumconditioner | [%] |
|---|---|
| Polymere aus Beispiel Nr. 1 (25 %ige wässrige Lösung) | 20,00 |
| Cremophor A 25 (Ceteareth 25/Fa. BASF) | 0,2 |
| Comperlan KD (Coamide DEA/Fa. Henkel) | 0,1 |
| Wasser | 69,7 |
| Propan/Butan | 10,0 |
| weiterer Zusatz: Parfüm, Konservierungsmittel .... | |

### Herstellung: Einwiegen und unter Rühren lösen. Abfüllen und Treibgas zusetzen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Schaumconditioner mit guten Eigenschaften erhalten.

### Beispiel 8:

| Haargel mit Aculyn 28: | [%] |
|---|---|
| Phase 1: | |
| Polymer aus Beispiel Nr. 1 (25 %ige wässrige Lösung) | 12,00 |
| Wasser, dest. | 37,00 |
| Aminomethylpropanol (38 %ige Lösung) | 1,0 |
| weiterer Zusatz: Konservierungsmittel, lösliches ethoxiliertes Silikon, Parfüm ... | |
| | |

| Phase 2: | |
|---|---|
| Aculyn 28 (1 %ige wässrige Suspension) | 50,00 |

### Herstellung:

Die Phasen 1 und 2 werden getrennt eingewogen und homogenisiert. Dann wird Phase 2 langsam in Phase 1 eingerührt. Es bildet sich ein im Wesentlichen klares, stabiles Gel.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Haargel mit Aculyn 28 mit guten Eigenschaften erhalten.

### Beispiel 9:

| Haargel mit Hydroxyethylcellulose: | [%] |
|---|---|
| Phase 1: | |
| Polymer aus Beispiel-Nr. 1 (25 %ige Lösung) | 12,00 |
| Wasser, dest. | 30,00 |
| weiterer Zusatz: Konservierungsmittel, lösliches ethoxiliertes Silikon, Parfüm ... | |

| | |
|---|---|
| Phase 2: | |
| | |
| Natrosol HR 250 (5 %ige Lösung) | 50,00 |
| Hydroxyethylcellulose (Fa. Hercules) | |

### Herstellung:

Die Phasen 1 und 2 werden getrennt eingewogen und homogenisiert. Dann wird Phase 2 langsam in Phase 1 eingerührt. Es bildet sich ein im Wesentlichen klares, stabiles Gel.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Haargel mit Hydroxyethylcellulose mit guten Eigenschaften erhalten.

### Beispiel 10:

| Conditionershampoo: | [%] |
|---|---|
| A) Texapon NSO 28 %ig (Natriumlaurethsulfat/Fa. Henkel) | 50,00 |
| Comperlan KS (Coamid DEA/Fa. Henkel) | 1,00 |
| Polymer aus Beispiel Nr. 1 (25 %ige wässrige Lösung) | 20,00 |
| Parfümöl | q. s. |
| | |
| B) Wasser | 27,5 |
| Natriumchlorid | 1,5 |
| Konservierungsmittel ... | q. s. |

### Herstellung:

Die Phasen 1 und 2 werden getrennt eingewogen und homogenisiert. Dann wird Phase 2 langsam in Phase 1 eingerührt. Es bildet sich ein im Wesentlichen klares, stabiles Gel.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Conditionershampoo mit guten Eigenschaften erhalten.

### Beispiel 11:

| Standard O/W-Creme: | | |
|---|---|---|
| Ölphase: | [%] | CTFA-Name |
| Cremophor A63,5 | Ceteareth-6 (and) Stearyl Alcohol | |
| Cremophor A25 | 3,5 | Ceteareth-25 |
| Glycerinmonostearat s.e. | 2,5 | Glycerylstearat |
| Paraffinöl | 7,5 | Paraffin Oil |
| Cetylalkohol | 2,5 | Cetyl Alcohol |
| Luvitol EHO | 3,2 | Cetearyl Octanoate |
| Vitamin-E-Acetat | 1,0 | Tocopheryl Acetate |
| Nip-Nip | 0,1 | Methyl- and Propyl-4-hydroxybenzoate (7:3) |

### Wasserphase:

| Polymer aus Beispiel Nr. 1 | | |
|---|---|---|
| (25 %ige Lösung) | 3,0 | |
| Wasser | 74,6 | |
| 1,2-Propylenglykol | 1,5 | Propylenglykol |
| Germall II | 0,1 | Imidazolidinyl-Harnstoff |

### Herstellung:

Die Öl- und Wasserphasen werden getrennt eingewogen und bei einer Temperatur von ca. 80 °C homogenisiert. Dann wird die Wasserphase langsam in die Ölphase eingerührt und unter Rühren langsam auf Raumtemperatur abgekühlt.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall eine Standard O/W-Creme mit guten Eigenschaften erhalten.

### Beispiel 12: Flüssiges Makeup

| A | |
|---|---|
| 1,70 | Glycerylstearat |
| 1,70 | Cetylalkohol |
| 1,70 | Ceteareth-6 |
| 1,70 | Ceteareth-25 |
| 5,20 | Capryl/Caprin-Triglycerid |
| 5,20 | Mineralöl |
| | |

| B | |
|---|---|
| q.s. | Konservierungsmittel |
| 4,30 | Propylenglykol |
| 12,50 | Polymer 1 (20 %ige wässrige Lösung) |
| 49,50 | dest. Wasser |
| | |

| C | |
|---|---|
| q.s. | Parfumöl |
| | |

| D | |
|---|---|
| 2,00 | Eisenoxid |
| 12,00 | Titandioxid |

### Herstellung:

Phase A und Phase B getrennt voneinander auf 80 °C erwärmen. Dann Phase B in Phase A mit einem Rührer mischen. Alles auf 40 °C abkühlen lassen und Phase C und Phase D zugeben. Wiederholt homogenisieren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein flüssiges Makeup mit guten Eigenschaften erhalten.

### Beispiel 13: Ölfreies Makeup

| A | |
|---|---|
| 0,35 | Veegum |
| 5,00 | Butylenglykol |
| 0,15 | Xanthangummi |
| | |

| B | |
|---|---|
| 34,0 | dest. Wasser |
| q.s. | Konservierungsmittel |
| 0,2 | Polysorbate-20 |
| 1,6 | Tetrahydroxypropylethylenediamin |
| | |

| C | |
|---|---|
| 1,0 | Siliciumdioxid |
| 2,0 | Nylon-12 |
| 4,15 | Glimmer (mica) |
| 6,0 | Titandioxid |
| 1,85 | Eisenoxid |
| | |

| D | |
|---|---|
| 4,0 | Stearinsäure |
| 1,5 | Glycerylstearat |
| 7,0 | Benzyllaurat |
| 5,0 | Isoeicosan |
| q.s. | Konservierungsmittel |
| | |

| E | |
|---|---|
| 0,5 | Panthenol |
| 0,1 | Imidazolidinyl-Hamstoff |
| 25,0 | Polymer 1 (20 %ige wässrige Lösung) |

### Herstellung:

Phase A mit Butylenglykol benetzen, in Phase B hineingeben und gut mischen. Phase AB auf 75 °C erwärmen. Phase C Einsatzstoffe pulverisieren, in Phase AB hineingeben und gut homogenisieren. Einsatzstoffe von Phase D mischen, auf 80 °C erwärmen und zu Phase ABC geben. Einige Zeit mischen, bis alles homogen ist. Alles in ein Gefäß mit Propellermischer übertragen. Einsatzstoffe von Phase E mischen, in Phase ABCD hineingeben und gut vermischen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein ölfreies Makeup mit guten Eigenschaften erhalten.

### Beispiel 14: Schimmerndes Gel

| A | |
|---|---|
| 32,6 | dest. Wasser |
| 0,1 | Dinatrium-EDTA |
| 25,0 | Natrosol (4 %ige wässrige Lösung) |
| 0,3 | Konservierungsmittel |
| | |

| B | |
|---|---|
| 0,5 | dest. Wasser |
| 0,5 | Triethanolamin |
| | |

| C | |
|---|---|
| 9,0 | Polymer 1 (25 %ige wässrige Lösung) |
| 1,0 | Polyquaternium-46 (20 %ige wässrige Lösung) |
| 5,0 | Eisenoxid |
| | |

| D | |
|---|---|
| 15,0 | dest. Wasser |
| 1,0 | D-Panthenol 50 P (Panthenol und Propylenglykol) |

### Herstellung:

Mit einem Propellermischer die Einsatzstoffe von Phase A in der angegebenen Reihenfolge gut mischen. Dann Phase B in Phase A hineingeben. Langsam rühren bis alles homogen ist. Phase C gut homogenisieren, bis die Pigmente gut verteilt sind. Phase C und Phase D zu Phase AB geben und gut mischen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein schimmerndes Gel mit guten Eigenschaften erhalten.

### Beispiel 15: Sonnenschutz-Gel

| Phase A | |
|---|---|
| 1,00 | hydriertes Ricinusöl-PEG-40 |
| 8,00 | Octyl Methoxycinnamate (Uvinul MC 80TM von BASF) |
| 5,00 | Octocrylene (Uvinul N 539 TM von BASF) |
| 0,80 | Octyl Triazone (Uvinul T 150 TM von BASF) |
| 2,00 | Butyl Methoxydibenzoylmethane (Uvinul BMBM TM von BASF) |
| 2,00 | Tocopherylacetat |
| q.s. | Parfümöl |
| | |

| Phase B | |
|---|---|
| 12,50 | Polymer 1 (20 %ige wässrige Lösung) |
| 0,30 | Acrylat/C₁₀₋₃₀ Alkylacrylat-Copolymer |
| 0,20 | Carbomer |
| 5,00 | Glycerin |
| 0,20 | Dinatrium-EDTA |
| q.s. | Konservierungsmittel |
| 62,80 | dest. Wasser |
| | |

| Phase C | |
|---|---|
| 0,20 | Natriumhydroxid |

### Herstellung:

Die Komponenten der Phase A mischen. Phase B quellen lassen und unter Homogenisieren in Phase A einrühren. Mit Phase C neutralisieren und erneut homogenisieren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Sonnenschutz-Gel mit guten Eigenschaften erhalten.

### Beispiel 16: Sonnenschutzemulsion mit TiO₂ und ZnO₂

| Phase A | |
|---|---|
| 6,00 | hydriertes Ricinusöl-PEG-7 |
| 2,00 | PEG-45/Dodecyl Glycol Copolymer |
| 3,00 | Isopropylmyristat |
| 8,00 | Jojobaöl (Buxus Chinensis) |
| 4,00 | Octyl Methoxycinnamate (Uvinul MC 80) |
| 2,00 | 4-Methylbenzylidene Camphor (Uvinul MBC 95) |
| 3,00 | Titandioxid, Dimethicon |
| 1,00 | Dimethicon |
| 5,00 | Zinkoxid, Dimethicon |
| | |

| Phase B | |
|---|---|
| 10,00 | Polymer 1 (20 %ige wässrige Lösung) |
| 0,20 | Dinatrium-EDTA |
| 5,00 | Glycerin |
| q.s. | Konservierungsmittel |
| 50,80 | dest. Wasser |
| | |

| Phase C | |
|---|---|
| q.s. | Parfümöl |

### Herstellung:

Die Phasen A und B getrennt auf ca. 85 °C erwärmen. Phase B in Phase A einrühren und homogenisieren. Abkühlen auf ca. 40 °C, Phase C hinzugeben und nochmals kurz homogenisieren.

Das Beispiel lässt sich mit den vernetzten Polyurethanen der Beispiele 2, 3, 4, 5, 6, 7, 8, 9, 10. 11, 12, 13 und 14 wiederholen. Es wird in jedem Fall eine Sonnenschutzemulsion mit TiO₂ und ZnO₂ mit guten Eigenschaften erhalten.

### Beispiel 17: Sonnenschutz-Lotion

| Phase A | |
|---|---|
| 6,00 | Octyl Methoxycinnamate (Uvinul MC 80 TM von BASF) |
| 2,50 | 4-Methylbenzylidene Camphor (Uvinul MBC 95 TM von BASF) |
| 1,00 | Octyl Triazone (Uvinul T 150 TM von BASF) |
| 2,00 | Butyl Methoxydibenzoylmethane (Uvinul BMBM TM von BASF) |
| 2,00 | PVP/Hexadecen-Copolymer |
| 5,00 | PPG-3 Myristyl Ether |
| 0,50 | Dimethicon |
| 0,10 | BHT, Ascorbylpalmitat, Citronensäure, Glycerylstearat |
| | Propylenglykol |
| 2,00 | Cetylalkohol |
| 2,00 | Kaliumcetylphosphat |
| | |

| Phase B | |
|---|---|
| 2,50 | Polymer 1 (20 %ige wässrige Lösung) |
| 5,00 | Propylenglykol |
| 0,20 | Dinatrium-EDTA |
| q.s. | Konservierungsmittel |
| 63,92 | dest. Wasser |
| | |

| Phase C | |
|---|---|
| 5,00 | Mineralöl |
| 0,20 | Carbomer |
| | |

| Phase D | |
|---|---|
| 0,08 | Natriumhydroxid |
| | |

| Phase E | |
|---|---|
| q.s. | Parfümöl |

### Herstellung:

Die Phasen A und B getrennt auf ca. 80 °C erwärmen. Phase B unter Homogenisieren in Phase A einrühren, kurz nachhomogenisieren. Phase C anschlämmen, in Phase AB einrühren, mit Phase D neutralisieren und nachhomogenisieren. Abkühlen auf ca. 40°C, Phase E zugeben, nochmals homogenisieren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall eine Sonnenschutz-Lotion mit guten Eigenschaften erhalten.

### Beispiel 18: Abziehbare Gesichtsmaske

| Phase A | |
|---|---|
| 57,10 | dest. Wasser |
| 6,00 | Polyvinylalkohol |
| 5,00 | Propylenglykol |
| | |

| Phase B | |
|---|---|
| 20,00 | Alkohol |
| 4,00 | PEG-32 |
| q.s | Parfümöl |
| | |

| Phase C | |
|---|---|
| 5,00 | Polyquatemium-44 |
| 2,70 | Polymer 1 (20 %ige wässrige Lösung) |
| 0,20 | Allantoin |

### Herstellung:

Phase A auf mind. 90 °C erwärmen und rühren bis gelöst. Phase B bei 50 °C lösen und in Phase A einrühren. Bei ca. 35 °C den Ethanolverlust ausgleichen. Phase C zugeben und unterrühren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall eine abziehbare Gesichtsmaske mit guten Eigenschaften erhalten.

### Beispiel 19: Gesichtsmaske

| Phase A | |
|---|---|
| 3,00 | Ceteareth-6 |
| 1,50 | Ceteareth-25 |
| 5,00 | Cetearylalkohol |
| 6,00 | Cetearyloctanoat |
| 6,00 | Mineralöl |
| 0,20 | Bisabolol |
| 3,00 | Glycerylstearat |
| | |

| Phase B | |
|---|---|
| 2,00 | Propylenglykol |
| 5,00 | Panthenol |
| 14,00 | Polymer 1 (20 %ige wässrige Lösung) |
| q.s. | Konservierungsmittel |
| 53,80 | dest. Wasser |
| | |

| Phase C | |
|---|---|
| q.s. | Parfümöl |
| 0,50 | Tocopherylacetat |

### Herstellung:

Phase A und B getrennt auf ca. 80 °C erwärmen. Phase B in Phase A unter Homogenisieren einrühren, kurz nachhomogenisieren. Abkühlen auf ca. 40 °C, Phase C hinzugeben, nochmals homogenisieren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall eine Gesichtsmaske mit guten Eigenschaften erhalten.

### Beispiel 20: Körperlotion-Schaum

| Phase A | |
|---|---|
| 1,50 | Ceteareth-25 |
| 1,50 | Ceteareth-6 |
| 4,00 | Cetearylalkohol |
| 10,00 | Cetearyloctanoat |
| 1,00 | Dimethicon |
| | |

| Phase B | |
|---|---|
| 3,00 | Polymer 1 (20 %ige wässrige Lösung) |
| 2,00 | Panthenol |
| 2,50 | Propylenglykol |
| q.s. | Konservierungsmittel |
| 74,50 | dest. Wasser |
| | |

| Phase C | |
|---|---|
| q.s. | Parfümöl |

### Herstellung:

Die Phasen A und B getrennt auf ca. 80 °C erwärmen. Phase B in Phase A einrühren und homogenisieren. Abkühlen auf ca. 40 °C, Phase C hinzugeben und nochmals kurz homogenisieren. Abfüllung: 90 % Wirkstoff und 10 % Propan/Butan bei 3,5 bar (20 °C).

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Körperlotion-Schaum mit guten Eigenschaften erhalten.

### Beispiel 21: Gesichtswasser für trockene und empfindliche Haut

| Phase A | |
|---|---|
| 2,50 | hydriertes Rizinusöl-PEG-40 |
| q.s. | Parfümöl |
| 0,40 | Bisabolol |
| | |

| Phase B | |
|---|---|
| 3,00 | Glycerin |
| 1,00 | Hydroxyethylcetyldimoniumphosphat |
| 5,00 | Zaubernuss (Hamamelis Virginiana) Destillat |
| 0,50 | Panthenol |
| 0,50 | Polymer 1 (25 %ige wässrige Lösung) |
| q.s. | Konservierungsmittel |
| 87,60 | dest. Wasser |

### Herstellung:

Phase A klar lösen. Phase B in Phase A einrühren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Gesichtswasser für trockene und empfindliche Haut mit guten Eigenschaften erhalten.

### Beispiel 22: Gesichtswaschpaste mit Peelingeffekt

| Phase A | |
|---|---|
| 58,00 | dest. Wasser |
| 15,00 | Polymer 1 (20 %ige wässrige Lösung) |
| 1,50 | Carbomer |
| q.s. | Konservierungsmittel |
| | |

| Phase B | |
|---|---|
| q.s. | Parfümöl |
| 7,00 | Potassium Cocoyl Hydrolyzed Protein |
| 4,00 | Cocamidpropylbetain |
| | |

| Phase C | |
|---|---|
| 1,50 | Triethanolamin |
| | |

| Phase D | |
|---|---|
| 13,00 | Polyethylen (Luwax ATM von BASF) |

### Herstellung:

Phase A quellen lassen. Phase B klar lösen. Phase B in Phase A einrühren. Mit Phase C neutralisieren. Danach Phase D einrühren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall eine Gesichtswaschpaste mit Peelingeffekt mit guten Eigenschaften erhalten.

### Beispiel 23: Gesichtsseife

| Phase A | |
|---|---|
| 25,0 | Kaliumcocoat |
| 20,0 | Disodium Cocoamphodiacetate |
| 2.0 | Lauramide DEA |
| 1,0 | Glykolstearat |
| 2,0 | Polymer 1 (25 %ige wässrige Lösung) |
| 50,0 | dest. Wasser |
| q.s. | Citronensäure |
| | |

| Phase B | |
|---|---|
| q.s. | Konservierungsmittel |
| q.s. | Parfumöl |

### Herstellung:

Phase A unter Rühren auf 70 °C erwärmen, bis alles homogen ist, pH-Wert auf 7,0 - 7,5 mit Zitronensäure einstellen, alles auf 50 °C abkühlen lassen und Phase B zugeben.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall eine Gesichtsseife mit guten Eigenschaften erhalten.

### Beispiel 24: Gesichtsreinigungsmilch, Typ O/W

| Phase A | |
|---|---|
| 1,50 | Ceteareth-6 |
| 1,50 | Ceteareth-25 |
| 2,00 | Glycerylstearat |
| 2,00 | Cetylalkohol |
| 10,00 | Mineralöl |
| | |

| Phase B | |
|---|---|
| 5,00 | Propylenglykol |
| q.s. | Konservierungsmittel |
| 5,0 | Polymer 1 (20 %ige wässrige Lösung) |
| 62,30 | dest. Wasser |
| | |

| Phase C | |
|---|---|
| 0,20 | Carbomer |
| 10,00 | Cetearyloctanoat |
| | |

| Phase D | |
|---|---|
| 0.40 | Tetrahydroxypropylethylendiamin |
| | |

| Phase E | |
|---|---|
| q.s. | Parfümöl |
| 0,10 | Bisabolol |

### Herstellung:

Die Phasen A und B getrennt auf ca. 80 °C erwärmen. Phase B unter Homogenisieren in Phase A einrühren, kurz nachhomogenisieren. Phase C anschlämmen, in Phase AB einrühren, mit Phase D neutralisieren und nachhomogenisieren. Abkühlen auf ca. 40 °C, Phase E zugeben, nochmals homogenisieren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall eine Gesichtsreinigungsmilch, Typ O/W mit guten Eigenschaften erhalten.

### Beispiel 25: Peeling-Creme, Typ O/W

| Phase A | |
|---|---|
| 3,00 | Ceteareth-6 |
| 1,50 | Ceteareth-25 |
| 3,00 | Glycerylstearat |
| 5,00 | Cetearylalkohol, Sodium Cetearyl Sulfate |
| 6,00 | Cetearyloctanoat |
| 6,00 | Mineralöl |
| 0,20 | Bisabolol |
| | |

| Phase B | |
|---|---|
| 2,00 | Propylenglykol |
| 0,10 | Dinatrium-EDTA |
| 3,00 | Polymer 1 (25 %ige wässrige Lösung) |
| q.s. | Konservierungsmittel |
| 59,70 | dest. Wasser |
| | |

| Phase C | |
|---|---|
| 0,50 | Tocopherylacetat |
| q.s. | Parfümöl |
| | |

| Phase D | |
|---|---|
| 10,00 | Polyethylen |

### Herstellung:

Die Phasen A und B getrennt auf ca. 80 °C erwärmen. Phase B in Phase A einrühren und homogenisieren. Abkühlen auf ca. 40 °C, Phase C hinzugeben und nochmals kurz homogenisieren. Anschließend Phase D unterrühren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall eine Peeling-Creme, Typ O/W mit guten Eigenschaften erhalten.

### Beispiel 26: Rasierschaum

| | |
|---|---|
| 6,00 | Ceteareth-25 |
| 5,00 | Poloxamer 407 |
| 52,00 | dest. Wasser |
| 1,00 | Triethanolamin |
| 5,00 | Propylenglykol |
| 1,00 | Lanolinöl-PEG-75 |
| 5,00 | erfindungsgemäßes Polymer 1 (20 %ige wässrige Lösung) |
| q.s. | Konservierungsmittel |
| q.s. | Parfümöl |
| 25,00 | Sodium Laureth Sulfate |

### Herstellung:

Alles zusammen wiegen, danach rühren bis gelöst. Abfüllung: 90 Teile Wirksubstanz und 10 Teile Propan/Butan-Mischung 25:75.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Rasierschaum mit guten Eigenschaften erhalten.

### Beispiel 27: After Shave Balsam

| Phase A | |
|---|---|
| 0,25 | Acrylat/C₁₀₋₃₀ Alkylacrylat-Copolymer |
| 1,50 | Tocopherylacetat |
| 0,20 | Bisabolol |
| 10,00 | Capryl/Caprin-Triglycerid |
| q.s. | Parfümöl |
| 1,00 | hydriertes Rizinusöl-PEG-40 |
| | |

| Phase B | |
|---|---|
| 1,00 | Panthenol |
| 15,00 | Alkohol |
| 5,00 | Glycerin |
| 0,05 | Hydroxyethylcellulose |
| 1,92 | Polymer 1 (25 %ige wässrige Lösung) |
| 64,00 | dest. Wasser |
| | |

| Phase C | |
|---|---|
| 0,08 | Natriumhydroxid |

### Herstellung:

Die Komponenten der Phase A mischen. Phase B unter Homogenisieren in Phase A einrühren, kurz nachhomogenisieren. Mit Phase C neutralisieren und erneut homogenisieren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein After Shave Balsam mit guten Eigenschaften erhalten.

### Beispiel 28: Körperpflegecreme

| Phase A | |
|---|---|
| 2,00 | Ceteareth-6 |
| 2,00 | Ceteareth-25 |
| 2,00 | Cetearylalkohol |
| 3,00 | Glycerylstearat SE |
| 5,00 | Mineralöl |
| 4,00 | Jojobaöl (Buxus Chinensis) |
| 3,00 | Cetearyloctanoat |
| 1,00 | Dimethicon |
| 3,00 | Mineralöl, Lanolinalkohol |
| | |

| Phase B | |
|---|---|
| 5,00 | Propylenglykol |
| 0,50 | Veegum |
| 1,00 | Panthenol |
| 8,50 | Polymer 1 (20 %ige wässrige Lösung) |
| 6,00 | Polyquatemium-44 (10 %ige wässrige Lösung) |
| q.s. | Konservierungsmittel |
| 54,00 | dest. Wasser |
| | |

| Phase C | |
|---|---|
| q.s. | Parfümöl |

### Herstellung:

Die Phasen A und B getrennt auf ca. 80 °C erwärmen. Phase B homogenisieren. Phase B unter Homogenisieren in Phase A einrühren, kurz nachhomogenisieren. Abkühlen auf ca. 40 °C, Phase C hinzugeben und nochmals kurz homogenisieren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall eine Körperpflegecreme mit guten Eigenschaften erhalten.

### Beispiel 29: Zahnpasta

| Phase A | |
|---|---|
| 34,79 | dest. Wasser |
| 3,00 | Polymer 1 (25 %ige wässrige Lösung) |
| 0,30 | Konservierungsmittel |
| 20,00 | Glycerin |
| 0,76 | Natriummonofluorphosphat |
| | |

| Phase B | |
|---|---|
| 1,20 | Natriumcarboxymethylcellulose |
| | |

| Phase C | |
|---|---|
| 0,80 | Aromaöl |
| 0,06 | Saccharin |
| 0,10 | Konservierungsmittel |
| 0,05 | Bisabolol |
| 1,00 | Panthenol |
| 0,50 | Tocopherylacetat |
| 2,80 | Siliciumdioxid |
| 1,00 | Natriumlaurylsulfat |
| 7,90 | Dicalciumphosphat, wasserfrei |
| 25,29 | Dicalciumphosphat-Dihydrat |
| 0,45 | Titandioxid |

### Herstellung:

Phase A lösen. Phase B in Phase A einstreuen und lösen. Phase C zugeben und unter Vakuum bei RT ca. 45 Min. rühren lassen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall eine Zahnpasta mit guten Eigenschaften erhalten.

### Beispiel 30: Mundwasser

| Phase A | |
|---|---|
| 2,00 | Aromaöl |
| 4,00 | hydriertes Rizinusöl-PEG-40 |
| 1,00 | Bisabolol |
| 30,00 | Alkohol |
| | |

| Phase B | |
|---|---|
| 0,20 | Saccharin |
| 5,00 | Glycerin |
| q.s. | Konservierungsmittel |
| 5,00 | Poloxamer 407 |
| 2,5 | Polymer 1 (20 %ige wässrige Lösung) |
| 50,30 | dest. Wasser |

### Herstellung:

Phase A und Phase B getrennt klar lösen. Phase B in Phase A einrühren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Mundwasser mit guten Eigenschaften erhalten.

### Beispiel 31: Prothesenhaftmittel

| Phase A | |
|---|---|
| 0,20 | Bisabolol |
| 1,00 | Betacarotin |
| q.s. | Aromaöl |
| 20,00 | Cetearyloctanoat |
| 5,00 | Siliciumdioxid |
| 33,80 | Mineralöl |
| | |

| Phase B | |
|---|---|
| 5,00 | Polymer 1 (20 %ige wässrige Lösung) |
| 35,00 | PVP (20 %ige Lösung in Wasser) |

### Herstellung:

Phase A gut mischen. Phase B in Phase A einrühren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Prothesenhaftmittel mit guten Eigenschaften erhalten.

### Beispiel 32: Hautpflegecreme, Typ O/W

| Phase A | |
|---|---|
| 8,00 | Cetearylalkohol |
| 2,00 | Ceteareth-6 |
| 2,00 | Ceteareth-25 |
| 10,00 | Mineralöl |
| 5,00 | Cetearyloctanoat |
| 5,00 | Dimethicon |
| | |

| Phase B | |
|---|---|
| 3,00 | Polymer 1 (25 %ige wässrige Lösung) |
| 2,00 | Panthenol, Propylenglykol |
| q.s. | Konservierungsmittel |
| 63,00 | dest. Wasser |
| | |

| Phase C | |
|---|---|
| q.s. | Parfümöl |

### Herstellung:

Phase A und B getrennt auf ca. 80 °C erwärmen. Phase B in Phase A unter Homogenisieren einrühren, kurz nachhomogenisieren. Abkühlen auf ca. 40 °C, Phase C hinzugeben, nochmals homogenisieren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall eine Hautpflegecreme, Typ O/W mit guten Eigenschaften erhalten.

### Beispiel 33: Hautpflegecreme, Typ W/O

| Phase A | |
|---|---|
| 6,00 | hydriertes Rizinusöl-PEG-7 |
| 8,00 | Cetearyloctanoat |
| 5,00 | Isopropylmyristat |
| 15,00 | Mineralöl |
| 2,00 | PEG-45/Dodecyl Glycol Copolymer |
| 0,50 | Magnesiumstearat |
| 0,50 | Aluminumstearat |
| | |

| Phase B | |
|---|---|
| 3,00 | Glycerin |
| 3,30 | Polymer 1 (20 %ige wässrige Lösung) |
| 0,70 | Magnesiumsulfat |
| 2,00 | Panthenol |
| q.s. | Konservierungsmittel |
| 48,00 | dest. Wasser |
| | |

| Phase C | |
|---|---|
| 1,00 | Tocopherol |
| 5,00 | Tocopherylacetat |
| q.s. | Parfümöl |

### Herstellung:

Die Phasen A und B getrennt auf ca. 80 °C erwärmen. Phase B in Phase A einrühren und homogenisieren. Abkühlen auf ca. 40 °C, Phase C hinzugeben und nochmals kurz homogenisieren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall eine Hautpflegecreme, Typ W/O mit guten Eigenschaften erhalten.

### Beispiel 34: Lippenpflegecreme

| Phase A | |
|---|---|
| 10,00 | Cetearyloctanoat |
| 5,00 | Polybuten |
| | |

| Phase B | |
|---|---|
| 0,10 | Carbomer |
| | |

| Phase C | |
|---|---|
| 2,00 | Ceteareth-6 |
| 2,00 | Ceteareth-25 |
| 2,00 | Glycerylstearat |
| 2,00 | Cetylalkohol |
| 1,00 | Dimethicon |
| 1,00 | Benzophenone-3 |
| 0,20 | Bisabolol |
| 6,00 | Mineralöl |
| | |

| Phase D | |
|---|---|
| 8,00 | Polymer 1 (20 %ige wässrige Lösung) |
| 3,00 | Panthenol |
| 3,00 | Propylenglykol |
| q.s. | Konservierungsmittel |
| 54,00 | dest. Wasser |
| | |

| Phase E | |
|---|---|
| 0,10 | Triethanolamin |
| | |

| Phase F | |
|---|---|
| 0,50 | Tocopherylacetat |
| 0,10 | Tocopherol |
| q.s. | Parfümöl |

### Herstellung:

Phase A klar lösen. Phase B dazugeben und homogenisieren. Phase C addieren und schmelzen bei 80 °C. Phase D auf 80 °C erwärmen. Phase D zu Phase ABC geben und homogenisieren. Abkühlen auf ca. 40 °C, Phase E und Phase F zugeben, nochmals homogenisieren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall eine Lippenpflegecreme mit guten Eigenschaften erhalten.

### Beispiel 35: Duschgel

| | |
|---|---|
| 50,00 | Sodium Laureth Sulfate, Magnesium Laureth Sulfate, Sodium Laureth-8 Sulfate, Magnesium Laureth-8 |
| 1,00 | Cocoamide DEA |
| 4,00 | Polymer 1 (25 %ige wässrige Lösung) |
| 2,00 | Sodium Laureth Sulfate, Glycol Distearate, Cocamide MEA, Laureth-10 |
| q.s. | Konservierungsmittel |
| q.s. | Parfümöl |
| 2,00 | Natriumchlorid |
| 41,00 | dest. Wasser |

### Herstellung:

Alle Komponenten gemeinsam einwiegen und bis zur Lösung rühren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Duschgel mit guten Eigenschaften erhalten.

### Beispiel 36: Duschgel

| | |
|---|---|
| 30,00 | Sodium Laureth Sulfate |
| 6,00 | Sodium Cocoamphodiacetate |
| 6,00 | Cocamidopropylbetain |
| 3,00 | Sodium Laureth Sulfate, Glycol Distearate, Cocamide MEA, Laureth-10 |
| 7,70 | Polyquaternium-44 |
| 1,50 | Polymer 1 (25 %ige wässrige Lösung) |
| 1,00 | Panthenol |
| q.s. | Konservierungsmittel |
| q.s. | Parfümöl |
| q.s. | Citronensäure |
| 0,50 | Natriumchlorid |
| 44,30 | dest. Wasser |

### Herstellung:

Die Komponenten der Phase A einwiegen und lösen. Den pH-Wert auf 6 bis 7 einstellen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Duschgel mit guten Eigenschaften erhalten.

### Beispiel 37: Klares Duschgel

| | |
|---|---|
| 40,00 | Sodium Laureth Sulfate |
| 5,00 | Decylglukosid |
| 5,00 | Cocamidopropylbetain |
| 0,50 | Polyquaternium-10 |
| 11,00 | Polymer 1 (20 %ige wässrige Lösung) |
| 1,00 | Panthenol |
| q.s. | Parfümöl |
| q.s. | Konservierungsmittel |
| q.s. | Citronensäure |
| 2,00 | Natriumchlorid |
| 35,50 | dest. Wasser |

### Herstellung:

Die Komponenten der Phase A einwiegen und klar lösen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein klares Duschgel mit guten Eigenschaften erhalten.

### Beispiel 38: Duschbad

| A | |
|---|---|
| 40,00 | Sodium Laureth Sulfate |
| 5,00 | Sodium C₁₂₋₁₅ Pareth-15 Sulfonate |
| 5,00 | Decylglukosid |
| q.s. | Parfümöl |
| 0,10 | Phytantriol |
| | |

| B | |
|---|---|
| 34,80 | dest. Wasser |
| 0,1 | Guarhydroxypropyltrimoniumchlorid |
| 11,00 | Polymer 1 (20 %ige wässrige Lösung) |
| 1,00 | Panthenol |
| q.s. | Konservierungsmittel |
| 1,00 | Laureth-3 |
| q.s. | Citronensäure |
| 2,00 | Natriumchlorid |

### Herstellung:

Die Komponenten der Phase A mischen. Die Komponenten der Phase B nacheinander zugeben und mischen. Den pH-Wert auf 6 bis 7 einstellen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Duschbad mit guten Eigenschaften erhalten.

### Beispiel 39: Flüssigseife

| A | |
|---|---|
| 43,26 | dest. Wasser |
| 0,34 | Aminomethylpropanol |
| 3,40 | Poly(Ethylacrylat/Methacrylsäure) (Luviflex Soft®, Fa. BASF) |
| | |

| B | |
|---|---|
| 40,00 | Sodium Laureth Sulfate |
| 10,00 | Cocamidopropylbetain |
| 1,00 | Polymer 1 (20 %ige wässrige Lösung) |
| q.s. | Parfümöl |
| q.s. | Konservierungsmittel |
| 2,00 | Natriumchlorid |

### Herstellung:

Die Komponenten der Phase A einwiegen und klar lösen. Die Komponenten der Phase B nacheinander zugeben und mischen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall eine Flüssigseife mit guten Eigenschaften erhalten.

### Beispiel 40: Flüssiges Fußbad

| A | |
|---|---|
| 1,00 | Nonoxynol-14 |
| 0,10 | Bisabolol |
| 1,00 | Pinienöl (Pinus Sylvestris) |
| | |

| B | |
|---|---|
| 5,00 | PEG-8 |
| 6,00 | Polymer 1 (20 %ige wässrige Lösung) |
| 0,50 | Triclosan |
| 30,00 | Sodium Laureth Sulfate |
| 3,00 | Polyquaternium-16 |
| 53,40 | dest. Wasser |
| q.s. | C. I. 19 140 + C. I. 42 051 |

### Herstellung:

### Phase A solubilisieren. Phase B mischen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein flüssiges Fußbad mit guten Eigenschaften erhalten.

### Beispiel 41: Erfrischungsgel

| A | |
|---|---|
| 0,60 | Carbomer |
| 45,40 | dest. Wasser |
| | |

| B | |
|---|---|
| 0,50 | Bisabolol |
| 0,50 | Farnesol |
| q.s. | Parfümöl |
| 5,00 | PEG-40 Hydrogenated Castor Oil |
| 2,50 | Polymer 1 (20 %ige wässrige Lösung) |
| 1,00 | Tetrahydroxypropylethylendiamin |
| 1,50 | Menthol |
| 43,00 | Alkohol |
| q.s. | C.I. 74 180, Direct Blue 86 |

### Herstellung:

Phase A quellen lassen. Phase B lösen. Phase B in Phase A einrühren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Erfrischungsgel mit guten Eigenschaften erhalten.

### Beispiel 42: Roll-on Antiperspirant

| A | |
|---|---|
| 0,40 | Hydroxyethylcellulose |
| 50,00 | dest. Wasser |
| | |

| B | |
|---|---|
| 25,00 | Alkohol |
| 0,10 | Bisabolol |
| 0,30 | Farnesol |
| 2,00 | PEG-40 Hydrogenated Castor Oil |
| q.s. | Parfümöl |
| | |

| C | |
|---|---|
| 5,00 | Aluminumchlorhydrat |
| 3,00 | Propylenglykol |
| 3,00 | Dimethicon-Copolyol |
| 3,00 | Polyquaternium-16 |
| 6,00 | Polymer 1 (20 %ige wässrige Lösung) |
| 2,20 | dest. Wasser |

### Herstellung:

Phase A quellen lassen. Phase B und C getrennt lösen. Phase A und B in Phase C einrühren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Roll-on Antiperspirant mit guten Eigenschaften erhalten.

### Beispiel 43: Transparenter Deostift

| | |
|---|---|
| 5,00 | Natriumstearat |
| 0,50 | Triclosan |
| 3,00 | Ceteareth-25 |
| 20,00 | Glycerin |
| 2,50 | Polymer 1 (20 %ige wässrige Lösung) |
| q.s. | Parfümöl |
| 60,00 | Propylenglykol |
| 0,20 | Bisabolol |
| 10,80 | dest. Wasser |

### Herstellung:

Phase A zusammenwiegen, schmelzen und homogenisieren. Anschließend in die Form gießen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein transparenter Deostift mit guten Eigenschaften erhalten.

### Beispiel 44: Wasserlösliches Badeöl

| | |
|---|---|
| 15,00 | Cetearyloctanoat |
| 15,00 | Capryl/Caprin-Triglycerid |
| 1,00 | Panthenol, Propylenglykol |
| 0,10 | Bisabolol |
| 2,00 | Tocopherylacetat |
| 2,00 | Retinylpalmitat |
| 0,10 | Tocopherol |
| 37,00 | PEG-7-Glyceryl-Cocoate |
| 2,00 | Polymer 1 (20 %ige wässrige Lösung) |
| 2,20 | dest. Wasser |
| q.s. | Parfümöl |
| 23,60 | PEG-40 Hydrogenated Castor Oil |

### Herstellung:

Mischen und rühren bis alles klar gelöst ist.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein wasserlösliches Badeöl mit guten Eigenschaften erhalten.

### Beispiel 45: Tagespflege-Aerosol

| A | |
|---|---|
| 4,00 | Ethylhexyl Methoxycinnamate |
| 1,50 | Octocrylen |
| 9,00 | Capryl/Caprin-Triglycerid |
| 5,00 | Simmondsia Chinensis (Jojoba) Seed Oil |
| 1,50 | Cyclomethicon |
| 3,00 | Hydrogenated Coco-Glycerides |
| 1,00 | PVP/Hexadecen-Copolymer |
| 1,00 | Ceteareth-6, Stearylalkohol |
| | |

| B | |
|---|---|
| 5,00 | Zinkoxid |

| C | |
|---|---|
| 2,00 | Ceteareth-25 |
| 1,20 | Panthenol |
| 0,20 | Sodium Ascorbyl Phosphate |
| 0,30 | Imidazolidinyl-Hamstoff |
| 0,10 | Disodium EDTA |
| 7,50 | Polymer 1 (20 %ige wässrige Lösung) |
| 56,67 | dest. Wasser |
| | |

| D | |
|---|---|
| 0,50 | Tocopherylacetat |
| 0,20 | Bisabolol |
| 0,33 | Capryl/Caprin-Triglycerid, Retinol |
| q.s. | Parfümöl |

### Herstellung:

Phase A auf 80 °C erwärmen. Phase A klar lösen. Phase B einarbeiten und homogenisieren. Phase C zugeben, erhitzen auf 80 °C, aufschmelzen und homogenisieren. Unter Rühren auf ca. 40 °C abkühlen, Phase D hinzugeben und kurz homogenisieren. 90 % Wirkstofflösung: 10 % Propan/Butan mit 3,5 bar (20 °C) abfüllen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Tagespflege-Aerosol mit guten Eigenschaften erhalten.

### Beispiel 46: Feuchtigkeitscreme

| A | |
|---|---|
| 3,00 | Vitis Vinifera (Grape) Seed Oil |
| 1,00 | Cyclopentasiloxan, Cyclohexasiloxan |
| 1,50 | Cyclomethicon |
| 2,00 | Soybean (Glycine Soja) Oil |
| 2,00 | Ethylhexyl Methoxycinnamate |
| 1,00 | Uvinul A Plus (BASF) |
| 1,00 | Hydrogenated Lecithin |
| 1,00 | Cholesterol |
| 2,00 | PEG-40 Hydrogenated Castor Oil |
| 5,00 | Cetearyloctanoat |
| 5,00 | Capryl/Caprin-Triglycerid |
| | |

| B | |
|---|---|
| 3,00 | Capryl/caprin-Triglycerid, Acrylat-Copolymer |
| | |

| C | |
|---|---|
| 3,00 | Polymer 1 (20 %ige wässrige Lösung) |
| 0,50 | Cocotrimonium Methosulfat |
| 2,00 | Panthenol, Propylenglykol |
| 3,00 | Glycerin |
| 0,10 | Dinatrium-EDTA |
| 60,30 | dest. Wasser |
| | |

| D | |
|---|---|
| 0,30 | Parfümöl |
| 0,30 | DMDM Hydantoin |
| 1,00 | Tocopherylacetat |
| 2,00 | Tocopherol |

### Herstellung:

Phase A auf 80°C erwärmen. Phase B in Phase A einrühren. Phase C auf ca. 80 °C erwärmen und unter Homogenisieren in Phase A+B einrühren. Unter Rühren auf ca. 40 °C abkühlen, Phase D hinzugeben und kurz homogenisieren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall eine Feuchtigkeitscreme mit guten Eigenschaften erhalten.

### Beispiel 47: Aerosol-Haarschaum

| A | |
|---|---|
| 2,00 | Cocotrimonium Methosulfat |
| 0,20 | Parfümöl |
| | |

| B | |
|---|---|
| 63,90 | dest. Wasser |
| 6,70 | Polymer 1 (25 %ige wässrige Lösung) |
| 0,50 | Poly(Ethylacrylat/Methacrylsäure) (Luviflex Soft®, Fa. BASF) |
| 0,10 | Aminomethylpropanol |
| 0,20 | Ceteareth-25 |
| 0,20 | Trimethylsilylamodimethicon, Trideceth-10, Cetrimonium Chloride |
| 0,10 | PEG-25 PABA |
| 0,20 | Hydroxyethylcellulose |
| 0,20 | PEG-8 |
| 0,20 | Panthenol |
| 15,00 | Alkohol |
| | |

| C | |
|---|---|
| 10,00 | Propan/Butan 3,5 bar (20 °C) |

### Herstellung:

Phasen A und B mischen und mit Treibgas abfüllen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Aerosol-Haarschaum mit guten Eigenschaften erhalten.

### Beispiel 48: Pumpmousse

| A | |
|---|---|
| 2,00 | Cocotrimonium Methosulfat |
| q.s. | Parfümöl |
| | |

| B | |
|---|---|
| 74,30 | dest. Wasser |
| 7,00 | Polyquatemium-46 (10 %ige wässrige Lösung) |
| 15,00 | Polymer 1 (20 %ige wässrige Lösung) |
| 0,50 | PEG-8 |
| 1,00 | Panthenol |
| q.s. | Konservierungsmittel |
| 0,20 | PEG-25 PABA (ethoxilierte p-Aminobenzoesäure) |

### Herstellung:

Die Komponenten der Phase A mischen. Die Komponenten der Phase B nacheinander zugeben und klar lösen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Pumpmousse mit guten Eigenschaften erhalten.

### Beispiel 49: Aerosolschaum

| | |
|---|---|
| 15,00 | Polymer 1 (20 %ige wässrige Lösung) |
| 5,00 | PVPNA-Copolymer (40 %ige wässrige Lösung) |
| 0,50 | Hydroxyethylcetyldimoniumphosphat |
| 0,20 | Ceteareth-25 |
| 0,40 | Parfümöl PC 910.781/Cremophor |
| 68,90 | dest. Wasser |
| q.s. | Konservierungsmittel |
| 10,00 | Propan/Butan 3,5 bar (20 °C) |

### Herstellung:

Alles zusammen wiegen, rühren bis gelöst, dann abfüllen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Aerosolschaum mit guten Eigenschaften erhalten.

### Beispiel 50: Farbstyling-Mousse

| A | |
|---|---|
| 2,00 | Cocotrimonium Methosulfat |
| q.s. | Parfümöl |
| | |

| B | |
|---|---|
| 33,50 | Polymer 1 (20 %ige wässrige Lösung) |
| 0,50 | Acrylatcopolymer (Luvimer 100 P®, Fa. BASF) |
| 0,10 | Aminomethylpropanol |
| 0,20 | Ceteareth-25 |
| 0,20 | Panthenol |
| 0,20 | Hydroxyethylcellulose |
| 10,00 | Alkohol |
| 43,17 | dest. Wasser |
| 0,08 | C.I. 12245, Basic Red 76 |
| 0,05 | C.I**.** 42510, Basic Violet 14 |
| | |

| C | |
|---|---|
| 10,00 | Propan/Butan 3,5 bar (20 °C) |

### Herstellung:

Alles zusammen wiegen, rühren bis gelöst, dann abfüllen. Nur für dunkelblondes und braunes Haar geeignet!

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Farbstyling-Mousse mit guten Eigenschaften erhalten.

### Beispiel 51: Pumphaarschaum

| A | |
|---|---|
| 1,50 | Cocotrimonium Methosulfat |
| q.s. | Parfümöl |
| | |

| B | |
|---|---|
| 10,00 | Polymer 1 (20 %ige wässrige Lösung) |
| 86,04 | dest. Wasser |
| | |

| C | |
|---|---|
| 0,46 | Aminomethylpropanol |
| 4,00 | PEG/PPG-25/25 Dimethicon/Acrylat-Copolymer |
| q.s. | Konservierungsmittel |

### Herstellung:

Phase A mischen. Phase B in Phase A einrühren. Phase C zugeben und rühren bis gelöst.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Pumphaarschaum mit guten Eigenschaften erhalten.

### Beispiel 52: Aquawax

| | |
|---|---|
| 50,00 | Polymer 1 (20 %ige wässrige Lösung) |
| q.s. | Parfümöl |
| q.s. | hydriertes Rizinusöl-PEG-40 |
| 0,10 | Diethylphthalat |
| 0,10 | Cetearylethylhexanoat |
| 0,10 | PEG-7 Glyceryl Cocoate |
| 0,10 | Konservierungsmittel |
| 47,70 | dest. Wasser |
| 2,00 | Capryl/Caprin-Triglycerid, Acrylat-Copolymer |

### Herstellung:

Alles mischen und homogenisieren. 15 Minuten nachrühren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Aquawax mit guten Eigenschaften erhalten.

### Beispiel 53: Rinse-off Conditioner und Repair Treatment

| A | |
|---|---|
| 0,20 | Cetearyloctanoat |
| 0,10 | Phytantriol |
| 2,00 | hydriertes Rizinusöl-PEG-40 |
| | |

| B | |
|---|---|
| q.s. | Parfümöl |
| 2,00 | Cocotrimonium Methosulfat |
| | |

| C | |
|---|---|
| 77,70 | dest. Wasser |
| | |

| D | |
|---|---|
| 2,00 | Polyquaternium-16 (20 %ige wässrige Lösung) |
| 5,00 | Polymer 1 (20 %ige wässrige Lösung) |
| 1,00 | Dimethicon-Copolyol |
| q.s. | Konservierungsmittel |
| 10,00 | Alkohol |
| q.s. | Citronensäure |

### Herstellung:

Die Phasen A und B getrennt mischen. Phase C in Phase B einrühren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Rinse-off Conditioner und Repair Treatment mit guten Eigenschaften erhalten.

### Beispiel 54: Haarkur

| A | |
|---|---|
| 2,00 | Ceteareth-6, Stearylalkohol |
| 1,00 | Ceteareth-25 |
| 6,00 | Cetearylalkohol |
| 6,00 | Cetearytoctanoat |
| 0,30 | Phytantriol |
| | |

| B | |
|---|---|
| 5,00 | Polymer 1 (20 %ige wässrige Lösung) |
| 0,70 | Guar Hydroxypropytrimonoium Chloride |
| 5,00 | Propylenglykol |
| 2,00 | Panthenol |
| 0,30 | Imidazolidinyl-Harnstoff |
| 69,00 | dest. Wasser |
| | |

| C | |
|---|---|
| 2,00 | Cosi Silk Soluble |
| 0,20 | Parfum |
| 0,50 | Phenoxyethanol |

### Herstellung:

Die Phasen A und B getrennt auf ca. 80 °C erwärmen. Phase B homogenisieren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall eine Haarkur mit guten Eigenschaften erhalten.

### Beispiel 55: Haar-Cocktail

| A | |
|---|---|
| 0,40 | Acrylate/C₁₀₋₃₀ Alkylacrylat-Crosspolymer |
| 2,00 | Dimethicon |
| 3,00 | Cyclomethicon, Dimethiconol |
| 2,00 | Phenyltrimethicon |
| 2,00 | Amodimethicone, Cetrimonium Chloride, Trideceth-10 |
| 0,50 | Dimethicon-Copolyol |
| 1,00. | Macadamia-Nussöl (Temifolia) |
| 0,50 | Tocopherylacetat |
| 1,00 | PEG-40 Hydrogenated Castor Oil |
| q.s. | Parfümöl |
| | |

| B | |
|---|---|
| 81,64 | dest. Wasser |
| 1,50 | Polymer 1 (20 %ige wässrige Lösung) |
| 0,46 | Aminomethylpropanol |
| 4,00 | PEG/PPG-25/25 Dimethicon/Acrylat-Copolymer |

### Herstellung:

Die Komponenten der Phase A mischen. Phase B lösen. Phase B unter Homogenisieren in Phase A einrühren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Haar-Cocktail mit guten Eigenschaften erhalten.

### Beispiel 56: Dauerwelle

### Well-Lösung

| A | |
|---|---|
| 68,95 | dest. Wasser |
| 0,20 | Cocamidopropylbetain |
| 0,20 | Polysorbate 20 |
| 6,25 | erfindungsgemäßes Polymer 1 (20 %ige wässrige Lösung) |
| 0,20 | Dinatrium-EDTA |
| 0,20 | Hydroxyethylcellulose |
| | |

| B | |
|---|---|
| 8,00 | Thioglykolsäure |
| | |

| C | |
|---|---|
| 11,00 | Ammoniumhydroxid |
| | |

| D | |
|---|---|
| 5,00 | Ammoniumcarbonat |

### Herstellung:

Die Komponenten der Phase A einwiegen und klar lösen. Phase B in Phase A einrühren.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall eine Well-Lösung mit guten Eigenschaften erhalten.

Beispiel 57: Fixierung:

| A | |
|---|---|
| 1,00 | PEG-40 Hydrogenated Castor Oil |
| 0,20 | Parfümöl |
| 83,60 | dest. Wasser |
| | |

| B | |
|---|---|
| 0,20 | Cocamidopropylbetain |
| 0,20 | Ceteareth-25 |
| 12,50 | Polymer 1 (20 %ige wässrige Lösung) |
| q.s. | Konservierungsmittel |
| | |

| C | |
|---|---|
| 2,30 | Wasserstoffperoxid |
| | |

| D | |
|---|---|
| q.s. | Phosphorsäure |

### Herstellung:

Phase A solubilisieren. Die Komponenten der Phase B nacheinander zugeben und klar lösen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall eine Fixierung mit guten Eigenschaften erhalten.

### Beispiel 58: dunkelbraune Permanenthaarfarbe (Oxidationshaarfarbe)

| A | |
|---|---|
| 46,90 | dest. Wasser |
| 0,20 | Natriumsulfit |
| 0,05 | Dinatrium-EDTA |
| 0,20 | p-Phenylendiamin |
| 0,30 | Resorcinol |
| 0,20 | 4-Amino-2-hydroxytoluol |
| 0,10 | m-Aminophenol |
| 1,50 | Oleylalkohol |
| 4,50 | Propylenglykol |
| 2,30 | Sodium C₁₂₋₁₅ Pareth-15 Sulfonate |
| 20,00 | Ölsäure |
| | |

| B | |
|---|---|
| 5,00 | Polymer 1 (20 %ige wässrige Lösung) |
| 13,70 | Ammoniumhydroxid |
| 6,00 | i-Propanol |
| q.s. | Parfum |

### Herstellung:

Phase A solubilisieren. Die Komponenten der Phase B nacheinander zugeben und mischen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall eine dunkelbraune Permanenthaarfarbe (Oxidationshaarfarbe) mit guten Eigenschaften erhalten.

### Beispiel 59: Entwickleremulsion (pH 3-4)

| | |
|---|---|
| 3,00 | Hexadecylalkohol |
| 10,00 | Polymer 1 (20 %ige wässrige Lösung) |
| 1,00 | Ceteareth-20 |
| 1,00 | Sodium C₁₂₋₁₅ Pareth-15 Sulfonate |
| 6,00 | Wasserstoffperoxid |
| 0,50 | Phosphorsäure |
| 0,01 | Acetanilid |
| 78,49 | dest. Wasser |

### Herstellung:

Die Komponenten nacheinander zugeben und mischen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall eine Entwickleremulsion (pH 3-4) mit guten Eigenschaften erhalten.

### Beispiel 60: hellbraune Semi-Permanenthaarfarbe

| | |
|---|---|
| 10,00 | Cocodiethanolamid |
| 4,00 | Natriumdodecylbenzylsulfonat, 50 %ig |
| 5,00 | Polymer 1 (20 %ige wässrige Lösung) |
| 6,00 | C₉₋₁₁ Pareth-3 |
| 2,50 | Natriumlaurylsulfat |
| 0,40 | 2-Nitro-p-phenylendiamin |
| 0,20 | HC Red No.3 |
| 0,20 | HC Yellow No.2 |
| 71,70 | dest. Wasser |

### Herstellung:

Die Komponenten nacheinander zugeben und mischen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall eine hellbraune Semi-Permanenthaarfarbe mit guten Eigenschaften erhalten.

### Beispiel 61: Shampoo

| | |
|---|---|
| 30,00 | Sodium Laureth Sulfate |
| 6,00 | Sodium Cocoamphoacetate |
| 6,00 | Cocamidopropylbetain |
| 3,00 | Sodium Laureth Sulfate, Glycol Distearate, Cocamide MEA, Laureth-10 |
| 5,00 | Polymer 1 (20 %ige wässrige Lösung) |
| 2,00 | Dimethicon |
| q.s. | Parfum |
| q.s. | Konservierungsstoff |
| q.s. | Citronensäure |
| 1,00 | Natriumchlorid |
| ad 100 | dest. Wasser |

### Herstellung:

Komponenten einwiegen und lösen. pH-Wert auf 6 bis 7 einstellen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Shampoo mit guten Eigenschaften erhalten.

### Beispiel 62: Shampoo

| | |
|---|---|
| 30,00 | Sodium Laureth Sulfate |
| 6,00 | Sodium Cocoamphoacetate |
| 6,00 | Cocamidopropylbetain |
| 3,00 | Sodium Laureth Sulfate, Glykol Distearate, Cocamide MEA, Laureth-10 |
| 5,00 | Polymer 1 (20 %ige wässrige Lösung) |
| 2,00 | Amodimethicon |
| q.s. | Parfum |
| q.s. | Konservierungsstoff |
| q.s. | Citronensäure |
| 1,00 | Natriumchlorid |
| ad 100 | dest. Wasser |

### Herstellung:

Komponenten einwiegen und lösen. pH-Wert auf 6 bis 7 einstellen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Shampoo mit guten Eigenschaften erhalten.

### Beispiel 63: Shampoo.

| | |
|---|---|
| 40,00 | Sodium Laureth Sulfate |
| 10,00 | Cocamidopropylbetain |
| 3,00 | Sodium Laureth Sulfate, Glykol Distearate, Cocamide MEA, Laureth-10 |
| 5,00 | Polymer 1 (20 %ige wässrige Lösung) |
| 2,00 | Dow Corning 3052 |
| q.s. | Parfum |
| q.s. | Konservierungsstoff |
| q.s. | Citronensäure |
| 2,00 | Cocamido DEA |
| ad 100 | dest. Wasser |

### Herstellung:

Komponenten einwiegen und lösen. pH-Wert auf 6 bis 7 einstellen.

Das Beispiel lässt sich mit den vernetzten Polyurethanen der Beispiele 2, 3, 4, 5, 6, 7, 8, 9, 10, 11. 12. 13 und 14 wiederholen. Es wird in jedem Fall ein Shampoo mit guten Eigenschaften erhalten.

### Beispiel 64: Antischuppen-Shampoo

| | |
|---|---|
| 40,00 | Sodium Laureth Sulfate |
| 10,00 | Cocamidopropylbetain |
| 10,00 | Disodium Laureth Sulfosuccinate |
| 2,50 | Sodium Laureth Sulfate, Glycol Distearate, Cocamide MEA, Laureth-10 |
| 5,00 | Polymer 1 (20 %ige wässrige Lösung) |
| 0,50 | Climbazol |
| q.s. | Parfum |
| q.s. | Konservierungsstoff |
| 0,50 | Natriumchlorid |
| ad 100 | dest. Wasser |

### Herstellung:

Komponenten einwiegen und lösen. pH-Wert auf 6 bis 7 einstellen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Antischuppen-Shampoo mit guten Eigenschaften erhalten.

### Beispiel 65: Shampoo

| | |
|---|---|
| 25,00 | Sodium Laureth Sulfate |
| 5,00 | Cocamidopropylbetain |
| 2,50 | Sodium Laureth Sulfate, Glycol Distearate,Cocamide MEA, Laureth-10 |
| 5,00 | Polymer 1 (20 %ige wässrige Lösung) |
| q.s. | Parfum |
| q.s. | Konservierungsstoff |
| 2,00 | Cocamido DEA |
| ad 100 | dest. Wasser |

### Herstellung:

Komponenten einwiegen und lösen. pH-Wert auf 6 bis 7 einstellen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Shampoo mit guten Eigenschaften erhalten.

### Beispiel 66: Shampoo

| | |
|---|---|
| 20,00 | Ammonium Laureth Sulfate |
| 15,00 | Ammonium Lauryl Sulfate |
| 5,00 | Cocamidopropylbetain |
| 2,50 | Sodium Laureth Sulfate, Glycol Distearate,Cocamide MEA, Laureth-10 |
| 5,00 | Polymer 1 (20 %ige wässrige Lösung) |
| q.s. | Parfum |
| q.s. | Konservierungsstoff |
| 0,50 | Natriumchlorid |
| ad 100 | dest. Wasser |

### Herstellung:

Komponenten einwiegen und lösen. pH-Wert auf 6 bis 7 einstellen.

Das Beispiel lässt sich mit den vernetzten Polyurethanen der Beispiele 2, 3, 4, 5, 6. 7, 8, 9, 10, 11, 12, 13 und 14 wiederholen. Es wird in jedem Fall ein Shampoo mit guten Eigenschaften erhalten.

### Beispiel 67: Klares Duschgel

| | |
|---|---|
| 40,00 | Sodium Laureth Sulfate |
| 5,00 | Decylglukosid |
| 5,00 | Cocamidopropylbetain |
| 5,00 | Polymer 1 (20 %ige wässrige Lösung) |
| 1,00 | Panthenol |
| q.s. | Parfum |
| q.s. | Konservierungsstoff |
| q.s. | Citronensäure |
| 2,00 | Natriumchlorid |
| ad 100 | dest. Wasser |

### Herstellung:

Komponenten einwiegen und lösen. pH-Wert auf 6 bis 7 einstellen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein klares Duschgel mit guten Eigenschaften erhalten.

### Beispiel 68: Shampoo

| | |
|---|---|
| 12,00 | Sodium Laureth Sulfate |
| 1,50 | Decylglukosid |
| 2,50 | Cocamidopropylbetain |
| 5,00 | Coco-Glucoside Glyceryl Oleate |
| 2,00 | Sodium Laureth Sulfate, Glycol Distearate, Cocamide MEA,Laureth-10 |
| 5,00 | Polymer 1 (20 %ige wässrige Lösung) |
| q.s. | Konservierungsstoff |
| q.s. | Sunset Yellow C. I. 15 985 |
| q.s. | Parfum |
| 1,00 | Natriumchlorid |
| ad 100 | dest. Wasser |

### Herstellung:

Komponenten einwiegen und lösen. pH-Wert auf 6 bis 7 einstellen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Shampoo mit guten Eigenschaften erhalten.

### Beispiel 69: Shampoo

| A | |
|---|---|
| 40,00 | Sodium Laureth Sulfate |
| 5,00 | Sodium C₁₂₋₁₅Pareth-15 Sulfonate |
| 5,00 | Decylglukosid |
| q.s. | Parfum |
| 0,10 | Phytantriol |
| | |

| B | |
|---|---|
| ad 100 | dest. Wasser |
| 5,00 | Polymer 1 (20 %ige wässrige Lösung) |
| 1,00 | Panthenol |
| q.s. | Konservierungsstoff |
| 1,00 | Laureth-3 |
| q.s. | Citronensäure |
| 2,00 | Natriumchlorid |

### Herstellung:

Komponenten der Phase A einwiegen und lösen. pH-Wert auf 6 bis 7 einstellen. Phase B zugeben und mischen.

Das Beispiel lässt sich mit den vernetzten Polyurethanen der Beispiele 2, 3, 4, 5, 6, 7, 8, 9, 10, 11, 12, 13 und 14 wiederholen. Es wird in jedem Fall ein Shampoo mit guten Eigenschaften erhalten.

## Patentansprüche

1. Vernetztes Polyurethan, das
A) mindestens ein Polytetrahydrofuran mit zwei endständigen Hydroxylgruppen pro Molekül und einem zahlenmittleren Molekulargewicht im Bereich von 650 bis 2000,
B) mindestens eine Verbindung, die mehr als zwei aktive Wasserstoffatome pro Molekül enthält,
C) mindestens eine Verbindung, die mindestens zwei aktive Wasserstoffatome und mindestens eine anionogene und/oder anionische Gruppe pro Molekül enthält,
D) ein Polyisocyanatgemisch, das Isophorondiisocyanat und Hexamethylendiisocyanat enthält, wobei das Gewichtsmengenverhältnis von Isophorondiisocyanat zu Hexamethylendiisocyanat in einem Bereich von 4:1 bis 15:1 liegt, und
E) mindestens eine von A) bis D) verschiedene Verbindung mit mindestens zwei aktiven Wasserstoffatomen und einem Molekulargewicht von 60 bis 5000,
eingebaut enthält und die Salze davon.

2. Polyurethan nach Anspruch 1, das zusätzlich als Komponente F) mindestens ein Polysiloxan mit wenigstens zwei aktiven Wasserstoffatomen pro Molekül eingebaut enthält.

3. Polyurethane nach einem der vorhergehenden Ansprüche, wobei die Komponente B) ausgewählt ist unter Triolen, Polyolen mit mehr als drei Hydroxylgruppen. Triaminen, Polyaminen mit mehr als drei primären und/oder sekundären Aminogruppen, Amin- und/oder Hydroxylgruppen-haltigen Polymeren und Mischungen davon.

4. Polyurethan nach einem der Ansprüche 1 bis 3, wobei die Komponente B) Trimethylolpropan umfasst

5. Polyurethan nach einem der Ansprüche 1 bis 3, wobei die Komponente B) ein Amingruppen- und/oder Hydroxylgruppen-haltiges Polymer umfasst, das durch radikalische Polymerisation wenigstens eines a,β-ethylenisch ungesättigten Monomers, das wenigstens eine funktionelle Gruppe aufweist, die ausgewählt ist unter Hydroxylgruppen, Amingruppen oder in Amingruppen überführbare Gruppen sowie gegebenenfalls weiterer Monomere, erhältlich ist.

6. Polyurethan nach Anspruch 5, wobei die Komponente B) ein Polymer umfasst, das ausgewählt ist unter Hydroxylgruppen-haltigen Polymeren, die eine OH-Zahl im Bereich von 0,3 bis 60 aufweisen, Amingruppen-haltigen Polymeren, die eine Aminzahl im Bereich von 0,3 bis 60 aufweisen und Hydroxyl- und Amingruppen-haltigen Polymeren, bei denen die Summe aus Hydroxyl- und Aminzahl in einem Bereich von 0,3 bis 60 liegt.

7. Polyurethan nach einem der Ansprüche 5 oder 6, wobei zur Polymerisation wenigstens ein Monomer a) eingesetzt wird, das ausgewählt ist unter Estern α,β-ethylenisch ungesättigter Mono- und Dicarbonsäuren mit Aminoalkoholen, die wenigstens eine primäre oder sekundäre Aminogruppe aufweisen. Amiden α,β-ethylenisch ungesättigter Mono- und Dicarbonsäuren mit Diaminen, die wenigstens eine primäre oder sekundäre Aminogruppe aufweisen, Estern α,β-ethylenisch ungesättigter Mono- und Dicarbonsäuren mit Diolen, Amiden α,β-ethylenisch ungesättigter Mono- und Dicarbonsäuren mit Aminoalkoholen, die eine primäre oder sekundäre Aminogruppe aufweisen, N,N-Diallylamin, und Mischungen davon,

8. Polyurethan nach Anspruch 7, wobei die zur Polymerisation eingesetzten Monomere a) N-tert.-Butylaminoethylmethacrylat umfassen oder N-tert.-Butylaminoethylmethacrylat als alleiniges Monomer a) eingesetzt wird.

9. Polyurethan nach einem der Ansprüche 5 bis 8, wobei die Komponente B) ein Amingruppen-haltiges Polymer umfasst, das
- N-tert.-Butylaminoethylmethacrylat
- Vinylimidazol
- Vinylpyrrolidon und
- Methacrylsäure
einpolymerisiert enthält.

10. Polyurethan nach einem der vorhergehenden Ansprüche, wobei die Komponente C) Dimethylolpropansäure umfasst

11. Polyurethan nach einem der vorhergehenden Ansprüche, wobei die Komponente D) aus einem Gemisch von Isophorondiisocyanat und Hexamethylendiisocyanat besteht

12. Polyurethan nach einem der vorhergehenden Ansprüche, wobei in Komponente D) das Gewichtsmengenverhältnis von Isophorondiisocyanat zu Hexamethylendiisocyanat in einem Bereich von 4:1 bis 10:1, liegt.

13. Polyurethan nach einem der Ansprüche 1 bis 12, aufgebaut aus
- 12 bis 35 Gew.%, bevorzugt 15 bis 30 Gew.-%, wenigstens einer Verbindung der Komponente A),
- 1 bis 20 Gew.-%. bevorzugt 3 bis 15 Gew.-%, wenigstens einer Verbindung der Komponente B), die ausgewählt Ist unter Amingruppen-haltigen Polymeren mit einer Aminzahl von mindestens 0,1 g KOH/g und einem zahlenmittleren Molekulargewicht von mindestens 1000,
- 5 bis 20 Gew.-%, bevorzugt 7 bis 18 Gew.-%, insbesondere 10 bis 15 Gew.-% wenigstens einer Verbindung der Komponente C),
- 23 bis 60 Gew.-%, bevorzugt 27 bis 55 Gel.-%, insbesondere 32 bis 50 Gew.-% wenigstens einer Verbindung der Komponente D),
- 2 bis 20 Gew.-%, bevorzugt 3 bis 15 Gew.-%, insbesondere 5 bis 12 Gew.-% wenigstens einer Verbindung der Komponente E),
- 0 bis 20 Ges'.-%, bevorzugt 0 bis 10 Gew.-% wenigstens einer Verbindung der Komponente F),
wobei sich die Komponenten zu 100 Gew.-% addieren.

14. Polyurethan nach einem der Ansprüche 1 bis 12, aufgebaut aus
- 15 bis 35 Gew.-%, bevorzugt 18 bis 30 Gew.-%, wenigstens einer Verbindung der Komponente A),
- 0,1 bis 5 Gew.-%, bevorzugt 0,5 bis 3 Gew.-%, insbesondere 0,7 bis 2,5 Gew.-%, wenigstens einer Verbindung der Komponente B), die ausgewählt ist unter Verbindungen, die mehr als zwei gegenüber NCO-Gruppen reaktive Gruppen aufweisen, mit einem Molekulargewicht im Bereich von etwa 80 bis weniger als 1000 g/mol, insbesondere Trimethylolpropan,
- 8 bis 20 Gew.-%, bevorzugt 10 bis 18 Gew.-%, insbesondere 12 bis 15 Gew.-% wenigstens einer Verbindung der Komponente C),
- 25 bis 60 Gew.-%, bevorzugt 30 bis 55 Gew.-%, insbesondere 35 bis 50 Gew.-% wenigstens einer Verbindung der Komponente D),
- 3 bis 20 Gew.-%, bevorzugt 5 bis 15 Gew.-%, insbesondere 7 bis 12 Gew.-% wenigstens einer Verbindung der Komponente E),
- 0 bis 20 Gew.-%, bevorzugt 0 bis 18 Gew.-% wenigstens einer Verbindung der Komponente F),
wobei sich die Komponenten zu 100 Gew.% addieren.

15. Kosmetisches oder pharmazeutisches Mittel, enthaltend
α) wenigstens ein vernetztes Polyurethan, wie in einem der Ansprüche 1 bis 14 definiert, wobei in Komponente D) das Gewichtsmengenverhältnis von Isophorondiisocyanat zu Hexamethylendiisocyanat in einem Bereich von 3:1 bis 15:1 liegt, und
β) wenigstens einen kosmetisch oder pharmazeutisch akzeptablen Träger.

16. Mittel nach Anspruch 15, wobei die Komponente β) ausgewählt ist unter
i) Wasser,
ii) wassermischbaren organischen Lösungsmitteln, vorzugsweise C₂-C₄-Alkanolen, insbesondere Ethanol,
iii) Ölen, Fetten. Wachsen,
iv) von iii) verschiedenen Estern von C₆-C₃₀-Monocarbonsäuren mit ein-, zwei-oder dreiwertigen Alkoholen,
v) gesättigten acyclischen und cyclischen Kohlenwasserstoffen,
vi) Fettsäuren,
vii) Fettalkoholen,
viii) Treibgasen,
und Mischungen davon.

17. Mittel nach einem der Ansprüche 15 oder 16, enthaltend wenigstens einen von den Komponenten α) und β) verschiedenen Zusatzstoff, der ausgewählt ist unter kosmetisch aktiven Wirkstoffen, Emulgatoren, Tensiden, Konservierungsmitteln, Parfümölen, Verdickern, Haarpolymeren, Haar und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebem, Feuchthaltemitteln, Rückfettern, Collagen, Eiweisshydrolysaten, Lipiden, Antioxidantlen, Entschäumern, Antistatika, Emollienzien und Weichmachern.

18. Mittel nach einem der Ansprüche 15 bis 17 in Form eines Gels, Schaums, Sprays, einer Mousse, Salbe, Creme, Emulsion, Suspension, Lotion, Milch oder Paste.

19. Verwendung eines Polyurethans, wie in einem der Ansprüche 1 bis 14 definiert, in Hautreinigungsmitteln. Mitteln zur Pflege und zum Schutz der Haut, Nagelpflegemitteln, Zubereitungen für die dekorative Kosmetik und Haarbehandlungsmitteln,

20. Verwendung nach Anspruch 19 in Haarbehandlungsmitteln als Festiger und/oder als Conditioner.

21. Verwendung nach Anspruch 20, wobei das Mittel in Form eines Haargels, Shampoos, Schaumfestigers, Haarwassers, Haarsprays oder Haarschaums vorliegt.

22. Verwendung eines Polyurethans, wie in einem der Ansprüche 1 bis 14 definiert, als Hilfsmittel in der Pharmazie, bevorzugt als oder in Beschichtungsmittel(n) für feste Arzneiformen, zur Modifizierung rheologischer Eigenschaften, als oberflächenaktive Verbindung, als oder in Klebemittel(n) sowie als oder In Beschichtungsmittel(n) für die Textil-, Papier-, Druck- und Lederindustrie.

## Claims

1. A crosslinked polyurethane which comprises, in incorporated form,
A) at least one polytetrahydrofuran with two terminal hydroxyl groups per molecule and a number-average molecular weight in the range from 650 to 2000,
B) at least one compound which comprises more than two active hydrogen atoms per molecule,
C) at least one compound which comprises at least two active hydrogen atoms and at least one anionogenic and/or anionic group per molecule,
D) a polyisocyanate mixture which comprises isophorone diisocyanate and hexamethylene diisocyanate, where the quantitative weight ratio of isophorone diisocyanate to hexamethylene diisocyanate is in a range from 4:1 to 15:1, and
E) at least one compound different from A) to D) with at least two active hydrogen atoms and a molecular weight of from 60 to 5000,
and the salts thereof.

2. The polyurethane according to claim 1, which additionally comprises, in incorporated form, as component F) at least one polysiloxane with at least two active hydrogen atoms per molecule.

3. The polyurethane according to one of the preceding claims, where component B) is chosen from triols, polyols with more than three hydroxyl groups, triamines, polyamines with more than three primary and/or secondary amino groups, amine- and/or hydroxyl-group-containing polymers and mixtures thereof.

4. The polyurethane according to one of claims 1 to 3, where component B) comprises trimethylolpropane.

5. The polyurethane according to one of claims 1 to 3, where component B) comprises an amine-group- and/or hydroxyl-group-containing polymer which is obtainable by free-radical polymerization of at least one α,β-ethylenically unsaturated monomer which has at least one functional group which is chosen from hydroxyl groups, amine groups or groups which can be converted to amine groups, and if appropriate further monomers.

6. The polyurethane according to claim 5, where component B) comprises a polymer which is chosen from hydroxyl-group-containing polymers which have an OH number in the range from 0.3 to 60, amine-group-containing polymers which have an amine number in the range from 0.3 to 60 and hydroxyl- and amine-group-containing polymers in which the sum of hydroxyl and amine number is in the range from 0.3 to 60.

7. The polyurethane as claimed in either claim 5 or 6, where, for the polymerization, at least one monomer a) is used which is chosen from esters of α,β-ethylenically unsaturated mono- and dicarboxylic acids with amino alcohols which have at least one primary or secondary amino group, amides of α,β-ethylenically unsaturated mono- and dicarboxylic acids with diamines which have at least one primary or secondary amino group, esters of α,β-ethylenically unsaturated mono- and dicarboxylic acids with diols, amides of α,β-ethylenically unsaturated mono- and dicarboxylic acids with amino alcohols which have a primary or secondary amino group, N,N-diallylamine, and mixtures thereof.

8. The polyurethane according to claim 7, where the monomers a) used for the polymerization comprise N-tert-butylaminoethyl methacrylate or N-tert-butylaminoethyl methacrylate is used as the sole monomer a).

9. The polyurethane according to one of claims 5 to 8, where component B) comprises an amine-group-containing polymer which comprises
- N-tert-butylaminoethyl methacrylate
- vinylimidazole
- vinylpyrrolidone and
- methacrylic acid
in copolymerized form.

10. The polyurethane as claimed in one of the preceding claims, where component C) comprises dimethylolpropanoic acid.

11. The polyurethane according to one of the preceding claims, where component D) consists of a mixture of isophorone diisocyanate and hexamethylene diisocyanate.

12. The polyurethane according to one of the preceding claims, where, in component D), the quantitative weight ratio of isophorone diisocyanate to hexamethylene diisocyanate is in a range from 4:1 to 10:1.

13. The polyurethane according to one of claims 1 to 12, constructed from
- 12 to 35% by weight, preferably 15 to 30% by weight, of at least one compound of component A).
- 1 to 20% by weight, preferably 3 to 15% by weight, of at least one compound of component B) which is chosen from amine-group-containing polymers with an amine number of at least 0.1 g of KOH/g and a number-average molecular weight of at least 1000,
- 5 to 20% by weight, preferably 7 to 18% by weight, in particular 10 to 15% by weight, of at least one compound of component C),
- 23 to 60% by weight, preferably 27 to 55% by weight, in particular 32 to 50% by weight, of at least one compound of component D),
- 2 to 20% by weight, preferably 3 to 15% by weight, in particular 5 to 12% by weight, of at least one compound of component E),
- 0 to 20% by weight, preferably 0 to 10% by weight, of at least one compound of component F),
where the components add up to 100% by weight.

14. The polyurethane according to one of claims 1 to 12, constructed from
- 15 to 35% by weight, preferably 18 to 30% by weight, of at least one compound of component A),
- 0.1 to 5% by weight, preferably 0.5 to 3% by weight, in particular 0.7 to 2.5% by weight, of at least one compound of component B), which is chosen from compounds which have more than two groups reactive toward NCO groups, with a molecular weight in the range from about 80 to less than 1000 g/mol, in particular trimethylolpropane,
- 8 to 20% by weight, preferably 10 to 18% by weight, in particular 12 to 15% by weight, of at least one compound of component C),
- 25 to 60% by weight, preferably 30 to 55% by weight, in particular 35 to 50% by weight, of at least one compound of component D),
- 3 to 20% by weight, preferably 5 to 15% by weight, in particular 7 to 12% by weight, of at least one compound of component E),
- 0 to 20% by weight, preferably 0 to 18% by weight, of at least one compound of component F),
where the components add up to 100% by weight.

15. A cosmetic or pharmaceutical composition comprising
α) at least one crosslinked polyurethane as defined in one of claims 1 to 14, where, in component D), the quantitative weight ratio of isophorone diisocyanate to hexamethylene diisocyanate is in a range from 3:1 to 15.1, and
β) at least one cosmetically or pharmaceutically acceptable carrier.

16. The composition according to claim 15, where the component β) is chosen from
i) water,
ii) water-miscible organic solvents, preferably C₂-C₄-alkanols, in particular ethanol,
iii) oils, fats, waxes,
iv) esters of C₆-C₃₀-monocarboxylic acids with mono-, di- or trihydric alcohols different from iii),
v) saturated acyclic and cyclic hydrocarbons,
vi) fatty acids,
vii) fatty alcohols,
viii) propellant gases,
and mixtures thereof.

17. The composition according to either claim 15 or 16, comprising at least one additive different from components α) and β) which is chosen from cosmetically active ingredients, emulsifiers, surfactants, preservatives, perfume oils, thickeners, hair polymers, hair and skin conditioners, graft polymers, water-soluble or dispersible silicone-containing polymers, light protection agents, bleaches, gel formers, care agents, colorants, tinting agents, tanning agents, dyes, pigments, bodying agents, humectants, refatting agents, collagen, protein hydrolyzates, lipids, antioxidants, antifoams, antistats, emollients and softeners.

18. The composition according to one of claims 15 to 17 in the form of a gel, foam, spray, mousse, ointment, cream, emulsion, suspension, lotion, milk or paste.

19. The use of a polyurethane as defined in one of claims 1 to 14, in skin-cleansing compositions, compositions for the care and protection of the skin, nail-care compositions, preparations for decorative cosmetics and hair-treatment compositions.

20. The use according to claim 19 in hair-treatment compositions as setting agents and/or as conditioners.

21. The use according to claim 20, where the composition is in the form of a hair gel, shampoo, setting foam, hair tonic, hairspray or hair foam.

22. The use of a polyurethane as defined in one of claims 1 to 14, as auxiliary in pharmacy, preferably as or in (a) coating(s) for solid drug forms, for modifying rheological properties, as surface-active compound, as or in (an) adhesive(s), and as or in (a) coating(s) for the textile, paper, printing and leather industry.

## Revendications

1. Polyuréthanne réticulé, qui contient incorporés
A) au moins un polytétrahydrofuranne comportant deux groupes hydroxy en bout de chaîne par molécule et ayant une masse moléculaire moyenne en nombre dans la plage de 650 à 2 000.
B) au moins un composé qui contient plus de deux atomes d'hydrogène actifs par molécule,
C) au moins un composé qui contient au moins deux atomes d'hydrogène actifs et au moins un groupe anionique et/ou anionogène par molécule,
D) un mélange de polyisocyanates qui contient de l'isophorone-diisocyanate et de l'hexaméthylène-diisocyanate, le rapport des quantités pondérales de l'isophorone-diisocyanate à l'hexaméthylène-diisocyanate se situant dans une plage allant de 4:1 à 15:1, et
E) au moins un composé différent de A) à D), comportant au moins deux atomes d'hydrogène actifs et ayant une masse moléculaire de 60 à 5 000,
et les sels d'un tel composé.

2. Polyuréthanne selon la revendication 1, qui contient en outre en tant que composant F) au moins un polysiloxane comportant au moins deux atomes d'hydrogène actifs incorporés par molécule.

3. Polyuréthanne selon l'une quelconque des revendications précédentes, dans lequel le composant B) est choisi parmi des triols, des polyols comportant plus de trois groupes hydroxy, des triamines, des polyamines comportant plus de trois groupes amino primaires et/ou secondaires, des polymères contenant des groupes amino et/ou hydroxy et des mélanges de ceux-ci.

4. Polyuréthanne selon l'une quelconque des revendications 1 à 3, dans lequel le composant B) comprend le triméthylolpropane.

5. Polyuréthanne selon l'une quelconque des revendications 1 à 3, dans lequel le composant B) comprend un polymère comportant des groupes amino et/ou hydroxy, qui peut être obtenu par polymérisation radicalaire d'au moins un monomère à insaturation α,β-éthylénique qui comporte au moins un groupe fonctionnel qui est choisi parmi les groupes hydroxy, les groupes amino ou les groupes pouvant être convertis en groupes amino, ainsi qu'éventuellement d'autre monomères.

6. Polyuréthanne selon la revendication 5, dans lequel le composant B) comprend un polymère qui est choisi parmi des polymères contenant des groupes hydroxy, qui présentent un indice de groupes OH dans la plage de 0,3 à 60, des polymères contenant des groupes amino, qui présentent un indice d'amine dans la plage de 0,3 à 60, et des polymères contenant des groupes hydroxy et amino, dans lesquels la somme de l'indice de groupes hydroxy et de l'indice d'amine se situe dans une plage allant de 0,3 à 60.

7. Polyuréthanne selon la revendication 5 ou 6, dans lequel on utilise pour la polymérisation au moins un monomère a) qui est choisi parmi des esters d'acides mono- et dicarboxyliques à insaturation α,β-éthylénique avec des aminoalcools qui comportent au moins un groupe amino primaire ou secondaire, des amides d'acides mono- et dicarboxyliques à insaturation α,β-éthylénique avec des diamines qui comportent au moins un groupe amino primaire ou secondaire, des esters d'acides mono- et dicarboxyliques à insaturation α,β-éthylénique avec des diols, des amides d'acides mono- et dicarboxyliques à insaturation α,β-éthylénique avec des aminoalcools qui comportent un groupe amino primaire ou secondaire, une N,N-dialkylamine, et des mélanges de ceux-ci.

8. polyuréthanne selon la revendication 7, dans lequel les monomères utilisés pour la polymérisation comprennent a) du méthacrylate de N-tert-butylaminoéthyle ou le méthacrylate de N-tert-butylaminoéthyle est utilisé comme seul monomère a).

9. Polyuréthanne selon l'une quelconque des revendications 5 à 8, dans lequel le composant B) comprend un polymère contenant des groupes amino, qui contient incorporés par polymérisation
- du méthacrylate de N-tert-butylaminoéthyle
- du vinylimidazole
- de la vinylpyrrolidone et
- de l'acide méthacrylique.

10. Polyuréthanne selon l'une quelconque des revendications précédentes, dans lequel le composant C) comprend de l'acide diméthylol-propionique.

11. Polyuréthanne selon l'une quelconque des revendications précédentes, dans lequel le composant D) consiste en un mélange d'isophorone-diisocyanate et d'hexaméthylène-diisocyanate.

12. Polyuréthanne selon l'une quelconque des revendications précédentes, dans lequel le rapport des quantités pondérales de l'isophorone-diisocyanate à l'hexaméthylène-diisocyanate dans le composant D) se situe dans une plage allant de 4:1 à 10:1.

13. Polyuréthanne selon l'une quelconque des revendications 1 à 12, constitué de
- 12 à 35 % en poids, de préférence 15 à 30 % en poids, d'au moins un composé du composant A),
- 1 à 20 % en poids, de préférence 3 à 15 % en poids, d'au moins un composé du composant B), qui est choisi parmi des polymères contenant des groupes amino, ayant un indice d'amine d'au moins 0,1 g de KOH/g et ayant une masse moléculaire moyenne en nombre d'au moins 1 000,
- 5 à 20 % en poids, de préférence 7 à 18 % en poids, en particulier 10 à 15 % en poids d'au moins un composé du composant C),
- 23 à 60 % en poids, de préférence 27 à 55 % en poids, en particulier 32 à 50 % en poids d'au moins un composé du composant D),
- 2 à 20 % en poids, de préférence 3 à 15 % en poids, en particulier 5 à 12 % en poids d'au moins un composé du composant E),
- 0 à 20 % en poids, de préférence 0 à 10 % en poids d'au moins un composé du composant F),
la somme des composants étant égale à 100 % en poids.

14. Polyuréthanne selon l'une quelconque des revendications 1 à 12, constitué de
- 15 à 35 % en poids, de préférence 18 à 30 % en poids, d'au moins un composé du composant A),
- 0,1 à 5 % en poids, de préférence 0,5 à 3% en poids, en particulier 0,7 à 2,5 % en poids, d'au moins un composé du composant B), qui est choisi parmi des composés qui comportent plus de deux groupes réactifs vis-à-vis de groupes NCO, ayant une masse moléculaire dans la plage d'environ 80 à moins de 1 000 g/mole, en particulier de triméthylolpropane,
- 8 à 20 % en poids, de préférence 10 à 18 % en poids, en particulier 12 à 15 % en poids d'au moins un composé du composant C),
- 25 à 60 % en poids, de préférence 30 à 55 % en poids, en particulier 35 à 50 % en poids d'au moins un composé du composant D),
- 3 à 20 % en poids, de préférence 5 à 15 % en poids, en particulier 7 à 12 % en poids d'au moins un composé du composant E),
- 0 à 20 % en poids, de préférence 0 à 18 % en poids d'au moins un composé du composant F),
la somme des composants étant égale à 100 % en poids.

15. Composition cosmétique ou pharmaceutique, contenant
α) au moins un polyuréthanne réticulé, tel que défini dans l'une quelconque des revendications 1 à 14, dans lequel le rapport des quantités pondérales de l'isophorone-diisocyanate à l'hexaméthylène-diisocyanate dans le composant D) se situe dans une plage allant de 3:1 à 15:1.
β) au moins un véhicule cosmétiquement ou pharmaceutiquement acceptable.

16. Composition selon la revendication 15, dans laquelle le composant β) est choisi parmi
i) l'eau,
ii) des solvants organiques miscibles à l'eau, de préférence des alcanols en C₂-C₄, en particulier l'éthanol,
iii) des huiles, des graisses, des cires,
iv) des esters, différents de iii), d'acides monocarboxyliques en C₆-C₃₀ avec des alcools mono-, di- ou trihydriques,
v) des hydrocarbures saturés acycliques ou cycliques,
vi) des acides gras,
vii) des alcools gras,
viii) des gaz propulseurs,
et des mélanges de ceux-ci.

17. Composition selon la revendication 15 ou 16, contenant au moins un additif différent des composants α) et β), qui est choisi parmi des substances actives à activité cosmétique, des émulsifiants, des tensioactifs, des conservateurs, des huiles essentielles, des épaississants, des polymères pour cheveux, des conditionneurs pour la peau et les cheveux, des polymères greffés, des polymères contenant des silicones, dispersables ou solubles dans l'eau, des photoprotecteurs, des décolorants, des agents gélifiants, des agents de soin, des agents colorants, des agents de nuançage, des agents bronzants, des colorants, des pigments, des agents de consistance, des agents hydratants, des agents de regraissage, le collagène, des hydrolysats de protéines, des lipides, des antioxydants, des antimousses, des agents antistatiques, des émollients et des assouplissants.

18. Composition selon l'une quelconque des revendications 15 à 17, sous forme d'un gel, d'un produit moussant, d'un produit à pulvériser en aérosol, d'une mousse, d'une pommade, d'une crème, d'une émulsion, d'une suspension, d'une lotion, d'un lait ou d'une pâte.

19. Utilisation d'un polyuréthanne, tel que défini dans l'une quelconque des revendications 1 à 14, dans des produits pour le nettoyage de la peau, des produits pour le soin et pour la protection de la peau, des produits pour le soin des ongles, des préparations pour la cosmétique de maquillage et des produits de traitement capillaire.

20. Utilisation selon la revendication 19, dans des produits de traitement capillaire, en tant que fixateurs et/ou en tant que conditionneurs.

21. Utilisation selon la revendication 20, dans laquelle le produit se trouve sous forme d'un gel capillaire, d'un shampooing, d'un fixateur en mousse, d'une lotion capillaire, d'une laque pour cheveux ou d'une mousse capillaire.

22. Utilisation d'un polyuréthanne, tel que défini dans l'une quelconque des revendications 1 à 14, en tant qu'adjuvant en pharmacie, de préférence en tant que ou dans des composition(s) d'enrobage pour des formes pharmaceutiques solides, pour la modification de propriétés rhéologiques, en tant que composé tensioactif, en tant qu'adhésif ou dans des adhésifs, ainsi qu'en tant que ou dans des composition(s) de revêtement pour l'industrie des textiles, du papier, de l'imprimerie et du cuir.
